# EUROPEAN PATENT SPECIFICATION

(11) **EP 0 979 101 B1**
(45) Date of publication and mention of the grant of the patent: **27.10.2010**
(21) Application number: 97932430.8
(22) Date of filing: 30.06.1997
(51) Int. Cl.: A61K 39/235, A61K 39/42, C07H 21/04, C07K 1/00, C07K 14/005, C12P 19/34, C12N 15/00, C12N 15/09, C12N 15/34, C12N 15/63, C12N 15/70

(54) **RECOMBINANT CANINE ADENOVIRUS 2 (CAV2) CONTAINING EXOGENOUS DNA**
REKOMBINANTER HUNDE-ADENOVIRUS 2 (CAV2), WELCHER EXOGENE DNA ENTHÄLT
ADENOVIRUS CANIN 2 RECOMBINANT (ACR2) CONTENANT DE l'ADN EXOGENE

(30) Priority: 03.07.1996 US 675556; 03.07.1996 US 675566
(43) Date of publication of application: 16.02.2000
(73) Proprietor: Merial, Inc., Lyon 07 (FR)
(72) Inventor: FISCHER, Laurent, Albany, NY 12208 (US)
(74) Representative: Harding, Charles Thomas
(86) International application number: PCT/US1997/011486
(87) International publication number: WO 1998/000166

(56) References cited:
- WO-A-91/11525
- WO-A-91/11525
- WO-A-94/26914
- WO-A-95/16048
- WO-A-96/12030
- US-A- 4 963 481
- US-A- 5 466 585
- US-A- 5 514 582
- DRAGULEV B P ET AL: "SEQUENCE ANALYSIS OF PUTATIVE E3 AND FIBER GENOMIC REGIONS OF TWO STRAINS OF CANINE ADENOVIRUS TYPE 1" VIROLOGY, RAVEN PRESS, NEW YORK, NY, US, vol. 183, no. 1, 1 July 1991 (1991-07-01), pages 298-305, XP000607410 ISSN: 0042-6822
- BEARD CLAYTON W ET AL: "Analysis of early region 3 mutants of mouse adenovirus type 1." JOURNAL OF VIROLOGY, vol. 70, no. 9, 1996, pages 5867-5874, XP002206290 ISSN: 0022-538X
- MORRISON MARK D ET AL: "Generation of E3-deleted canine adenoviruses expressing canine parvovirus capsid by homologous recombination in bacteria." VIROLOGY, vol. 293, no. 1, 1 February 2002 (2002-02-01), pages 26-30, XP002206291 February 1, 2002 ISSN: 0042-6822
- PROC. NATL. ACAD. SCI. U.S.A., June 1994, Vol. 91, No. 13, ENGELHARDT et al., "Ablation of E2A in Recombinant Adenoviruses Improves Transgene Persistence and Decreases Inflammatory Response in Mouse Liver", pages 6196-6200.
- BIO/TECHNOLOGY, September 1994, Vol. 12, No. 9, DORAI et al., "Mammalian Cell Expression of Single-Chain Fv (sFv) Antibody Proteins and Their C-Terminal Fusions with Interleukin-2 and Other Effector Domains", pages 890-897.
- D.C. BLOOD & VIRGINIA P. STUDDERT: "Baillière's Comprehensive Veterinary Dictionary" 1988, BAILLIÈRE TINDALL , LONDON * page 258 *

## Description

### FIELD

This description relates to recombinant adenoviruses, methods of making them, uses for them (including as a vector for replicating DNA), expression products from them, and uses for the expression products. This description also relates to promoters and expression cassettes, especially truncated promoters and expression cassettes containing the promoters.

More particularly, this description relates to recombinant canine adenoviruses (CAV) and methods of making them, uses for them (including as a vector for replicating DNA), expression products from them, and uses for the expression products. Recombinant CAV2 viruses, especially those wherein the exogenous DMA has been inserted into the CAV2 E3 and/or into the right end of the genome between the right ITR and the E4 transcription unit, and methods of making them, uses for them (including in immunological, immunogenic, vaccine or therapeutic compositions, or as a vector for cloning, replicating or expressing DNA and methods of using the compositions or vector), expression products from them, and uses for the expression products are preferred.

However, the description broadly relates to a CAV synthetically modified to contain therein exogenous DNA, wherein a non-essential region of the CAV genome or a portion thereof has been deleted from the CAV. The CAV is preferably packaged as an infectious CAV with respect to cells in which CAV naturally replicates. The non-essential region of the CAV genome or portion thereof deleted from the CAV is preferably the E3 region or a portion thereof. The exogenous DNA is preferably present in the E3 region, the E1 region, the E4 region, or the region located between the right ITR and the E4 region. And, the CAV can be a CAV2.

The recombinant CAV can be a vector for expression or cloning of heterologous DNA. The heterologous DNA can encode any desired expression product. Preferred expression products include: an epitope of interest, a biological response modulator, a growth factor, a recognition sequence, a therapeutic gene, or a fusion protein. Thus, the heterologous DNA can encode any or all of these products. Accordingly, the heterologous DNA can bye a transgene.

The epitope of interest can be antigen or immunogen or epitope thereof of a human or veterinary pathogen or toxin. Therefore, the description further relates to immunological, antigenic or vaccine compositions, containing the expression products. Further, since the CAV vector, in certain instances, can be administered directly to a suitable host, the description relates to compositions containing the CAV vector. The compositions can be immunological, antigenic, vaccine, or therapeutic (e.g., Compositions for stimulating an immunological response - local or systemic - including, but not limited to a protective response, or for gene therapy). The description therefore futher relates to methods of inducing an immunological response, or of transferring genetic information (e.g., gene therapy) comprising administering the composition to a suitable vertebrate host (animal or human).

Additionally, since the expression product can be isolated from the CAV vector *in vitro* or from cells infected or transfected by the CAV vector *in vitro*, the description relates to methods for expressing a product, e.g., comprising inserting the exogenous DNA into a CAV as a vector to obtain a recombinant CAV, e.g., by recombination or by cleaving and ligating and obtaining recombinant CAV therefrom, followed by infection or transfection of suitable cells *in vitro* with the recombinant CAV, and optionally extracting, purifying or isolating the expression product from the cells.

As the expression products can provider an antigenic, immunological or protective (vaccine) response, the description further relates to products therefrom; namely, antibodies and uses thereof. More in particular, the expression products can elicit antibodies. The antibodies can be formed into monoclonal antibodies; and, the antibodies or expression products can be used in kits, assays, tests, and the like involving binding, so that the description relates to these uses too.

Additionally, since the recombinants of the description can be used to replicate DNA, the description relates to recombinant CAV as a vector and methods for replicating DNA by infecting cells with the recombinant and harvesting DNA therefrom. The resultant DNA can be used as probes or primers or for amplification.

The description still further relates to promoters and expression cassettes containing the promoters, for use in recombinant viruses or plasmids.

In this aspect, the description specifically relates to a truncated transcriptionally active promoter for a recombinant virus or plasmid which comprises a region transactivated with a transactivating protein provided by the virus or a system into which the plasmid is inserted and the minimal promoter region of the promoter. The description also relates to an expression cassette comprising the promoter, and to viruses or plasmids containing the promoter or expression cassette. The expression cassette can include a functional truncated polyadenylation signal.

Several publications are cited in the following text, with full citation of each set forth in the section headed References or with full citation occurring where cited.

### BACKGROUNED OF THE INVENTION

The patent and scientific literature includes various viral vector systems, uses therefor, and exogehous DNA for expression of protein by such systems, as well as uses for such proteins and uses for products from such proteins.

For instance, recombinant poxvirus (e.g., vaccinia, avipox virus) and exogenous DNA for expression in viral vector systems can be found in U.S. Patent Nos. 5,174,993 and 5,505,941 (e.g., recombinant avipox virus, vaccinia virus; rabies glycoprotein (G), gene, turkey influenza hemagglutinin gene, gp51,30 envelope gene of bovine leukemia virus, Newcastle Disease Virus (NDV) antigen, FelV envelope gene, RAV-1 env gene, NP (nudeoprotein gene of Chicken/Pennsylvania/1/83 influenza virus), matrix and preplomer gene of infectious bronchitis virus; HSV gD; entomopox promoter; *inter alia*), U.S. Patent No. 5,338,683, e.g., recombinant vaccinia virus, avipox virus; DNA encoding Herpesvirus glycoproteins, *inter alia*; U.S. Patent No. 5,494,807 (e.g., recombinant vaccinia, avipox; exogenous DNA encoding antigens from rabies, Hepatitis B, JEV, YF, Dengue, measles, pseudorabies, Epstein-Barr, HSV, HIV, SIV, EHV, BHV, HCMV, canine parvovirus, equine influenza, FeLV, FHV, Hantaan, C. tetani, avian influenza, mumps, NDV, *inter alia*); U.S. Patent No. 5,503,834 (e.g., recombinant vaccinia, avipox, Morbillivirus [e.g., measles F, hemagglutinin, *inter alia*]); U.S. Patent No. 4,722,848 (e.g., recombinant vaccinia virus; HSV tk, glycoproteins [e.g., gB, gD], influenza HA; Hepatitis B [e.g., HBsAg], *inter alia*); U.K. Patent GB 2 269 820 B and U.S. Patent No. 5,514,375 (recombinant poxvirus; flavivirus structural proteins); WO 92/22641 (e.g., recombinant poxvirus; immunodeficiency virus, *inter alia*); WO 93/03145 (e.g., recombinant poxvirus; IBDV, *inter alia*); WO 94/16716 and U.S. application Serial No. 08/184,009, filed January 19, 1994 (e.g., recombinant poxvirus; cytokine and/or tumor associated antigens, *inter alia*); and PCT/US94/06652 (*Plasmodium* antigens such as from each stage of the *Plasmodium* life cycle).

Baculovirus expression systems, exogenous DNA for expression therein, and purification of recombinant proteins therefrom can be found in Richardson, C.D. (Editor), Methods in Molecular Biology 39, "Baculovirus Expression Protocols" (1995 Humana Press Inc.) (see, e.g., Ch.18 for influenza HA expression, Ch.19 for recombinant protein purification techniques), Smith et al., "Production of Huma Beta Interferon in Insect Cells Infected with a Baculovirus Expression Vector," Molecular and Cellular Biology, Dec., 1983, Vol. 3, No. 12, p. 2156-2165; Pennock et al., "Strong and Regulated Expression of Escherichia coli B-Galactosidase in Infect Cells with a Baculovirus vector," Molecular and Cellular Biology Mar. 1984, Vol. 4, No. 3, p. 399-406; EPA 0 370 573 (Skin test and test kit for AIDS, discussing baculovirus expression systems containing portion of HIV-1 env gene, and citing U.S. application Serial No. 920,197, filed October 16, 1986 and EP Patent publication No. 265785).

U.S. Patent No. 4,769,331 relates to herpesvirus as a vector.

There are also poliovirus and adenovirus vector systems (see, e.g., Kitson et al., J. Virol. 65, 3068-3075, 1991; Grunhaus et al., 1992, "Adenovirus as cloning vectors," Seminars in Virology (Vol. 3) p. 237-52, 1993; Ballay et al. EMBO Journal, vol. 4, p. 3861-65; Graham, Tibtech 8, 85-87, April, 1990; Prevec et al., J. Gen Virol. 70, 429-434).

PCT WO91/11525 relates to CAV2 modified to contain a promoter-gene sequence within the region from the SmaI site close to the end of the inverted terminal repeat region up to the promoter for the early region 4 (E4).

CAV, and particularly CAV2, has numerous problems. Several of these problems are discussed below. A significant problem is that the CAV genome can only accept a limited amount of exogenous DNA. That is, only a limited amount of exogenous DNA can be inserted into the CAV genome. Thus, CAV is "insert size limited" and therefore presents a significant problem which must be addressed if CAV is to be a useful vector for a constellation of cloning and expression applications.

The efficient transmission of many viral infections via the oronasal route has provided the impetus for assessing the efficacy of viral vector-based vaccine candidates via the same route. However, since the spread of most live replicating vaccines within the vaccinee and their spread to or contacts with the general environment are well documented (for examples see Schwartz et al., 1974, Mueller et al., 1989, Oualikene et al., 1994), the choice of an adequate viral vector is not obvious.

To address legitimate safety concerns, vector selection preferably involves consideration of characterized live attenuated vaccines as the apparent safety thereof is established. For vaccination of humans, various vectors based on replicating live attenuated viruses are under consideration. To date, there are documented approaches based on human adenoviruses (HAVs) serotype 4 and 7 (Lubeck et al., 1989, Chanda et al., 1990, Chengalvala et al., 1991, 1994, Hsu et al., 1994 ), influenza viruses (for a review Garcia-Sastre and Palese, 1995) and poliovirus and related viruses (for a review Girard et al., 1995).

In the field of veterinary medicine, several vectors based on replicating live attenuated viruses are currently being analyzed with the objective to apply those recombinant vectors as vaccines either parenterally or via the natural route of infection, thereby stimulating local protection. Among the best characterized at this point are members of the poxviridae family [e.g., fowlpox-based vectors (Edbauer et al. 1990, Taylor et al., 1995 and ref. therein)], herpesviridae family [e.g., pseudorabies virus-based vectors (Sedegah et al. 1992, Mettenleiter et al. 1994, Hooft van Iddekinge et al., 1996 and ref. therein), turkey herpes virus-based vectors (Ross et al. 1993, Darteil et al. 1995 and ref. therein), feline herpes virus-based vectors (Cole et al., 1990, Wardley et al., 1992, Willense et al., 1996), infectious laryngotracheitis virus-based vectors (Guo et al., 1994), bovine herpes virus-based vectors (Kit et al. 1991)] and to a lesser extent members of the Adenoviridae family [bovine adenovirus 3-based vectors (Mittal et al., 1995)].

The canine species provides an appropriate model for oronasal immunizations. As such, the canine adenovirus serotype 2 (CAV2) for which attenuated vaccinal strains exist that can be safely administrated either parenterally or via oronasal route, provides a viable immunization vehicle for canine vaccination. Canine distemper virus (CDV) infection of dogs provides a good example of a respiratory infection in this target species. Further, a relatively direct experimental CDV challenge system is accessible and allows a direct comparison between CAV2 based-vaccine candidates and previously developed classical CDV vaccines.

CAV2 was first isolated from an outbreak of upper respiratory tract infection in dogs by Ditchfield et al. (1962). Since then, the virus has been isolated from the respiratory tract of dogs with respiratory diseases both in the US and in Europe (Binn et al. 1967, Appel and Percy, 1970, Assaf et al. 1978, Danskin 1973). Experimental studies have resulted in mild respiratory disease following aerosol inoculation of CAV2 (Swango et al. 1970, Appel , 1970). Several CAV2-based vaccines have been developed and extensively used worldwide for the vaccination of puppies and adult dogs. Immunization with CAV2 has even been shown to protect against an experimental challenge exposure with a serologically related strain of CAV1, which is fatal to non-vaccinated dogs (Fairchild and Cohen, 1969, Appel et al. 1973, Bass et al. 1980). The apparent safety of CAV2 as a vaccine has been well evidenced by the lack of vaccine-induced and vaccine-associated complications in dogs and other animal species including man during its 30 years of utility. Further, results from field serological surveys indicate that many wild animals (foxes, raccoons, skunks and mongooses) are asymptomatically exposed to CAV2 or to an antigenically related virus infection (Summer et al., 1988). A vaccinal strain of canine adenovirus serotype 2 (CAV2), therefore, provides a unique example of a safe replication-competent, host-restricted virus which can be considered for the derivation of effective vector-based vaccine candidate for vaccination, especially of dogs.

HAVS have been shown to be valuable mammalian cell expression vectors (for a review see Graham et al. 1988) and are currently being evaluated both as recombinant viral vaccine candidates (for reviews see Randrianarison-Jewtoukoff and Perricaudet 1995, Imler 1995) and as vectors for gene therapy (for reviews see Perricaudet and Perricaudet 1995). There are two major groups of HAVs, and a third, less explored, group of recombinant HAVs.

The first group of these adenovirus vectors corresponds to replication-incompetent recombinant adenoviruses which are based on viruses deleted of their E1 region. The E1 region encodes proteins which are essential for virus replication in tissue culture. It has, however, been demonstrated that replication-incompetent recombinant adenoviruses deleted of their E1 region can be propagated in the 293 cell line (Graham et al., 1977) which constitutively expresses the E1 region (Haj-Ahmad et al., 1986).

Deletion of the E1 region not only increases the amount of foreign DNA which can be inserted into HAVs, but also limits their replication in human cells and thus considerably improves the safety characteristics of the corresponding recombinant HAVs in humans. Most of the HAV-based vaccine candidates against veterinary and humans pathogens are currently based on E1-deleted vectors. Despite their limited replicative capacity, protection data in challenge experiments have been described (Prevec et al., 1989, McDermott et al., 1989, Lubeck et al., 1989, Eloit et al., 1990, Ragot et al., 1993, Wesseling et al., 1993, Both et al., 1993, Gallichan et al., 1993, Hsu et al., 1994, Breker-Klasser et al., 1995). The property of inducing a protective immune response even in the absence of vector replication is shared by other host restricted viral vectors, the most promising of which being the canarypox virus-based vector ALVAC (Taylor et al., 1991, see Perkus et al., 1995 for a review).

When the goal is a replication competent adenovirus vector, the use of the E1 region as an insertion site is thus not desirable; and, the E1 region therefore has heretofore had deficiencies and presented problems. These deficiencies and problems are compounded when a replication competent adenovirus displaying safety characteristics with respect to humans is desired. In particular, while the E1 region deletion in HAVs may limit replication in human cells and improve safety characteristics with respect to humans, as discussed below, the possibility of recombination between E1 transformed cell lines and E1 deleted recombinant adenoviruses has been documented and thus the safety profile of E1 transformed cell lines appears questionable, thereby rendering any benefit from using E1 region deleted adenoviruses potentially illusory and exascerbating deficiencies and problems heretofore in the use of E1 region deleted adenoviruses (since propagation of E1 region deleted adenoviruses is in cells which constitutively express the E1 region).

The second group of adenovirus vectors corresponds to recombinant adenoviruses which are replication-competent in human cells but replication-incompetent in most non-human animal cells. Those viruses are characterized by a substitution of part of the E3 region with foreign gene expression cassettes. The E3 region has been shown to be non-essential both *in vitro* and *in vivo* for infectious virus formation (Kelly and Lewis 1973, Kapoor et al., 1981, Morin et al., 1987, Lubeck et al., 1989). Numerous recombinant HAVs have therefore been generated by replacement of part of the E3 region (Morin et al., 1987, Chengalvala et al., 1991, 1994, Prevec et al., 1989, Johnson et al., 1988, Lubeck et al., 1989, Dewar et al., 1989, Natuk et al., 1993, Hsu et al., 1994).

However, since proteins encoded by the E3 region have been shown to alter various aspects of the host immune responses (for a review see Wold and Gooding 1991), E3 deletion may have some impact on the pathogenic profile of corresponding recombinant viruses. Indeed, it has been demonstrated in a cotton rat model that deletion of the E3 region from HAV serotype 5 increases virus pulmonary pathogenicity (Ginsberg et al., 1989). However, it has also been demonstrated that a recombinant bovine Ad3, partially deleted within its E3 region, produces lesions in cotton rats similar to those observed with the parental wt bovine Ad3, therefore suggesting that safety of bovine Ad3-based vectors may be sufficient for the derivation of live recombinant virus vaccines for cattle (Mittal et al., 1996). These results also show that the impact of deletions within the E3 region of any specific adenovirus should be considered on a case-by-case approach.

The CAV2 E3 region has been identified and characterized previously (Linne, 1992). However, based on the available published data (Linne 1992), the precise definition of an insertion site in the CAV2 E3 region is not obvious. DNA sequence analysis revealed that the organization of the CAV2 E3 region differs significantly from that described for HAVs. The human adenovirus E3 region corresponds to a stretch of at least 3 kbp containing at least 8 open reading frames (orf) whereas the CAV2 E3 region is only 1.5 kbp long and contains only 3 orfs. None of these orfs have a significant level of homology with HAV E3 orfs. From such preliminary comparative analyses, it appears reasonable to speculate that human and canine adenoviruses genomes have evolved differently.

The definition of an insertion site within the CAV2 E3 region is further complicated by the complex splicing and polyadenylation pattern which characterizes the adenovirus family (for a review Imperiale et al., 1995). RNA splicing donor and aceptor sites localized within the E3 region may be important for the maturation of several essential mRNAs even though their coding sequences are localized outside of the E3 region.

Further, since the E3 region is located within a genome region of high transcriptional activity (for a review Sharp et al., 1984), the insertion of foreign DNA at this site has a potential detrimental impact on the biology of the recombinant virus. Additionally, the E3 region is located downstream of the major late promoter (MLP), where interference between transcription of recombinant gene and transcription initiated at the MLP has been demonstrated (Zu et al., 1995).

Problems in the art to be addressed therefore include: minimizing phenotypic alterations of the recombinant virus, and the definition of an insertion site in a less transcriptionnally active region. And, in general, it can be said that the E3 region presents problems in the art which should be addressed.

The less explored third group of recombinant HAVs is based on the insertion of recombinant DNA between the right inverted terminal repeat (ITR) and the E4 promoter. The ITRs contain sequences which are essential for viral DNA replication and efficient packaging of the viral genomic DNA. While a region between the right inverted terminal repeat (ITR) and the E4 promoter may accommodate exogenous DNA sequences (Saito et al., 1985, Chanda et al., 1990), adenoviruses-based vectors have severe limitations in the amount of foreign DNA they can carry, as the packaging capacity of recombinant hAd5 is limited to a genome of approximatively 105% of the wild-type genome (Bett et al. 1993); thus presenting a problem in the art.

While the region between the right ITR and the E4 region may represent an additional insertion site candidate for the generation of CAV2 recombinant viruses, and PCT WO 91/11525 may relate to a SmaI site close to the leftward extremity of the ITR as a potential insertion site. Contrary to the teachings of WO91/11525, there appears to be an upper limit for insertion at this site as Applicant attempted insertions at this site and was able to insert a 400 bp DNA fragment, but larger insertions such as a fragment approximately 1 kbp repeatedly failed to be introduced into the site. Hence, a problem in the art is the utility of this site.

Therefore, the E4 promoter region has heretofore had deficiencies and presented problems.

Initial characterization of the CAV2 genome at the molecular level has been described in the literature. Restriction analysis of several strains of both CAV2 and CAV1 (Jouvenne et al., 1987, Macartney et al., 1988, Spibey and Cavanagh 1989) and sequence analysis of the corresponding E1, E3 and ITRs regions have been reported (Cavanagh et al., 1991, Linne 1992). Although the overall genomic organization of canine adenoviruses is similar to those described for other *Adenoviridae* family members, the precise organisation of CAV2 genomic E3 region is unique.

Accordingly, one cannot merely extrapolate from one member to another member of the Adenoviridae family, thereby providing yet another problem in the art.

Further still, when addressing any or all of the aforementioned deficiencies or problems, it would be preferred to avoid any dependence on an endogenous promoter like the E3 or the MLP promoters. However, the pattern of expression of the recombinant gene may be a critical parameter in the overall expression and ergo in the efficacy of the recombinant in a vaccine or immunological composition (Darteil et al., 1995, Xu et al., 1995, Hooft van Iddekinge et al., 1996).

Several cellular and viral promoters have been involved in the derivation of recombinant HAVs. Among the best characterized are b-actin, SV40 early, SV40 late, hAD MLP, and hCMV-IE (Zu et al., 1995). The hCMV-IE promoter may have promise as an upstream regulatory region, since it is associated with the highest level and the longest persistence of recombinant protein expression in tissue culture. This promoter also appears to operate in almost every cell line tested thus far. A potential for cell type independent promoter activity can be regarded as a clear advantage.

It has been demonstrated that the hCMV-IE promoter can be transactivated by HAV infection (Gorman et al., 1989). The large size of this promoter (approximately 850 bp) is a problem with respect to the size limitations of recombinant CAV vector. Thus, one cannot merely extrapolate from past successes with this promoter to a recombinant CAV vector.

Adenoviruses are known to strongly repress the synthesis of cellular proteins after the onset of viral DNA replication (for a review Zhang and Schneider, 1993). Thus, replication-competent recombinant adenoviruses have heretofore had a potential for a strong limitation of the recombinant protein expression after the onset of DNA replication.

Similarly, Saito et al. (1985) demonstrated that a recombinant human adenovirus serotype 5 can produce high amounts of recombinant mRNA but that almost no recombinant protein is obtained.

Late adenovirus mRNAs are characterized by the presence of a tripartite leader (TPL) sequence in their 5' untranslated region (5'UTR). The presence of the TPL can be an important component of the translatability of late adenovirus mRNAs. Further, it has been demonstrated that in an hAd5 background, the presence of the TPL is a feature of the translational control of a recombinant SV40 T antigen expressed from adenovirus late promoter (Thummel et al. 1983).

Another important problem to address in the design of an expression cassette is the size of the polyadenylation signal. Even still further, the problems in the art include establishing conditions to transfect CAV2 DNA into monolayers. The infectivity of purified naked adenovirus DNA is low. Using a calcium phosphate-based procedure, Graham and Van der Berg (1973) report a yield of 1 pfu/mg of purified DNA. This is not an efficient process for isolating recombinant viruses. Several approaches have been proposed to attempt to address this problem; but, none heretofore have fully addressed the problem, and particularly without raising additional issues such as safety.

For instance, DNA protein complexes have been purified and are reported to have an increased infectivity (5x10³ pfu/mg) (Sharp et al., 1976) over naked DNA. Similarly, covalently closed circles of adenovirus DNA have also been shown to be infectious (Graham, 1984).

A widely used procedure to derive recombinant HAVs is based on the utilization of the 293 cell line which has been transformed with the HAV E1 region (Graham et al., 1977). Previously, it has been reported that the derivation of bovine and canine adenovirus recombinants was dependent on the utilization of cell lines transformed with the corresponding adenovirus E1 region (PCT WO 91/11525, Mittal et al. , 1995a). However, since the genes encoded by the E1 region of some adenoviruses have been shown to contribute to the transformation of rodent cells (reviewed by Grand, 1987), the safety profile of E1 transformed cell line appear questionable. The presence of potent transactivators within the adenovirus E1 region (for a review Nevins, 1993) is also well established and further extends safety concerns which can be raised regarding E1 transformed cell lines.

Thus, transfection conditions independent of use of an E1 transformed cell line, especially with good yields, would be a significant advance in the art.

Accordingly, it is believed that a recombinant CAV, preferably a recombinant CAV2, having exogenous DNA inserted therein and a non-essential region or portion thereof deleted therefrom, especially such a CAV which is packaged as an infectious CAV with respect to cells in which CAV naturally replicates, or a CAV containing exogenous DNA within the E3 and/or the right end of the genome between the right ITR and the E4 transcription unit, and methods for making such recombinants, and uses for such recombinants, as described herein (above and below), has not been taught or suggested. Further, it is believed that a truncated transcriptionally active promoter for a recombinant virus or plasmid which comprises a region transactivated with a transactivating protein provided by the virus or a system into which the plasmid is inserted and the minimal promoter region of the promoter, an expression cassette comprising the promoter, and viruses or plasmids containing the promoter or expression cassette, have not been heretofore described or suggested. And, such a recombinant CAV and methods of making and using such a recombinant CAV, and such a promoter, expression cassette and viruses and plasmids containing the promoter or expression cassette present an advancement over prior recombinants, especially since as to humans CAV is a non-replicating vector and the promoter and expression cassette address insert size limits of recombinant viruses.

### OBJECTS AND SUMMARY or THE INVENTION

The present invention provides a canine adenovirus 2 (CAV2) synthetically modified to contain therein exogenous DNA, wherein the E3 region or a portion thereof has been deleted from the CAV2, and wherein exogenous DNA is present in a region located between the right ITR and the E4 region, or wherein the exogenous DNA is inserted into both the CAV2 E3 region and a region located between the right ITR and the E4 transcription unit and which is packaged as an infectious CAV2 with respect to cells in which CAV2 naturally replicates.

In another aspect, the present invention provides a vector for expression of heterologous DNA comprising the CAV2 of the present invention.

In a further aspect, the present invention provides a vector for cloning heterologous DNA comprising the CAV2 of the present invention.

The present invention provides, in a further aspect, an immunogenic, immunological or vaccine composition containing the virus of the present invention, and a pharmaceutically acceptable carrier or diluent.

The present invention provides, in another aspect, an immunogenic, immunological or vaccine composition containing the vector of the present invention and a pharmaceutically acceptable carrier or diluent.

In another aspect, the present invention provides a therapeutic composition containing the vector of the present invention and a pharmaceutically acceptable carrier or diluent.

The present invention provides, in another aspect, a method of expressing a protein or gene product or an expression product which comprises infecting or transfecting a cell *in vitro* with a virus as defined in the present invention.

The present invention provides, in a further aspect, a method of expressing a protein or gene product or an expression product which comprises infecting or transfecting a cell *in vitro* with a vector as defined in the present invention.

In a further aspect, the present invention provides a method of cloning a heterologous DNA sequence comprising infecting or transfecting a cell *in vitro* with a virus as defined in the present invention.

Additionally, the present invention provides a method for cloning a heterologous DNA sequence comprising infecting or transfecting a cell *in vitro* with a vector as defined in the present invention.

Further, the present invention provides an *in vitro* method for preparing the virus of the present invention comprising inserting the exogenous DNA into a non-essential region of the CAV2 genome.

In addition, the present invention provides an immunogenic, immunological or vaccine composition according to the present invention for use in vaccination.

The description mentions a recombinant adenovirus, preferably a recombinant canine adenovirus (CAV), such as a recombinant canine adenovirus-2 (CAV2).

The description also mentions a recombinant which contains exogenous DNA, preferably in a non-essential region, and which has had a non-essential region of the CAV genome, or a portion thereof, deleted therefrom; and, preferably to provide such a recombinant which is packaged as an infectious CAV with respect to cells in which GAV naturally replicates.

The description mentions a recombinant CAV containing exogenous DNA wherein the exogenous DNA is inserted into the E3 or both the E3 and the region located between the right ITR and the E4 transcription unit.

The description mentions a transcritionally active truncated promoter, an expression cassette containing the promoter, and viruses and plasmids containing the promoter or the expression cassette; including to provide such an expression cassette containing a truncated polyadenylation signal.

Further objects of the description include any or all of: to provide expression products from such recombinants, methods for expressing products from such recombinants, compositions containing the recombinants or the expression products, methods for using the expression products, methods for using the compositions, DNA from the recombinants, and methods for replicating DNA from the recombinants.

Another object of the description is an adenovirus-based, e.g., CAV-based, preferably CAV2-based, vector, or compositions containing the vector, or methods for making or using the vector with consideration of any, any combination, or all, of the earlier-discussed deficiencies and/or problems in the art.

Accordingly, the description provides a CAV synthetically modified to contain therein exogenous DNA, wherein a non-essential region of the CAV genome or a portion thereof has been deleted from the CAV. The CAV is preferably packaged as an infectious CAV with respect to cells in which CAV naturally replicates. Any non-essential region or portion thereof can be deleted from the CAV genome, and the viability and stability of the recombinant CAV resulting from the deletion can be used to ascertain whether a deleted region or portion thereof is indeed non-essential. The non-essential region of the CAV genome or portion thereof deleted from the CAV is preferably the E3 region or a portion thereof. The exogenous DNA is present in any non-essential region (and viability and stability of the recombinant CAV resulting from the insertion of exogenous DNA can be used to ascertain whether a region into which exogenous DNA is inserted is non-essential). The E3 region, the E1 region, the E4 region, or a region located between the right ITR and the E4 region, are presently preferred as non-essential regions for insertion of exogenous DNA into the CAV genome.

Additionally, the description provides a recombinant CAV comprising heterologous DNA in a non-essential region of the CAV genome, wherein the heterologous DNA is in the E3 or both the E3 and the region located between the right ITR and the E4 transcription unit.

The CAV of these embodiments is preferably a CAV2.

The description further provides a vector for cloning or expression of heterologous DNA comprising the recombinant CAV.

The heterologous DNA encodes an expression product comprising: an epitope of interest, a biological response modulator, a growth factor, a recognition sequence, a therapeutic gene, or a fusion protein.

An epitope of interest is an antigen or immunogen or immunologically active fragment thereof from a pathogen or toxin of veterinary or human interest.

An epitope of interest can be an antigen of a veterinary pathogen or toxin, or from an antigen of,a veterinary pathogen or toxin, or another antigen or toxin which elicits a response with respect to the pathogen, of from another antigen or toxin which elicits a response with respect to the pathogen, such as, for instance: as Morbillivirus antigen, e.g., a canine distemper virus or measles or rinderpest antigen such a HA or F; a rabies glycoprotein, e.g., rabies glycoprotein G; an avian influenza antigen, e.g., turkey influenza HA, Chicken/Pennsylvania/1/83 influenza antigen such a nudeoprotein (NP); a bovine leukemia virus antigen, e.g., gp51.30 envelope; a Newcastle Disease Virus (NDV) antigen, e.g., HN or F; a feline leukemia virus antigen (FeLV), e.g., FeLV envelope protein; RAV-1 env; matrix and/or preplomer of infectious bronchitis virus; a Herpesvirus glycoprotein, e.g., a glycoprotein from feline herpesvirus, equine herpesvirus, bovine herpesvirus, pseudorabies virus, canine herpesvirus, or cytomegalovirus; a flavivirus antigen, e.g., a Japanese encephalitis virus (JEV) antigen; an immunodeficiency virus antigen, e.g., a feline immunodeficiency virus (FIV) antigen or a simian immunodeficiency virus (SIV) antigen; a parvovirus antigen, e.g., canine parvovirus; an equine influenza antigen; a Marek's Disease virus antigen; an poxvirus antigen, e.g., an ectromelia antigen, a canarypox virus antigen or a fowlpox virus antigen; or an infectious bursal disease virus antigen, e.g., VP2, VP3, VP4.

An epitope of interest can be an antigen of a human pathogen or toxin, or from an antigen of a human pathogen or toxin, or another antigen or toxin which elicits a response with respect to the pathogen, or from another antigen or toxin which elicits a response with respect to the pathogen, such as, for instance: a Morbillivirus antigen, e.g., a measles virus antigen such as HA or F; a rabies glycoprotein, e.g., rabies virus glycoprotein G; an influenza antigen, e.g., influenza virus HA or N; a Herpesvirus antigen, e.g., a glycoprotein of a herpes simplex virus (HSV), a human cytomegalovirus (HCMV), Epstein-Barr; a flavivirus antigen, a JEV, Yellow Fever virus or Dengue virus antigen; a Hepatitis virus antigen, e.g., HBsAg; an immunodeficiency virus antigen, e.g., an HIV antigen such as gp120, gp160; a Hantaan virus antigen; a *C. tetani* antigen; a mumps antigen; a pneumococcal antigen, e.g., PspA; a *Borrelia* antigen, e.g., OspA, OspB, OspC of *Borrelia* associated with Lyme disease such as *Borrelia burgdorferi, Borrelia afzelli* and *Borrelia garinii*; a chicken pox (varicella zoster) antigen; or a *Plasmodium* antigen.

Of course, the foregoing lists are intended as exemplary, as the epitope of interest can be an antigen of any veterinary or human pathogen or from any antigen of any veterinary or human pathogen.

Since the heterologous DNA can be a growth factor or therapeutic gene, the recombinant CAV can be used in gene therapy. Gene therapy involves transferring genetic information; and, with respect to gene therapy and immunotherapy, reference is made to U.S. Patent No. 5,252,479,and the documents cited in it and to WO 94/16716 and U.S. application Serial No. 08/184,009, filed January 19, 1994 and the documents cited therein.

The growth factor or therapeutic gene, for example, can encode a disease-fighting protein, a molecule for treating cancer, a tumor suppressor, a cytokine, a tumor associated antigen, or interferon; and, the growth factor or therapeutic gene can, for example, be selected from the group consisting of a gene encoding alpha-globin, beta-globin, gamma-globin, granulocyte macrophage-colony stimulating factor, tumor necrosis factor, an interleukin, macrophage colony stimulating factor, granulocyte colony stimulating factor, erythropoietin, mast cell growth factor, tumor suppressor p53, retinoblastoma, interferon, melanoma associated antigen or B7.

The description still further provides an immunogenic, immunological or vaccine composition containing the recombinant CAV virus or vector, and a pharmaceutically acceptable carrier or diluent. An immunological composition containing the recombinant CAV virus or vector (or an expression product thereof) elicits an immunological response - local or systemic. The response can, but need not be, protective. An immunogenic composition containing the recombinant CAV virus or vector (or an expression products thereof) likewise elicits a local or systemic immunological response which can, but need not be, protective. A vaccine composition elicits a local or systemic protective response. Accordingly, the terms "immunological composition" and "immunogenic composition" include a "vaccine composition" (as the two former terms can be protective compositions).

The description therefore also provides a method of inducing an immunological response in a host vertebrate comprising administering to the host an immunogenic, immunological or vaccine composition comprising the recombinant CAV virus or vector and a pharmaceutically acceptable carrier or diluent. For purposes of this specification, "animal" includes all vertebrate species, except humans; and "vertebrate" includes all vertebrates, including animals (as "animal" is used herein) and humans. And, of course, a subset of "animal" is "mammal", which for purposes of this specification includes all mammals, except humans.

For human administration, recombinant CAV, especially CAV2, provides the advantage of expression without productive replication. This thus provides the ability to use recombinants of the description in immunocompromised individuals; and, provides a level of safety to workers in contact with recombinants of the description. Therefore, the the description comprehends methods for amplifying or expressing a protein by administering or inoculating a host with a recombinant CAV virus or vector, e.g., CAV2, whereby the host is not a canine or not a natural host of the recombinant virus or vector, and there is expression without productive replication.

Furthermore, since CAV, and especially CAV2, is used as vaccinial strains in dogs, the present description provides a means for introducing additional epitope(s) of interest of antigen(s) of a canine pathogen(s) or toxin(s) into the vaccinial CAV, e.g., CAV2, strains for a recombinant CAV expressing those additional epitope(s) of interest and thereby providing a means to elicit *in vivo* responses to those epitope(s) of interest and canine adenovirus by inoculating a dog or pup with the vaccinial recombinant CAV. The additional epitope(s) of interest can be an antigen of a canine pathogen (other than adenovirus) or toxin, from an antigen of a canine pathogen (other than adenovirus) or toxin, another antigen which elicits a response in dogs or pups to the canine pathogen (other than adenovirus) or toxin, or from another antigen which elicits a response in dogs or pups to the canine pathogen (other than adenovirus) or toxin (an example of the latter two epitopes of interest are measles HA and F and epitopes thereon which elicit a protective response against canine distemper virus in dogs or pups; see U.S. Patent No. 5,503,834).

Accordingly the present description provides that the recombinant vaccinial CAV can contain heterologous DNA encoding an epitope of interest, from any antigen of a canine pathogen or toxin, for instance: rabies, canine herpesvirus, canine distemper virus, canine parvovirus and the like. In this regard, reference is made to copending U.S. applications Serial Nos. 08/413,118, filed March 29, 1995 (canine herpesvirus DNA), 08/224,657, filed April 6, 1994 (canine distemper), 08/416,646, filed April 5, 1995 (canine distemper), and 08/486,969, filed June 7, 1995 (rabies combination compositions) and U.S. Patent No. 5,529,780 (canine herpesvirus DNA) and the documents cited therein. Thus, the description envisions CAV recombinants containing exogenous DNA coding for more than one protein, e.g., coding for two or more epitopes such as antigens of canine pathogens. The description also envisions compositions containing CAV recombinants in combination with other antigens.

The description even further provides a therapeutic composition containing the recombinant CAV virus or vector and a pharmaceutically acceptable carrier or diluent. The therapeutic composition is useful in the gene therapy and immunotherapy embodiments of the description, e.g., in a method for transferring genetic information to an animal or human in need of such comprising administering to the host the composition; and, accordingly includes methods for transferring genetic information.

In yet another embodiment, the description provides a method of expressing a protein or gene product or an expression product which comprises infecting or transfecting a cell *in vitro* with a recombinant CAV virus or vector of the description and optionally extracting, purifying or isolating the protein, gene product or expression product or DNA from the cell. And, the description provides a method for cloning or replicating a heterologous DNA sequence comprising infecting or transfecting a cell *in vitro* or *in vivo* with recombinant CAV virus or vector of the description and optionallly extracting, purifying or isolating the DNA from the cell or progeny virus

The description in another aspect provides a method for preparing the recombinant CAV virus or vector of the description comprising inserting the exogenous DNA into a non-essential region of the CAV genome.

The method can further comprise deleting a non-essential region from the CAV genome, preferably prior to inserting the exogenous DNA.

The method can comprise *in vivo* recombination (even though CAV DNA is infectious). Thus, the method can comprise transfecting a cell with CAV DNA in a cell-compatible medium in the presence of donor DNA comprising the exogenous DNA flanked by DNA sequences homologous with portions of the CAV genome, whereby the exogenous DNA is introduced into the genome of the CAV, and optionally then recovering CAV modified by the *in vivo* recombination.

The method can also comprise cleaving CAV DNA to obtain cleaved CAV DNA, ligating the exogenous DNA to the cleaved CAV DNA to obtain hybrid CAV-exogenous DNA, tranfecting a cell with the hybrid CAV-exogenous DNA, and optionally then recovering CAV modified by the presence of the exogenous DNA.

Since *in vivo* recombination is comprehended, the description accordingly also provides a plasmid comprising donor DNA not naturally occurring in CAV encoding a polypeptide foreign to CAV, the donor DNA is within a segment of CAV DNA which would otherwise be co-linear with a non-essential region of the CAV genome such that DNA from a non-essential region of CAV is flanking the donor DNA.

The exogenous DNA can be inserted into CAV to generate the recombinant CAV in any orientation which yields stable integration of that DNA, and expression thereof, when desired.

The exogenous DNA in the recombinant CAV virus or vector of the description can include a promoter. The promoter can be from a herpesvirus. For instance, the promoter can be a cytomegalovirus (CMV) promoter, such as a human CMV (HCMV) or murine CMV promoter.

The promoter is preferably a truncated transcriptionally active promoter which comprises a region transactivated with a transactivating protein provided by the virus and the minimal promoter region of the full-length promoter from which the truncated transcriptionally active promoter is derived. For purposes of this specification, a "promoter" is composed of an association of DNA sequences corresponding to the minimal promoter and upstream regulatory sequences; a "minimal promoter" is composed of the CAP site plus TATA box (minimum sequences for basic level of transcription; unregulated level of transcription); and, "upstream regulatory sequences" are composed of the upstream element(s) and enhancer sequence(s). Further, the term "truncated" indicates that the full-length promoter is not completely present, i.e., that some portion of the full-length promoter has been removed. And, the truncated promoter can be derived from a herpesvirus such as MCMV or HCMV, e.g., HCMV-IE or MCMV-IE.

The promoter can truncated so that there is up to a 40% and even up to a 90% reduction in size, from a full-length promoter based upon base pairs; for instance, with the murine CMV-IE promoter, and HCMV-IE promoter, respectively. Indeed, a truncated promoter of the description can consist essentially of an enhancer region which is transactivated by a transactivating protein provided by a virus or system into which the truncated promoter is inserted, and the minimal promoter. Thus, as little as 60% and even as little as 10% of the original base pairs of the full-length promoter can be present in a promoter of the description.

Given that nature provided so many more base pairs for promoters than now has been discovered necessary, the promoters, and expression cassettes, viruses and plasmids containing the truncated promoters of the description are indeed surprising. Indeed, the promoters of the description obtain superior performance in comparison with full-length promoters, and, without necessarily wishing to be bound by any one particular theory, it is believed that this superior performance is due to the truncation. Further, truncation or promoters addresses the insert size limit problem of recombinant viruses and plasmids, particularly CAV.

Thus, the description even still further provides, a truncated transcriptionally active promoter for a recombinant virus or plasmid which comprises a region transactivated with a transactivating protein provided by the virus or a system into which the plasmid is inserted and the minimal promoter region of a full-length promoter from which the truncated transcriptionally active promoter is derived.

Like the aforementioned promoter, the inventive promoter is preferably a herpesvirus, e.g., a MCMV or HCMV such as MCMV-IE or HCMV-IE promoter; and, there can be up to a 40% and even up to a 90% reduction in size, from a full-length promoter, based upon base pairs.

The description thus also provides an expression cassette for insertion into a recombinant virus or plasmid comprising the truncated transcriptionally active promoter. The expression cassette can further include a functional truncated polyadenylation signal; for instance an SV40 polyadenylation signal which is truncated, yet functional. Considering that nature provided a larger signal, it is indeed surprising that a truncated polyadenylation signal is functional; and, a truncated polyadenylation signal addresses the insert size limit problems of recombinant viruses such as CAV. The expression cassette can also include exogenous or heterologous DNA with respect to tha virus or system into which it is inserted; and that DNA can be exogenous or heterologous DNA as described herein.

Even further surprisingly, the present description provides a recombinant CAV, preferably GAV2, wherein at least one non-essential loci, such as the E3 region, is employed for generation of the recombinant. Based on data derived from HAVs and bovine Ad3, part of this region may be non-essential both *in vitro* and *in vivo* for infectious virus formation and thus can be considered as an insertion region. Accordingly, in an aspect, the present description provides the generation of a CAV E3 deletion or partial deletion mutant (e.g., E3 ORF1 and/or ORF2); and, this mutant additionally demonstrates that the entire CAV E3 region is not necessary in tissue culture and thus can be used as an insertion site in the generation of recombinant CAV. And therefore, the present description encompasses a recombinant CAV wherein endogenous DNA is deleted and/or exogenous DNA introduced in the E3 region; preferably one or more non-essential domains within the E3 region, e.g., ORF2.

A deletion within the E3 region can also provide additional capacity for insertion of heterologous sequences into the CAV genome. For example, such deletions can compensate for the introduction of a large expression cassette into the right end of the genome. In this regard, by the methods herein taught, without undue experimentation, the skilled artisan can readily identify additional non-essential domains, preferably in the E3 region, and additional non-essential regions.

In another aspect, the description surprisingly provides a recombinant CAV, preferably CAV2, wherein deletions within non-essential regions are relative to insertion of heterologous DNA. For instance, deletions within non-essential regions can be substantially similiar, e.g., compensatory, to the insertion of heterologous DNA in another region, such as, without limitation, the E4/right ITR region.

Nucleotide sequence comparisons between the ITRs from various CAV2 strains indicate some variability immediately upstream of the right ITR (Cavanagh et al., 1991, Spibey, 1991). Applicants' engineered a novel and nonobvious insertion site within this region; and therefore, the present description in a further aspect encompasses CAV recombinants having exogenous DNA inserted therein. Further, the E4/right ITR region, as herein demonstrated, can surprisingly accept much larger fragments of heterologous DNA than the previously described SmaI site, further addressing the insert size limit of CAV.

Since the E4/right ITR site is localized in a region of the CAV genome with little transcriptional activity (for a review see Sharp et al., 1984), insertion thereinto does not significantly impact the biology of the CAV recombinant virus.

As discussed above, in an embodiment, the present description provides novel and nonobvious expression cassette(s) for insertion of exogenous DNA into CAV; the cassette(s) comprising appropriate heterologous eukaryotic regulatory sequences. In a preferred embodiment, the description provides expression cassette(s) rationally designed with consideration of packaging limitations and biological characteristics associated with viruses and plasmids such as adenovirus-based vectors. The ability to truncate MCMV and HCMV promoters to as small as an enhancer region which is transactivated with a transactivating protein provided by the virus or system into which the promoter is inserted and the minimal promoter demonstrates that promoters from other eukaryotic viruses, and especially from other herpesviruses, can be similarly truncated, without undue experimentation from this disclosure and the knowledge in the art; and, the description comprehends truncated promoters from such other viruses.

In a more specific aspect, the present description encompasses CAV, preferably CAV2; recombinants comprising the HCMV-IE or MCMV-IB promoter, preferably a truncated promoter therefrom. Preferably, the HCMV-IE or MCMV-IE promoter or a truncated promoter therefrom is transactivated by CAV-induced gene products.

In the aspects of the present description which include a truncated transcriptionally active (or competent) promoter (preferably a truncated transcriptionally active eukaryotic virus promoter such as a herpesvirus promoter, e.g., a HCMV or MCMV promoter), by "active" (or "competent"), the truncated transcriptionally active promoter should exhibit at least 80%, preferably at least 85%, more preferably at least 90%, and most preferably at least 95% of the transcriptional activity of the pristine or full length promoter. Deletion of nucleotides or of portions or of regions of the full length promoter can be done from the herein teachings, without undue experimentatin, for generation of active fragments in addition to those exemplified. The degree truncation, i.e., amount of base pairs deleted, from the original full length or pristine promoter, in terms of percentage, can be any amount up to 90%, so long as the truncated promoter remains "active" or "competent". Thus, a truncated transcriptionally active promoter can be, in terms of base pairs with respect to the full length or pristine promoter, about 5% to about 95%, preferably about 10% to about 90%, more peferably about 10% to about 60% and most preferably about 10% to about 40% of the full length or pristine promoter, with specific embodiments being about 10% and about 40% of the full length or pristine promoter (i.e., deletions from the full length or pristine promoter, in terms of base pairs, of about 95% to about 5%, preferably about 90% to about 10%, more preferably about 90% to about 40%, and most preferably about 90% to about 60% of the base pairs of the full length or pristine promoter, with deletions of about 90% and about 60% of base pairs of the full length or pristine promoter being specific embodiments). Indeed, all that need be retained of the original, full length or pristine promoter, at a minimum, is the minimal promoter and a region which is transactivated with a transactivating protein provided by the virus or system into which the promoter is inserted.

The deletion of portions of a promoter such as the HCMV-IE, is to reduce its size so as to address the deficiencies and/or problems of the size of promoters such as the HCMV-IE promoter and the packing limitations of adenoviruses.

In a particular aspect, the present description provides an active fragment of the HCMV-IE having a size of 91 bp or an active fragment of the MCMV-IE having a size of 466 bp, i.e., a truncated transcriptionally active HCMV-IE of about 91 bp or a truncated transcriptionally active MCMV-IE of about 466 bp. (The present description can encompass HCMV-IE or MCMV-IE fragments having substantial base pair size and/or homology with respect to the 91 bp or 466 bp fragment, e.g., as to base pair size and/or homology, at least 80%, preferably at least 85%, more preferably at least 90%, and most preferably at least 95% of the 91 bp or 466 bp fragment.) The fragment can be inserted into a CAV such as CAV2; and therefore, the description encompasses a recombinant CAV such as CAV2 comprising an active fragment of HCMV-IE or MCMV-IE, i.e., a truncated transcriptionally active promoter derived from HCMV-IE or MCMV-IE, and preferably, the 91 bp or 466 bp fragment or an active fragment having substantial base pair size and/or homology to the 91 bp or 466 bp fragment.

Size reduction considerations for preparing the particular 91 bp or 466 bp fragment, or any other active fragment of the HCMV-IE or MCMV-IE promoter, can, as discussed above, be from the known molecular organization of the HCMV-IE or MCMV-IE promoter (Boshart et al., 1985).

It is surprising that such small versions of the full length or pristine promoter, such as the 91 bp or 466 bp fragment, are still able to be "active" (as the term is discussed above), and even drive an equivalent high level of transcription activity in CAV, particularly CAV2, infected cells as the 850 bp version of HCMV-IE and the 766 bp version of MCMV-IE, respectively.

The 91 bp fragment or an active fragment having substantial base pair size to the 91 bp fragment is especially surprising as it is believed to be the smallest promoter element which has been used in an adenovirus-based recombinant virus.

By following the herein considerations applied to the HCMV-IE and MCMV-IE promoter for generation of "active" fragments thereof, "active" fragments of promoters other than HCMV-IE or MCMV-IE, e.g., from other eukaryotic viruses such as other herpesviruses which are exogenous to adenovirus, e.g., CAV2, can be produced, without undue experimentation; and therefore, the present description provides a fragment of a promoter exogenous to an adenovirus, i.e., a truncated transcriptionally active promoter, which is active like the full length promoter in the adenovirus when introduced into the adenovirus. The adenovirus is preferably CAV such as CAV2.

Thus, in another aspect the present description provides a fragment of the murine CMV-IE (MCMV-IE) promoter (Dorsh-Hasler et al., 1985), i.e., a truncated transcriptionally active promoter derived from MCMV-IE, which is active in adenovirus, e.g., CAV2. Indeed, in adenovirus such as CAV2 infected cells the 466 bp CMV-IRE promoter element exhibits activity like the HCMV-IE 91 bp promoter element.

In yet another aspect, the description provides a promoter which is active in adenovirus, e.g., CAV2, which has extended the translation of recombinant mRNAs into the late phase of the viral cycle; and, recombinants comprising the promoter, as well as compositions comprising the recombinants and methods for making and using the promoter, the recombinants and the compositions. Such a promoter can comprise an HCMV-IE promoter or active fragment thereof wherein the 5'UTR has been replaced with the human Ad2 TPL.

In still another aspect, the description provides an insertion cassette for generating recombinant adenoviruses, e.g., CAV2, and to recombinants comprising the cassette, as well as compositions comprising the recombinants and methods for making and using the cassette, the recombinants and the compositions. This cassette preferably comprises a minimizd polyadenylation sequence ("minimized poly-A"), such as a minimized polyadenylation sequence from SV40 ("minimized SV40 poly-A"). The minimized SV40 poly-A can be any length less than the full length or native or pristine SV40 poly-A to as small as about 153 bp (plus or minus 10%).

It is demonstrated herein that such a minimized SV40 poly-A is still associated with the same high level of steady stable mRNA as the wild-type element in adenovirus, e.g., CAV2, infected cells. The minimized SV40 poly-A cassette can be used to minimize DNA inserted into adenovirus; and, this addresses the capacity deficiencies and problems of adenoviruses. Further, from the minimization of the SV40 polyadenylation signal, other similar sequences can be derived, from other sources, without undue experimentation.

Indeed, it is believed that heretofore an expression cassette having size and components which have been optimized for the expression of a recombinant protein by an adenovirus-based vector has not been described in the literature.

In an even further aspect, the present description provides conditions and ergo methods to transfect purified adenovirus, e.g., CAV, preferably CAV2, DNA into canine monolayers.

In preferred embodiments of the description transfection conditions are independent of the utilization of a E1 transformed canine cell line. This procedure provides good yields, including yields of approximately 5x10³ pfu/ug of purified CAV DNA. And, this procedure avoids the utilization of E1 transformed cells for the derivation and propagation of CAV recombinant viruses, thereby avoiding the safety issues surrounding E1 transformed cells.

The present description thus provides recombinant adenoviruses, preferably CAV, more preferably CAV2, and methods for making and using them, and compositions containing them or expression products from them. Any suitable non-essential region can be used for insertion into the genome or deletion from the genome. Such sites include E4, E1, and E3. Two insertion sites are presently preferred: the first is within the E3 region and the second located between the right ITR and the E4 transcription unit (preferably the SmaI site); the former site or both sites (combined) are preferred. The CAV E3 ORF2, e.g., Cay2 E2 ORF2, is presently most preferred.

The results herein also demonstrate that the CAV E3 is non-essential for replication in tissue culture. This represents the first successful attempt to derive recombinant CAV viruses and thus constitutes a basis for products based upon recombinant CAV such as CAV2, e.g., immunological, antigenic or vaccine compositions containing the recombinant CAV or expression products therefrom.

Accordingly, the present description comprehends a CAV such as CAV2 synthetically modified to contain therein exogenous DNA (DNA not naturally occurring in CAV, or not naturally occurring in CAV at the insertion site) in a non-essential region of the CAV2 genome. The non-essential region is preferably the CAV E3 or both the CAV E3 and the right end of the genome such as the SmaI site.

The description further comprehends antibodies elicited by the inventive compositions and/or recombinants and uses for such antibodies. The antibodies, or the product (epitopes of interest) which elicited them, or monoclonal antibodies from the antibodies, can be used in binding assays, tests or kits to determine the presence or absence of an antigen or antibody.

Flanking DNA used in the description can be from the site of insertion or a portion of the genome adjacent thereto (wherein "adjacent" includes contiguous sequences, e.g., codon or codons, as well as up to as many sequences, e.g., codon or codons, before there is an intervening insertion site).

The exogenous or heterologous DNA (or DNA foreign to CAV, or DNA not naturally occurring in CAV) can be DNA encoding any of the aforementioned epitopes of interest, as listed above. In this regard, with respect to *Borrelia* DNA, reference is made to U.S. Patent No. 5,523,089, WO93/08306, PCT/US92/08697, Molecular Microbiology (1989), 3(4), 479-486, and PCT publications WO 93/04175, and WO 96/06165. With respect to pneumococcal epitopes of interest, reference is made to Briles et al. WO 92/14488, with respect to tumor viruses reference is made to Molecular Biology of Tumour Viruses, RNA TUMOR VIRUSES (Second Edition, Edited by Weiss et al., Cold Spring Harbor Laboratory 1982) (e.g., page 44 *et seq*. - Taxonomy of Retroviruses). With respect to DNA encoding other epitopes of interest, attention is directed to the documents cited in the BACKGROUND OF THE INVENTION, for instance: U.S. Patent Nos. 5,174,993 and 5,505,941 (e.g., recombinant avipox virus, vaccinia virus; rabies glycoprotein (G), gene, turkey influenza hemagglutinin gene, gp51, 30 envelope gene of bovine leukemia virus, Newcastle Disease Virus (NDV) antigen, FelV envelope gene, RAV-1 env gene, NP (nudeoprotein gene of Chicken/Pennsylvania/1/83 influenza virus), matrix and preplomer gene of infectious bronchitis virus; HSV gD; entomopox promoter, *inter alia*), U.S. Patent No. 5,338,683, e.g., recombinant vaccinia virus, avipox virus; DNA encoding Herpesvirus glycoproteins, inter *alia*; U.S. Patent No. 5,494,807 (e.g., recombinant vaccinia, avipox; exogenous DNA encoding antigens from rabies, Hepatitis B; JEV, YF, Dengue, measles, pseudorabies, Epstein-Barrr, HSV, HIV, SIV, EHV, BHV, HCMV, canine parvovirus, equine influenza, FeLV, FHV, Hantaan, *C. tetani*, asian influenza, mumps, NDV, *inter alia*); U.S. Patent No. 5,503,834 (e.g., recombinant vaccinia, avipox, Morbillivirus [e.g., measles F, hemagglutinin, *inter alia*]); U.S. Patent No. 4,722,848 (e.g., recombinant vaccinia virus; HSV tk, glycoproteins [e.g., gB, gD], influenza HA, Hepatitis B [e.g., HBsAg], *inter alia*); U.K. Patent GB 2 269 820 B and U.S. Patent No. 5,514,375 (recombinant poxvirus; flavivirus structural proteins); WO 92/22641 (e.g., recombinant poxvirus; immunodeficiency virus, *inter alia*); WO 93/03145 (e.g., recombinant poxvirus; IBDV, *inter alia*); WO 94/16716 and U.S. application Serial No. 08/184,009, filed January 19, 1994 (e.g., recombinant poxvirus; cytokine and/or tumor associated antigens, *inter alia*); and PCT/US94/06652 (*Plasmodium* antigens such as from each stage of the *Plasmodium* life cycle).

In particular, since the tag and other exogenous DNA had been incorporated into CAV2, as in the recombinants described in the Examples, other exogenous DNA can be incorporated into CAV2. Therefore, instead of the exogenous DNA used to generate vCA1, vCA2, vCA3, vCA4, vCA5, vCA6, vCA7, vCA8, and vCA-CDVF1-@12bp-up-SmaI, the exogenous DNA of the above-listed documents and/or those otherwise cited herein are used to generate additional CAV2 recombinants with the exogenous DNA in regions as in vCA2 through vCA8 and vCA-CDVF1-@12bp-up-SmaI and deletions as in vCA2 through vCA8 and vCA-CDVF1-@12bp-up-SmaI (e.g., insertions in the E3 or at the region between the right ITR and the E4 transcription unit or at both sites and deletions in the E3 region) including recombinants containing coding for multiple antigens, as herein described (including with subfragment promoters, reduced or modified polyadenylation cassettes, and promoters with 5' UTR replaced). Analysis demonstrates expression. Compositions are prepared by admixture with a carrier or diluent for administration to a vertebrate (animal or human) hosts for generating responses, including antibody responses.

The exogenous DNA can include a marker, e.g., a color or light marker. The exogenous DNA can also code for a product which would be detrimental to an insect host such that the expression product can be a pesticide or insecticide. The exogenous DNA can also code for an ahti-fungal polypeptide; and, for information on such a polypeptide and DNA therefor, reference is made to U.S. Patent No. 5,421,839 and the documents cited therein.

In addition, the present description provides a method for mapping a non-essential region in the adenovirus, preferably CAV, e.g., CAV2, genome, comprising preparing donor DNA comprising DNA not naturally occurring in CAV present within a segment of CAV DNA otherwise co-linear with a portion of the CAV genome such that by *in vivo* recombination the donor DNA can be introduced into a region of the CAV genome, introducing said donor DNA into the CAV genome by *in vivo* recombination, recovering recombinants, and determining stability and viability thereof and expression or presence of the DNA not naturally occurring in CAV and/or absence of endogenous CAV DNA in the recombinants, whereby viability and stability of recombinants and expression or presence of the DNA not naturally occurring in CAV and/or absence of endogenous CAV DNA indicates that the region into which the donor DNA was introduced is non-essential. This method is employed in the Examples below. The donor DNA can be marker DNA such that by hybridization one can determine whether it has been incorporated into the genome, e.g., hybridization to the marker DNA or failure to hybridize to endogenous DNA replaced by the marker.

These and other objects and embodiments within the present invention are described or are obvious from the following detailed description.

### BRIEF DESCRIPTION OF DRAWINGS

In the following Detailed Description, reference will be made to the accompanying drawings, incorporated herein by reference, wherein:
Figure 1 shows a complete DNA sequence of pLF027 ((6,995 bp) (SEQ ID NO: 1) CAV2 HindIII A fragment starts at nucleotide #689 and ends at nucleotide #4,725. CAV2 E3 region starts at nucleotide #1,414 and ends at nucleotide #2,945. CAV2 E3 ORF1 starts at nucleotide #8 and ends at nucleotide #346. CAV2 E3 ORF2 starts at nucleotide #384 and ends at nucleotide #1,478. CAV2 E3 ORF3 starts at nucleotide #1,019 and ends at nucleotide #483. The remaining nucleotides correspond to pBSSK+);
Figure 2 shows a restriction map of pLF027;
Figure 3 shows a complete DNA sequence of pLF047A ((6,959 bp) (SEQ ID NO: 2) The 23 bp BlgII/MluI linker starts at nucleotide #1,485 and ends at nucleotide #1,508. The remaining sequences correspond to pLF027);
Figure 4 shows a restriction map of pLF047A;
Figure 5 shows a complete DNA sequence of pLF049A ((7,002 bp) (SEQ ID NO: 3) The 63 bp BlgII/MluI linker starts at nucleotide #2,138 and ends at nucleotide #2,201. The remaining sequences correspond to pLF047A);
Figure 6 shows a restriction map of pLF049A;
Figure 7 shows a complete DNA sequence of pLF086 ((6,581 bp) (SEQ ID NO: 4) The 63 bp BlaII/MluI linker starts at nucleotide #2,295 and ends at nucleotide #2,358. The remaining sequences correspond to pLF047A);
Figure 8 shows a restriction map of pLF086;
Figure 9 shows a complete DNA sequence of pLF056 ((6,196 bp) (SEQ ID NO: 5) CAV2 SalI B fragment starts at nucleotide #1 and ends at nucleotide #3,274. The right ITR (196 bp) starts at nucleotide #3,078 and ends at nucleotide #3,274. The SmaI site is localized at position #3,088. The remaining nucleotides correspond to pBSSK+);
Figure 10 shows a restriction map of pLF056;
Figure 11 shows a complete DNA sequence of pLF061 ((6,503 bp) (SEQ ID NO: 6) The 306 bp heterologous DNA tag starts at nucleotide #3,091 and ends at nucleotide #3,397. The remaining nucleotides correspond to pLF056);
Figure 12 shows a restriction map of pLF061;
Figure 13 shows a complete DNA sequence of pLF022 ((4,504 bp) (SEQ ID NO: 7) The hCMV-IE (145 bp) promoter starts at nucleotide #2 and ends at nucleotide #147. All other nucleotides correspond to pCAT basic sequences and include: the CAT reporter gene which starts at nucleotide #209 and ends at nucleotide #868, the SV40 small t antigen and polyadenylation signal (856 bp) which starts at nucleotide #958 and ends at nucleotide #1,814 and the ampicillin resistance gene which starts at nucleotide #2,467 and ends at nucleotide #3,327);
Figure 14 shows a restriction map of pLF022;
Figure 15 shows a complete DNA sequence of pLF062 ((3,812 bp) (SEQ ID NO: 8) The hCMV-IE (145 bp) promoter starts at nucleotide #2 and ends at nucleotide #147. The CAT reporter gene starts at nucleotide #209 and ends at nucleotide #868. The SV40 polyadenylation signal (241 bp) starts at nucleotide #881 and ends at nucleotide #1,122. The ampicillin resistance gene starts at nucleotide #1,775 and ends at nucleotide #2,635);
Figure 16 shows a restriction map of pLF062; Figure 17 shows a complete DNA sequence of pLF066 ((4,009 bp) (SEQ ID NO: 9) The hCMV-IE (145 bp) promoter starts at nucleotide #2 and ends at nucleotide #147. The Ad2 TPL (202 bp) starts at nucleotide #154 and ends at nucleotide #356. The CAT reporter gene starts at nucleotide #406 and ends at nucleotide #1,065. The SV40 polyadenylation signal (241 bp) starts at nucleotide #1,077 and ends at nucleotide #1,319. The ampicillin resistance gene starts at nucleotide #1,972 and ends at nucleotide #2,832);
Figure 18 shows a restriction map of pLF066; Figure 19 shows a complete DNA sequence of pLF069 ((3,955 bp) (SEQ ID NO: 10) The hCMV-IE (91 bp) promoter starts at nucleotide #2 and ends at nucleotide #93. The Ad2 TPL (202 bp) starts at nucleotide #100 and ends at nucleotide #302. The CAT reporter gene starts at nucleotide #352 and ends at nucleotide #1,011. The SV40 polyadenylation signal (241 bp) starts at nucleotide #1,024 and ends at nucleotide #1,265. The ampicillin resistance gene starts at nucleotide #1,918 and ends at nucleotide #2,778);
Figure 20 shows a restriction map of pLF069;
Figure 21 shows a complete DNA sequence of pLF077 ((3,861 bp) (SEQ ID NO: 11) The hCMV-IE (91 bp) promoter starts at nucleotide #2 and ends at nucleotide #93. The Ad2 TPL (202 bp) starts at nucleotide #100 and ends at nucleotide #302. The CAT reporter gene starts at nucleotide #352 and ends at nucleotide #1,011. The SV40 polyadenylation signal (153 bp) starts at nucleotide #1018 and ends at nucleotide #1,171. The ampicillin resistance gene starts at nucleotide #1,824 and ends at nucleotide #2,684);

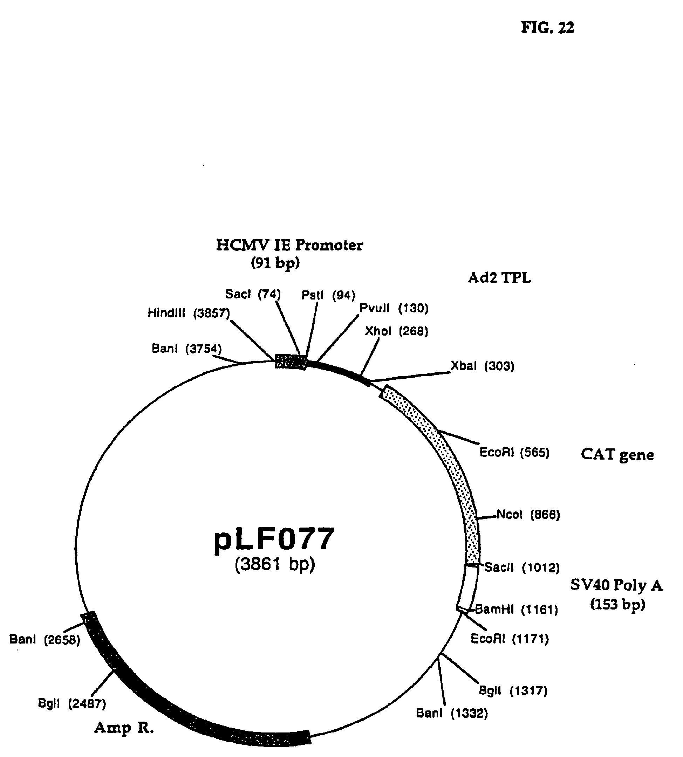

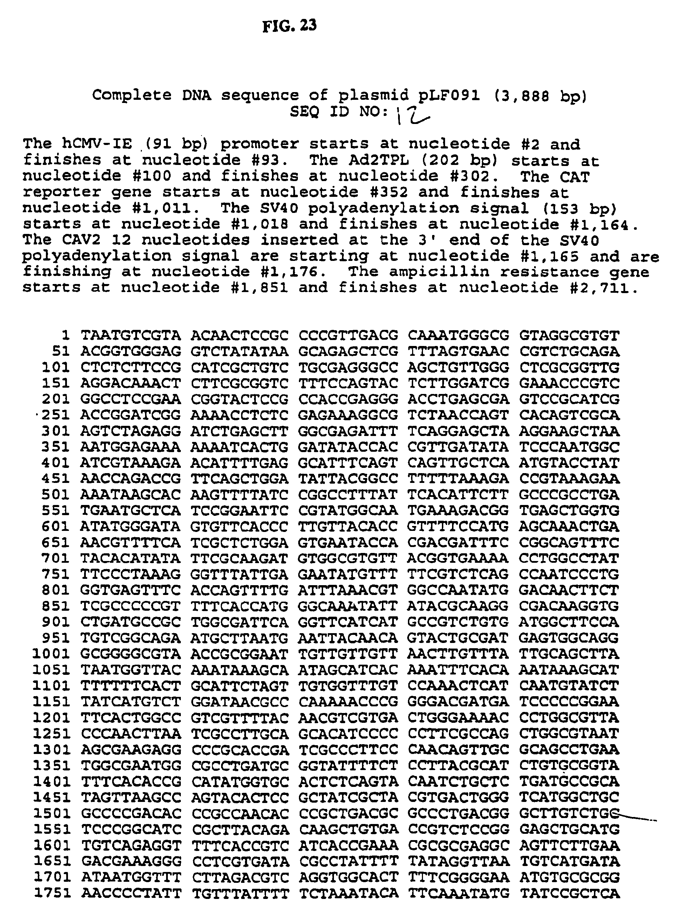

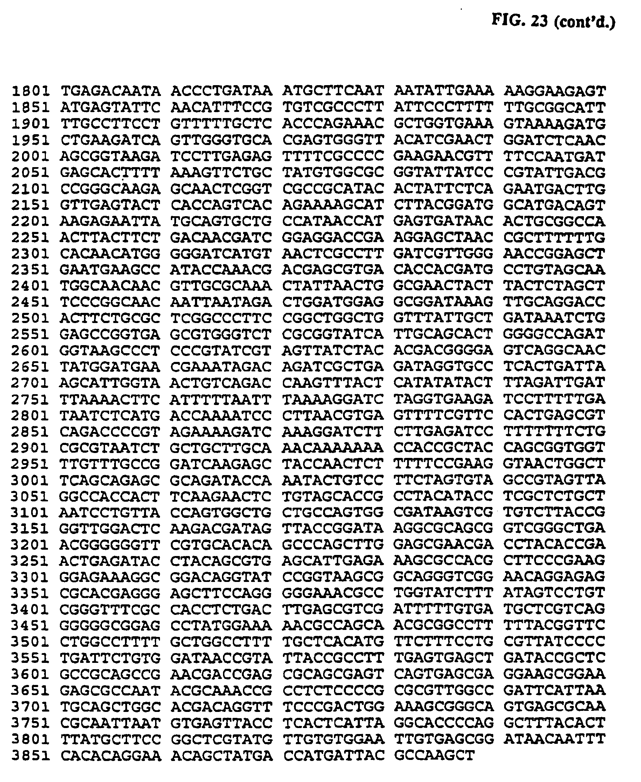

Figure 22 shows a restriction map of pLF077; Figure 23 shows a complete DNA sequence of pLF091 ((3,888 bp) (SEQ ID NO: 12) The hCMV-IE (91 bp) promoter starts at nucleotide #2 and ends at nucleotide #93. The Ad2 TPL (202 bp) starts at nucleotide #100 and ends at nucleotide #302. The CAT reporter gene starts at nucleotide #352 and ends at nucleotide #1,011. The SV40 polyadenylation signal (153 bp) starts at nucleotide #1,018 and ends at nucleotide #1,164. The CAV2 12 nucleotides inserted at the 3' end of the SV40 polyadenylation signal are starting at nucleotide #1,165 and are finishing at nucleotide #1,176. The ampicillin resistance gene starts at nucleotide #1,851 and ends at nucleotide #2,711);
Figure 24 shows a restriction map of pLF091; Figure 25 shows a complete DNA sequence of pLF092 ((7,379 bp) (SEQ ID NO: 13) The CAT expression cassette (as defined in pLF091) starts at nucleotide #1 and ends at nucleotide #1,179. The CAV2 left flanking arm (182 bp) starts at nucleotide #1,180 and ends at nucleotide #1,362. The CAV2 right flanking arm (3,090 bp) starts at nucleotide #4,285 and ends at nucleotide #7,375. The remaining nucleotides corresponds to pBSSK+);
Figure 26 shows a restriction map of pLF092; Figure 27 shows a complete DNA sequence of pLF105 ((6,243 bp) (SEQ ID NO: 14) The polylinker starts at nucleotide #3,092 and ends at nucleotide #3,123. The CAV2 left flanking arm (182 bp) starts at nucleotide #3,123 and ends at nucleotide #3,321. The CAV2 right flanking arm (3,090 bp) starts at nucleotide #1 and ends at nucleotide #3,091. The remaining nucleotides correspond to pBSSK+);
Figure 28 shows a restriction map of pLF105;
Figure 29 shows a complete DNA sequence of pLF102 ((6,615 bp) (SEQ ID NO: 15) The 305 bp BlgII/MluI linker starts at nucleotide #1,471 and ends at nucleotide #1,776. The remaining sequences correspond to pLF086;
Figure 30 shows a restriction map of pLF102);
Figure 31 shows a complete DNA sequence of pLF1116A ((6,450 bp) (SEQ ID NO: 16) The 311 bp MluI/MluI linker starts at nucleotide #1,092 and ends at nucleotide #1,403. The remaining sequences correspond to pLF086);
Figure 32 shows a restriction map of pLF1116A;
Figure 33 shows a complete DNA sequence of pLF100 ((6,247 bp) (SEQ ID NO: 17) The 302 bp DraIII/MluI linker starts at nucleotide #898 and ends at nucleotide #1,200. The remaining sequences correspond to pLF086);
Figure 34 shows a restriction map of pLF100; Figure 35 shows a complete DNA sequence of pLF120 ((6,048 bp) (SEQ ID NO: 18) The 311 bp DraIII/MluI linker starts at nucleotide #898 and ends at nucleotide #1,209. The remaining sequences correspond to pLF086);
Figure 36 shows a restriction map of pLF120;
Figure 37 shows a complete DNA sequence of pLF043 ((5,109 bp) (SEQ ID NO: 19) CDV HA coding sequence starts at nucleotide #35 and ends at nucleotide #2175. CDV HA ORF stop codon is #1847. The partial vaccinia H6 promoter starts at nucleotide #7 and ends at nucleotide #35. The remaining sequences correspond to pBSSK+);
Figure 38 shows a restriction map of pLF043; Figure 39 shows a complete DNA sequence of pLF098 ((5,070 bp) (SEQ ID NO: 20) CDV HA expression cassette starts at nucleotide #1 and ends at nucleotide #2372. The remaining sequences correspond to pLF069);
Figure 40 shows a restriction map of pLF098;
Figure 41 shows a complete DNA sequence of pLF099A ((8,618 bp) (SEQ ID NO: 21) CDV HA expression cassette starts at nucleotide #3120 and ends at nucleotide #5,494. The remaining sequences correspond to pLF105);
Figure 42 shows a restriction map of pLF099A;
Figure 43 shows a complete DNA sequence of pLF108 ((4,965 bp) (SEQ ID NO: 22) the 3' most region of the vaccinia virus H6 promoter is located between position#1 and 29; the CDV F1 coding sequence begins at position#30 and terminates at position#2,018; the remaining sequences correspond to pBSSK+);
Figure 44 shows a restriction map of pLF108;
Figure 45 shows a complete DNA sequence of pLF111 ((5,241 bp) (SEQ ID NO: 23) CDV F1 expression cassette begins at position #1 and terminates at position #2,556; the remaining sequences correspond to pLF069);
Figure 46 shows a restriction map of pLF111;
Figure 47 shows a complete DNA sequence of pLF128 ((5,147 bp) (SEQ ID NO: 24) CDV F1 expression cassette begins at position #1 and terminates at position #2,452; the remaining sequences correspond to pLF077);
Figure 48 shows a restriction map of pLF128;
Figure 49 shows a complete DNA sequence of pLF130A ((8,792 bp) (SEQ ID NO: 25) CDV F1 expression cassette begins at position #3,126 and terminates at nucleotide #5,669; the CAV2 SalI.B left flanking arm (3,091 bp) is located between position #1 and 3,091; the CAV2 SalI.B right flanking arm (182 bp) is located between position #5,688 and 5,870; the remaining sequences correspond to pLF105); and,
Figure 50 shows a restriction map of pLF130A.

### DETAILED DESCRIPTION

As mentioned earlier, the present description relates to recombinant adenovirus, such as CAV, preferably CAV2, methods for making and using them, and to compositions containing them or their expression products; and, to promoters and expression cassettes

More specifically, this description relates to recombinant CAV such as CAV2, especially those wherein exogenous DNA has been inserted into a non-essential region and/or a non-essential region is deleted and methods of making them, uses for them (including as a vector for replicating DNA), expression products from them, and uses for the expression products. The CAV E3 region, preferably ORF2, is preferred for insertion and/or deletion.

The uses for recombinant viruses, and for products therefrom can be determined without undue experimentation from the documents set forth in the BACKGROUND OF THE INVENTION and the discussion under the SUMMARY OF THE INVENTION.

The heterologous or exogenous DNA in recombinants of the description preferably encodes an expression product comprising: an epitope of interest, a biological response modulator, a growth factor, a recognition sequence, a therapeutic gene, or a fusion protein. With respect to these terms, reference is made to the following discussion, and generally to Kendrew, THE ENCYCLOFEDIA OF MOLECULAR BIOLOGY (Blackwell Science Ltd 1995) and Sambrook, Fritsch, Maniatis, Molecular Cloning, A LABORATORY MANUAL (2d Edition, cold Spring Harbor Laboratory Press, 1989).

As to antigens for use in vaccine or immunological compositions, reference is made to the documents and discussion set forth in the BACKGROUND OF THE INVENTION and the discussion under the SUMMARY OF THE INVENTION; see also Stedman's Medical Dictionary (24th edition, 1982, e.g., definition of vaccine (for a list of antigens used in vaccine formulations; such antigens or epitopes of interest from those antigens can be used in the description, as either an expression product of the recombinant virus, or in a multivalent composition containing a recombinant virus or an expression product therefrom).

As to epitopes of interest, one skilled in the art can determine an epitope or immunodominant region of a peptide or polypeptide and ergo the coding DNA therefor from the knowledge of the amino acid and corresponding DNA sequences of the peptide or polypeptide, as well as from the nature of particular amino acids (e.g., size, charge, etc.) and the codon dictionary, without undue experimentation.

A general method for determining which portions of a protein to use in an immunological composition focuses on the size and sequence of the antigen of interest. "In general, large proteins, because they have more potential determinants are better antigens than small ones. The more foreign an antigen, that is the less similar to self configurations which induce tolerance, the more effective it is in provoking an immune response." Ivan Roitt, Essential Immunology, 1988.

As to size: the skilled artisan can maximize the size of the protein encoded by the DNA sequence to be inserted into the viral vector (keeping in mind the packaging limitations of the vector). To minimize the DNA inserted while maximizing the size of the protein expressed, the DNA sequence can exclude introns (regions of a gene which are transcribed but which are subsequently excised from the primary RNA transcript).

At a minimum, the DNA sequence can code for a peptide at least 8 or 9 amino acids long. This is the minimum length that a peptide needs to be in order to stimulate a CD4+ T cell response (which recognizes virus infected cells or cancerous cells). A minimum peptide length of 13 to 25 amino acids is useful to stimulate a CD8+ T cell response (which recognizes special antigen presenting cells which have engulfed the pathogen). See Kendrew, *supra*. However, as these are minimum lengths, these peptides are likely to generate an immunological response, i.e., an antibody or T cell response; but, for a protective response (as from a vaccine composition), a longer peptide is preferred.

With respect to the sequence, the DNA sequence preferably encodes at least regions of the peptide that generate an antibody response or a T cell response. One method to determine T and B cell epitopes involves epitope mapping. The protein of interest "is fragmented into overlapping peptides with proteolytic enzymes. The individual peptides are then tested for their ability to bind to an antibody elicited by the native protein or to induce T cell or B cell activation. This approach has been particularly useful in mapping T-cell epitopes since the T cell recognizes short linear peptides complexed with MHC molecules. The method is less effective for determining B-cell epitopes" since B cell epitopes are often not linear amino acid sequence but rather result from the tertiary structure of the folded three dimensional protein. Janis Kuby, Immunology, (1992) pp. 79-80.

Another method for determining an epitope of interest is to choose the regions of the protein that are hydrophilic. Hydrophilic residues are often on the surface of the protein and are therefore often the regions of the protein which are accessible to the antibody. Janis Kuby, Immunology, (1992) p. 81.

Yet another method for determining an epitope of interest is to perform an X-ray crystallographic analysis of the antigen (full length)-antibody complex. Janis Kuby, Immunology, (1992) p. 80.

Still another method for choosing an epitope of interest which can generate a T cell response is to identify from the protein sequence potential HLA anchor binding motifs which are peptide sequences which are known to be likely to bind to the MHC molecule.

The peptide which is a putative epitope of interest, to generate a T cell response, should be presented in a MHC complex. The peptide preferably contains appropriate anchor motifs for binding to the MHC molecules, and should bind with high enough affinity to generate an immune response. Factors which can be considered are: the HLA type of the patient (vertebrate, animal or human) expected to be immunized, the sequence of the protein, the presence of appropriate anchor motifs and the occurance of the peptide sequence in other vital cells.

An immune response is generated, in general, as follows: T cells recognize proteins only when the protein has been cleaved into smaller peptides and is presented in a complex called the "major histocompatability complex MHC" located on another cell's surface. There are two classes of MHC complexes - class I and class II, and each class is made up of many different alleles. Different patients have different types of MHC complex alleles; they are said to have a 'different HLA type.'

Class I MHC complexes are found on virtually every cell and present peptides from proteins produced inside the cell. Thus, Class I MHC complexes are useful for killing cells which when infected by viruses or which have become cancerous and as the result of expression of an oncogene. T cells which have a protein called CD4 on their surface, bind to the MHC class I cells and secrete lymphokines. The lymphokines stimulate a response; cells arrive and kill the viral infected cell.

Class II MHC complexes are found only on antigen- presenting cells and are used to present peptides from circulating pathogens which have been endocytosed by the antigen- presenting cells. T cells which have a protein called CD8 bind to the MHC class II cells and kill the cell by exocytosis of lytic granules.

Some guidelines in determining whether a protein is an epitopes of interest which will stimulate a T cell response, include: Peptide length - the peptide should be at least 8 or 9 ammino acids long to fit into the MHC class I complex and at least 13-25 amino acids long to fit into a class II MCH complex. This length is a minimum for the peptide to bind to the MHC complex. It is preferred for the peptides to be longer than these lengths because cells may cut the expressed peptides. The peptide should contain an appropriate anchor motif which will enable it to bind to the various class I or class II molecules with high enough specificity to generate an immune response (See Bocchia, M. et al, Specific Binding of Leukemia Oncogene Fusion Protein Peptides to HLA Class I Molecules, Blood 85:2680-2684; Englehard, VH, Structure of peptides associated with class I and class II MHC molecules Ann. Rev. Immunol. 12:181 (1994)). This can be done, without undue experimentation, by comparing the sequence of the protein of interest with published structures of peptides associated with the MHC molecules. Protein epitopes recognized by T cell receptors are peptides generated by enzymatic degradation of the protein molecule and are prestnted on the cell surface in association with class I or class II MHC molecules.

Further, the skilled artisan can ascertain an epitope of interest by comparing the protein sequence with sequences listed in the protein data base. Regions of the protein which share little or no homology are better choices for being an epitope of that protein and are therefore useful in a vaccine or immunological composition. Regions which share great homology with widely found sequences present in vital cells should be avoided.

Even further, another method is simply to generate or express portions of a protein of interest, generate monoclonal antibodies to those portions of the protein of interest, and then ascertain whether those antibodies inhibit growth *in vitro* of the pathogen from which the from which the protein was derived. The skilled artisan can use the other guidelines set forth in this disclosure and in the art for generating or expressing portions of a protein of interest for analysis as to whether antibodies thereto inhibit growth *in vitro*. For example, the skilled artisan can generate portions of a protein of interest by: selecting 8 to 9 or 13 to 25 amino acid length portions of the protein, selecting hydrophylic regions, selecting portions shown to bind from X-ray data of the antigen (full length)-antibody complex, selecting regions which differ in sequence from other proteins, selecting potential HLA anchor binding motifs, or any combination of these methods or other methods known in the art.

Epitopes recognized by antibodies are expressed on the surface of a protein. To determine the regions of a protein most likely to stimulate an antibody response one skilled in the art can preferably perform an epitope map, using the general methods described above, or other mapping methods known in the art.

As can be seen from the foregoing, without undue experimentation, from this disclosure and the knowledge in the art, the skilled artisan can ascertain the amino acid and corresponding DNA sequence of an epitope of interest for obtaining a T cell, B cell and/or antibody response. In addition, reference is made to Gefter et al., U.S. Patent No. 5,019,384, issued May 28, 1991, and the documents it cites (Note especially the "Relevant Literature" section of this patent, and column 13 of this patent which discloses that: "A large number of epitopes have been defined for a wide variety of organisms of interest. Of particular interest are those epitopes to which neutralizing antibodies are directed. Disclosures of such epitopes are in many of the references cited in the Relevant Literature section.")

With respect to expression of a biological response modulator, reference is made to Wohlstadter, "Selection Methods," WO 93/19170, published 30 September 1993, and the documents cited therein.

For instance, a biological response modulator modulates biological activity; for instance, a biological response modulator is a modulatory component such as a high molecular weight protein associated with non-NMDA excitatory amino acid receptors and which allosterically regulates affinity of AMPA binding (See Kendrew, *supra*). The recombinant of the present invnention can express such a high molecular weight protein.

More generally, nature has provided a number of precedents of biological response modulators. Modulation of activity may be carried out through mechanisms as complicated and intricate as allosteric induced quaternary change to simple presence/absence, e.g., expression/degradation, systems. Indeed, the repression/activation of expression of many biological molecules is itself mediated by molecules whose activities are capable of being modulated through a variety of mechanisms.

Table 2 of Neidhardt et al Physiology of the Bacterial Cell (Sinauer Associates Inc., Publishers, 1990), at page 73, lists chemical modifications to bacterial proteins. As is noted in that table, some modifications are involved in proper assembly and other modifications are not, but in either case such modifications are capable of causing modulation of function. From that table, analogous chemical modulations for proteins of other cells can be determined, without undue experimentation.

In some instances modulation of biological functions may be mediated simply through the proper/improper localization of a molecule. Molecules may function to provide a growth advantage or disadvantage only if they are targeted to a particular location. For example, a molecule may be typically not taken up or used by a cell, as a function of that molecule being first degredaded by the cell by secretion of an enzyme for that degradation. Thus, production of the enzyme by a recombinant can regulate use or uptake of the molecule by a cell. Likewise, the recombinant can express a molecule which binds to the enzyme necessary for uptake or use of a molecule, thereby similarly regulating its uptake or use.

Localization targeting of proteins carried out through cleavage of signal peptides another type of modulation or regulation. In this case, a specific endoprotease catalytic activity can be expressed by the recombinant.

Other examples of mechanisms through which modulation of function may occur are RNA virus poly-proteins, allosteric effects, and general covalent and non-covalent steric hindrance. HIV is a well studied example of an RNA virus which expresses non-functional poly-protein constructs. In HIV "the gag, pol, and env poly-proteins are processed to yield, respectively, the viral structural proteins p17, p24, and p15--reverse transcriptase and integrase--and the two envelope proteins gp41 and gp120" (Kohl et al., PNAS USA 85:4686-90 (1988)). The proper cleavage of the poly-proteins is crucial for replication of the virus, and virions carrying inactive mutant HIV protease are non-infectious (Id.). This is another example of the fusion of proteins down-modulating their activity. Thus, it is possible to construct recombinant viruses which express molecules which interfere with endoproteases, or which provide endoproteases, for inhibiting or enhancing the natural expression of certain proteins (by interfering with or enhancing cleavage).

The functional usefulness of enzymes may also be modulated by altering their capability of catalyzing a reaction. Illustrative examples of modulated molecules are zymogens, formation/disassociation of multi-subunit functional complexes, RNA virus poly-protein chains, allosteric interactions, general steric hindrance (covalent and non-covalent) and a variety of chemical modifications such as phosphorylation, methylation, acetylation, adenylation, and uridenylation (see Table 1 of Neidhardt, *supra*, at page 315 and Table 2 at page 73).

Zymogens are examples of naturally occurring protein fusions which cause modulation of enzymatic activity. Zymogens are one class of proteins which are converted into their active state through limited proteolysis. See Table 3 of Reich, Proteases and Biological Control, Vol. 2, (1975) at page 54). Nature has developed a mechanism of down-modulating the activity of certain enzymes, such as trypsin, by expressing these enzymes with additional "leader" peptide sequences at their amino termini. With the extra peptide sequence the enzyme is in the inactive zymogen state. Upon cleavage of this sequence the zymogen is converted to its enzymatically active state. The overall reaction rates of the zymogen are "about 10⁵-10⁶ times lower than those of the corresponding enzyme" (See Table 3 of Reich, *supra* at page 54).

It is therefore possible to down-modulate the function of certain enzymes simply by the addition of a peptide sequence to one of its termini. For example, with knowledge of this property, a recombinant can express peptide sequences containing additional amino acids at one or both terminii.

The formation or disassociation of multi-subunit enzymes is another way through which modulation may occur. Different mechanisms may be responsible for the modulation of activity upon formation or disassociation of multi-subunit enzymes.

Therefore, sterically hindering the proper specific subunit interactions will down-modulate the catalytic activity. And accordingly, the recombinant of the description can express a molecule which sterically hinders a naturally occurring enzyme or enzyme complex, so as to modulate biological functions.

Certain enzyme inhibitors afford good examples of functional down-modulation through covalent steric hindrance or modification. Suicide substrates which irreversibly bind to the active site of an enzyme at a catalytically important amino acid in the active site are examples of covalent modifications which sterically block the enzymatic active site. An example of a suicide substrate is TPCK for chymotrypsin (Fritsch, Enzyme Structure and Mechanism, 2d ed; Freeman & Co. Publishers, 1984)). This type of modulation is possible by the recombinant expressing a suitable suicide substrate, to thereby modulate biological responses (e.g., by limiting enzyme activity).

There are also examples of non-covalent steric hindrance including many repressor molecules. The recombinant can express repressor molecules which are capable of sterically hindering and thus down-modulating the function of a DNA sequence by preventing particular DNA-RNA polymerase interactions.

Allosteric effects are another way through which modulation is carried out in some biological systems. Aspartate transcarbamoylase is a well characterized allosteric enzyme. Interacting with the catalytic subunits are regulatory domains. Upon binding to CTP or UTP the regulatory subunits are capable of inducing a quaternary structural change in the holoenzyme causing down-modulation of catalytic activity. In contrast, binding of ATP to the regulatory subunits is capable of causing up-modulation of catalytic activity (Fritsch, *supra*). Using methods of the description, molecules can be expressed which are capable of binding and causing modulatory quaternary or tertiary changes.

In addition, a variety of chemical modifications, e.g., phosphorylation, methylation, acetylation, adenylation, and uridenylation may be carried out so as to modulate function. It is known that modifications such as these play important roles in the regulation of many important cellular components. Table 2 of Neidhardt, *supra*, at page 73, lists different bacterial enzymes which undergo such modifications. From that list, one skilled in the art can ascertain other enzymes of other systems which undergo the same or similar modifications, without undue experimentation. In addition, many proteins which are implicated in human disease also undergo such chemical modifications. For example, many oncogenes have been found to be modified by phosphorylation or to modify other proteins through phosphorylation or dephosphorylation. Therefore, the ability afforded by the description to express modulators which can modify or alter function, e.g., phosphorylation, is of importance.

From the foregoing, the skilled artisan can use the present description to express a biological response modulator, without any undue experimentation.

With respect to expression of fusion proteins by recombinants, reference is made to Sambrook, Fritsch, Maniatis, Molecular Cloning, A LABORATORY MANUAL (2d Edition, Cold Spring Harbor Laboratory Press, 1989) (especially Volume 3), and Kendrew, *supra*. The teachings of Sambrook et al., can be suitably modified, without undue experiementation, from this disclosure, for the skilled artisan to generate recombinants expressing fusion proteins.

With regard to gene therapy and immunotherapy, reference is made to U.S. Patent Nos. 4,690,915 and 5, 252, 479, together with the documents cited therein it and on their face, and to WO 94/16716 and U.S. application Serial No. 08/184,009, filed January 19, 1994, together with the documents cited therein.

A growth factor can be defined as multifunctional, locally acting intercellular signalling peptides which control both ontogeny and maintenance of tissue and function (see Kendrew, especially at page 455 *et seq*.).

The growth factor or therapeutic gene, for example, can encode a disease-fighting protein, a molecule for treating cancer, a tumor suppressor, a cytokine, a tumor associated antigen, or interferon; and, the growth factor or therapeutic gene can, for example, be selected from the group consisting of a gene encoding alpha-globin, beta-globin, gamma-globin, granulocyte macrophage-colony stimulating factor, tumor necrosis factor, an interleukin (e.g., an interleukin selected from interleukins 1 to 14, or 1 to 11, or any combination thereof), macrophage colony stimulating factor, granulocyte colony stimulating factor, erythropoietin, mast cell growth factor, tumor suppressor p53, retinoblastoma, interferon, melanoma associated antigen or B7. U.S. Patent No. 5,252,479 provides a list of proteins which can be expressed in an adenovirus system for gene therapy, and the skilled artisan is directed to that disclosure. WO 94/16716 and U.S. application Serial No. 08/184,009, filed January 19, 1994, provide genes for cytokines and tumor associated antigens and immunotherapy methods, including ex vivo methods, and the skilled artisan is directed to those disclosures.

Thus, one skilled in the art can create recombinants expressing a growth factor or therapeutic gene and use the recombinants, from this disclosure and the knowledge in the art, without undue experimentation.

Moreover, from the foregoing and the knowledge in the art, no undue experimentation is required for the skilled artisan to construct a recombinant which expresses an epitope of interest, a biological response modulator, a growth factor, a recognition sequence, a therapeutic gene, or a fusion protein; or for the skilled artisan to use such a recombinant.

It is noted that the exogenous or heterologous DNA can itself include a promoter for driving expression in the recombinant CAV, or the exogenous DNA can simply be coding DNA and appropriately placed downstream from an endogenous promoter to drive expression. Further, multiple copies of coding DNA or use of a strong or early promoter or early and late promoter, or any combination thereof, can be done so as to amplify or increases expression. Thus, the exogenous or heterologous DNA can be suitably positioned with respect to an endogenous promoter like the E3 or the MLP promoters, or those promoters can be translocated to be inserted at another location, with the exogenous or heterologous DNA. The coding DNA can be DNA coding for more than one protein so as to have expression of more than one product from the recombinant CAV.

The expression products can be antigens, immunogens or epitopes of interest; and therefore, the description further relates to immunological, antigenic or vaccine compositions containing the expression products. Further, since the CAV vector, in certain instances, can be administered directly to a suitable host, the description relates to compositions containing the CAV, preferably CAV2, vector. Additionally, since the expression product can be isolated from the CAV, preferably CAV2, vector *in vitro* or from cells infected or transfected by the CAV vector *in vitro,* the description relates to methods for expressing a product, e.g., comprising inserting the exogenous DNA into a CAV as a vector, e.g., by restriction/ligation or by recombination followed by infection or transfection of suitable cells *in vitro* with a recombinant CAV, and optionally extracting, purifying or isolating the expression product from the cells. Any suitable extraction, purification or isolation techniques can be employed; and reference is made to the discussion and documents in the BACKGROUND OF THE INVENTION and SUMMARY OF THE INVENTION.

In particular, after infecting cells with the recombinant CAV, the protein(s) from the expression of the exogenous DNA are collected by known techniques such as chromatography (see Robbins, EPA 0162738A1; Panicali, EPA 0261940A2); Richardson, supra; Smith et al., supra; Pennock et al., supra; EP Patent Publication No. 0265785). The collected protein(s) can then be employed in a vaccine, antigenic or immunological composition which also contains a suitable carrier.

Thus, the recombinant CAV can be used to prepare proteins such as antigens, immunogens, epitopes of interest, etc. which can be further used in immunological, antigenic or vaccine compositions. It is noted that a recombinant CAV expressing a product detrimental to growth or development of insects can be used to prepare an insecticide, and a recombinant CAV expressing a product detrimental to growth of plants can be used to prepare a herbicide (by isolating the expression product and admixing it with an insecticidally or herbicidally acceptable carrier or diluent) and a recombinant CAV expressing an anti-fungal polypeptide can be used to prepare an anti-fungal preparation (by isolating the expression product and admixing it with a suitable carrier or diluent).

As the expression products can provide an antigenic, immunological or protective (vaccine) response, the description further relates to products therefrom; namely, antibodies and uses thereof. More in particular, the expression products can elicit antibodies. The antibodies can be formed into monoclonal antibodies; and, the antibodies or expression products can be used in kits, assays, tests, and the like involving binding, so that the description relates to these uses too. Additionally, since the recombinants of the description can be used to replicate DNA, the description relates to recombinant CAV as a vector and methods for replicating DNA by infecting or transfecting cells with the recombinant and harvesting DNA therefrom. The resultant DNA can be used as probes or primers or for amplification.

The administration procedure for recombinant CAV or expression product thereof, compositions of the description such as immunological, antigenic or vaccine compositions or therapeutic compositions can be via a parenteral route (intradermal, intramuscular or subcutaneous). Such an administration enables as systemic immune response. The administration can be via a mucosal route, e.g., oral, nasal, genital, etc. Such an administration enables a local immune response.

More generally, the antigenic, immunological or vaccine compositions or therapeutic compositions (compositions containing the CAV, preferably CAV2, recombinants of the description or expression products) can be prepared in accordance with standard techniques well known to those skilled in the pharmaceutical or vetinary arts. Such compositions can be administered in dosages and by techniques well known to those skilled in the medical arts taking into consideration such factors as the breed or species, age, sex, weight, and condition of the particular patient, and the route of administration. The compositions can be administered alone, or can be co-administered or sequentially administered with other compositions of the description or with other immunological, antigenic or vaccine or therapeutic compositions. Such other compositions can include purified native antigens or epitopes or antigens or epitopes from the expression by a recombinant CAV or another vector system; and are administered taking into account the aforementioned factors.

Examples of compositions of the description include liquid preparations for orifice, e.g., oral, nasal, anal, genital, e.g., vaginal, etc., administration such as suspensions, syrups or elixirs; and, preparations for parenteral, subcutaneous, intradermal, intramuscular or intravenous administration (e.g., injectable administration) such as sterile suspensions or emulsions. In such compositions the recombinant may be in admixture with a suitable carrier, diluent, or excipient such as sterile water, physiological saline, glucose or the like.

Antigenic, immunological or vaccine compositions typically can contain an adjuvant and an amount of the recombinant CAV or expression product to elicit the desired response. In human applications, alum (aluminum phosphate or aluminum hydroxide) is a typical adjuvant. Saponin and its purified component Quil A, Freund's complete adjuvant and other adjuvants used in research and veterinary applications have toxicities which limit their potential use in human vaccines. Chemically defined preparations such as muramyl dipeptide, monophosphoryl lipid A, phospholipid conjugates such as those described by Goodman-Snitkoff et al. J. Immunol. 147:410-415 (1991) encapsulation of the protein within a proteoliposome as described by Miller et al., J. Exp. Med. 176:1739-1744 (1992) and encapsulation of the protein in lipid vesicles such as Novasome^{™} lipid vesicles (Micro Vescular Systems, Inc., Nashua, NH) can also be used.

The composition may be packaged in a single dosage form for immunization by parenteral (i.e., intramuscular, intradermal or subcutaneous) administration or orifice administration, e.g., perlingual (i.e., oral), intragastric, mucosal including intraoral, intraanal, intravaginal, and the like administration. And again, the effective dosage and route of administration are determined by the nature of the composition, by the nature of the expression product, by expression level if recombinant CAV2 is directly used, and by known factors, such as breed or species, age, sex, weight, condition and nature of host, as well as LD₅₀ and other screening procedures which are known and do not require undue experimentation. Dosages of expressed product can range from a few to a few hundred micrograms, e.g., 5 to 500 µg. The recombinant can be administered in any suitable amount to achieve expression at these dosage levels. The vaccinal CDV2 is administered in an amount of about 10^{3.5} pfu; thus, the recombinant is preferably administered in at least this amount; more preferably about 10⁴ pfu to about 10⁶ pfu. Other suitable carriers or diluents can be water or a buffered saline, with or without a preservative. The expression product or recombinant CAV may be lyophilized for resuspension at the time of administration or can be in solution.

The carrier may also be a polymeric delayed release system. Synthetic polymers are particularly useful in the formulation of a composition having controlled release. An early example of this was the polymerization of methyl methacrylate into spheres having diameters less than one micron to form so-called nano particles, reported by Kreuter, J., Microcapsules and Nanoparticles in Medicine and Pharmacology, M. Donbrow (Ed). CRC Press, p. 125-148.

Microencapsulation has been applied to the injection of microencapsulated pharmaceuticals to give a controlled release. A number of factors contribute to the selection of a particular polymer for microencapsulation. The reproducibility of polymer - synthesis and the microencapsulation process, the cost of the microencapsulation materials and process, the toxicological profile, the requirements for variable release kinetics and the physicochemical compatibility of the polymer and the antigens are all factors that must be considered. Examples of useful polymers are polycarbonates, polyesters, polyurethanes, polyorthoesters and polyamides, particularly those that are biodegradable.

A frequent choice of a carrier for pharmaceuticals and more recently for antigens is poly (d,1-lactide-co-glycolide) (PLGA). This is a biodegradable polyester that has a long history of medical use in erodible sutures, bone plates and other temporary prostheses where it has not exhibited any toxicity. A wide variety of pharmaceuticals including peptides and antigens have been formulated into PLGA microcapsules. A body of data has accumulated on the adaption of PLGA for the controlled release of antigen, for example, as reviewed by Eldridge, J.H., et al. Current Topics in Microbiology and Immunology, 1989, 146:59-66. The entrapment of antigens in PLGA microspheres of 1 to 10 microns in diameter has been shown to have a remarkable adjuvant effect when administered orally. The PLCA microencapsulation process uses a phase separation of a water-in-oil emulsion. The compound of interest is prepared as an aqueous solution and the PLGA is dissolved in a suitable organic solvents such as methylene chloride and ethyl acetate. These two immiscible solutions are co-emulsified by high-speed stirring. A non-solvent for the polymer is then added, causing precipitation of the polymer around the aqueous droplets to form embryonic microcapsules. The microcapsules are collected, and stabilized with one of an assortment of agents (polyvinyl alcohol (PVA), gelatin, alginates, polyvinylpyrrolidone (PVP), methyl cellulose) and the solvent removed by either drying in vacuo or solvent extraction.

Thus, solid, including solid-containing-liquid, liquid, and gel (including "gel caps") compositions are envisioned. Additionally, the vectors, e.g., recombinant CAV2, and the expression products therefrom can stimulate an immune or antibody response in animals. From those antibodies, by techniques well-known in the art, monoclonal antibodies can be prepared and, those monoclonal antibodies, can be employed in well known antibody binding assays, diagnostic kits or tests to determine the presence or absence of antigen(s) and therefrom the presence or absence of the natural causative agent of the antigen or, to determine whether an immune response to that agent or to the antigen(s) has simply been stimulate.

Monoclonal antibodies are immunoglobulin produced by hybridoma cells. A monoclonal antibody reacts with a single antigenic determinant and provides greater specificity than a conventional, serum-derived antibody. Furthermore, screening a large number of monoclonal antibodies makes it possible to select an individual antibody with desired specificity, avidity and isotope. Hybridoma cell lines provide a constant, inexpensive source of chemically identical antibodies and preparations of such antibodies can be easily standardized. Methods for producing monoclonal antibodies are well known to those of ordinary skill in the art, e.g., Koprowski, H. et al., U.S. Pat. No. 4,196,265, issued Apr. 1, 1989.

Uses of monoclonal antibodies are known. One such use is in diagnostic methods, e.g., David, G. and Greene, H., U.S. Pat. No. 4,376,110, issued Mar. 8, 1983.

Monoclonal antibodies have also been used to recover materials by immunoadsorption chromatography, e.g. Milstein, C., 1980, scientific American 243:66, 70.

Furthermore, the recombinant CAV or expression products therefrom can be used to stimulate a response in cells *in vitro* or *ex vivo* for subsequent reinfusion into a patient. If the patient is seronegative, the reinfusion is to stimulate an immune response, e.g., an immunological or antigenic response such as active immunization. In a seropositive individual, the reinfusion is to stimulate or boost the immune system against a pathogen.

The recombinant CAV of the description are also useful for generating DNA for probes or for PCR primers which can be used to detect the presence or absence of hybridizable DNA or to amplify DNA, e.g., to detect a pathogen in a sample or for amplifying DNA.

Furthermore, as discussed above, the description comprehends promoters and expression cassettes which are useful in adenovirus systems, as well as in any viral or cell system which provides a transactivating protein. The promoter is preferably a truncated transcriptionally active promoter for a recombinant virus or plasmid which comprises a region transactivated with a transactivating protein provided by the virus or a system into which the plasmid is inserted and the minimal promoter region of a full-length promoter from which the truncated transcriptionally active promoter is derived.

Like the promoter is preferably a derived from a eukaryotic virus such as a herpesvirus, e.g., a MCMV or HCMV such as MCMV-IE or HCMV-IE promoter; and, there can be up to a 40% and even up to a 90% reduction in size, from a full-length promoter, based upon base pairs.

The expression cassette of the description can further include a functional truncated polyadenylation signal; for instance an SV40 polyadenylation signal which is truncated, yet functional. The expression cassette can contain exogenous or heterologous DNA (with respect to the virus or system into which the promoter or expression cassette is being inserted); for instance exogenous or heterologous coding DNA as herein described above, and in the Examples. This DNA can be suitably positioned and operably linked to the promoter for expression. The expression cassette can be inserted in any orientation; preferably the orientation which obtains maximum expression from the system or virus into which the expression cassette is inserted.

While the promoter and expression cassette are specifically exemplified with reference to adenoviruses, the skilled artisan can adapt these embodiments of the description to other viruses and to plasmids for cells such as eukaryotic cells, without undue experimentation, by simply ascertaining whether the virus, plasmid, cell or system provides the transactivating protein.

As to HCMV promoters, reference is made to U.S. Patents Nos. 5,168,062 and 5,385,839. As to transfecting cells with plasmid DNA for expression therefrom, reference is made to Felgner et al. (1994), J. Biol. Chem. 269, 2550-2561. And, as to direct injection of plasmid DNA as a simple and effective method of vaccination against a variety of infectious diseases reference is made to Science, 259:1745-49, 1993, It is therefore within the scope of this description that the promoter and expression cassette be used in systems other than adenovirus; for example, in plasmids for the direct injection of plasmid DNA.

Other utilities also exist for embodiments of the description.

The following non-limiting Examples are given by way of illustration only and are not to be considered a limitation of this invention.

### EXAMPLES

### EXAMPLE 1 - Virus and cell line identifications

The described stock of canine adenovirus type 2 (CAV2) was produced at Rhone Merieux Inc. (Athens, Ga) under the reference CAV2 Lot # 0830 pool - 033093, with a titer of 10^{7.4} TCID₅₀/ml. Madin and Darby canine kidney (MDCK) cell line was also provided by Rhone Merieux Inc. CAV2 is commercially available from Rhone Merieux Inc. as a canine vaccine.

### EXAMPLE 2 - Virus culture and cloning

MDCK cell suspensions were seeded in MEM (Gibco, Grand Island, NY) supplemented with 7.5% fetal bovine serum (Sigma, St Louis, MO), sodium pyruvate (Gibco, 1 mM final), glutamine (Gibco, 2 mM final), penicillin (Gibco, 50 U/ml), streptomycin (Gibco, 50 mg/ml) and non essential amino acids (NEA)(Gibco, 0.1 mM final) and cultured at 37^{itch} in 5% CO₂. Confluent MDCK cells were infected with serial dilutions of CAV2 and cultured under a 0.6% agarose overlay at 37^{itch} in 5% CO₂. CAV2 was subjected to several rounds of plaque purification. A plaque purified CAV2 was amplified in a T25 MDCK flask. When the culture CPE was complete, infected cells were collected and their CAV2 content was titrated on MDCK cell monolayers under agarose. The virus stock was further amplified by infecting a confluent T175 MDCK flask with a multiplicity of infection (MOI) of 0.1. The titre of the T175 MDCK flask amplified virus was established to be 10⁸ p.f.u/ml.

### EXAMPLE 3 - Viral DNA purification

Roller bottles containing confluent MDCK cell monolayers (10⁸ cells/bottle) were infected at a MOI of 0.1 pfu/cell with plaque purified CAV2 virus. Three days later the infected monolayer were harvested and subjected to low speed centrifugation (1K g, 15 minutes, 15°C). The cell pellets were stored at -70°C. The frozen pellets were subsequently thawed at 37°C and carefully resuspended in 10mM Tris HCl pH 8.0 and 10 mM EDTA buffer (35 ml/10⁸ cells) to limit cellular DNA shearing. SDS was added to the resuspended pellets to a final concentration of 1%. After 15 minutes incubation at room temperature NaCl was added to a concentration of 1.25 M. After 3 hours incubation at 4°C the material was centrifuged at 25K g for 20 minutes at 4°C. Dense white pellets containing salts and cellular DNA were discarded and supernatants were digested with Proteinase K (300 µg/ml final concentration) at 42°C for 4 hours and subsequently heated at 65°C for 30 minutes. Two cycles of phenol-chloroform and chloroform extractions were performed prior to recovery of viral DNA by ethanol precipitation in the presence of 0.3 M sodium acetate pH 6.0. The viral DNA pellet was washed with 70% ethanol before being air dried for 1 hour and subsequently resuspended in 2 ml of H₂O. This procedure typically yields approximatively 4 mg of purified CAV2 DNA. Purified viral DNA was stored at -20°C until further utilization.

### EXAMPLE 4 - Viral DNA restriction analysis

Aliquots of purified CAV2 DNA were digested with a set of restriction enzymes purchased from Boehringer Mannheim Corp. (Indianapolis, IN) accordingly to the manufacturer's specifications. Restricted DNA samples were fractionated by electrophoresis on a 1% agarose gel and the corresponding restriction fragments were visualized under UV light after staining of the gel with ethidium bromide (4 µg/ml). Table 1 summarizes the size of the various restriction fragments.

### EXAMPLE 5 -Identification and characterization of the restriction fragment containing the E3 region

### 1. Southern blot analysis of specific endonuclease restricted CAV2 DNA.

Four µg aliquots of purified CAV2 DNA were digested with BamHI, BglI, HindII, HindIII and PstI, respectively, before being fractionated by electrophoresis through a 1% agarose gel. The gel was soaked in 0.25 M HCL for 30 minutes before being washed in H₂O for 5 minutes. Viral DNA was subsequently denatured in 0.5 M NaOH and 0.9M NaCl solution for 30 minutes. After being rinsed with H₂O for 5 minutes, DNA was renatured by two subsequent baths in 0.5 M tris HCl pH 7.5 containing 3 M NaCl. DNA was subsequently transferred overnight in 10xSSC (1.5M NaCl, 0.15M Na Citrate pH 7.4) buffer onto a nylon membrane (Hybond N, Amersham Life Sciences, Cleveland, OH). The nylon membrane was air dried for one hour before being submitted to UV cross-linking for 3 minutes. A 6 hours prehybridization was performed at 65°C in 4xSSC, 25% Denhardt's solution (v/v), 0.1% SDS (v/v), 0.1% Na pyrophosphate and denatured hering sperm DNA (500 µg/ml) solution.

### 2. Preparation of the probes specific for CAV2 PVIII and Fiber genes.

Since in most adenoviruses the E3 region is comprised between the two structural genes, PVIII and fiber, Applicant took advantage of a previously published partial sequence of the CAV2 (Manhattan strain) genome (Linne, 1992) to design two specific primers pairs for each of these genes. Oligonucleotides LF189 (5'-TCAGTCATAGCCATCGACAGA-3') (SEQ ID NO: 26) and LF190 (5'-GTGCTGGCTGGCACGGGCATT-3') (SEQ ID NO: 27) were designed to correspond to sequences within the 3' end of the CAV2 PVIII gene whereas oligonucleotides LF191 (5'-ATGTCCACCAAAGTCCCCTCT-3') (SEQ ID NO: 28) and LF192 (5'-CCCGGGGCGTCGTATGGATAT3') (SEQ ID NO: 29) were designed to correspond to sequences within the 5' end of the CAV2 fiber gene.

A 302 bp DNA PVIII specific probe was generated by mixing 10 ng of purified CAV2 DNA with 5 µl of 10X PCR buffer, 3.75 µl of 2 mM dNTPs, 26 µl H₂O, 0.25 µl of Taq polymerase (5.0 u/µl), 5 µl of 5 µM 5'end primer LF189 and 5 µl of 5 µM 3'end primer LF190. A 30 cycle PCR amplification was performed in a 0.5 ml tube containing 40 µl of mineral oil using the following profile: 94°C 1 minute, 55°C 1 minute and 72°C 1 minute.
A 190 bp DNA Fiber specific probe was generated by PCR by swapping primer LF189 with primer LF191 and primer LF190 with primer LF192 in the previously described protocol. Both PCR reactions were electrophoresed through a 1% agarose gel and the corresponding PCR products were isolated using the Gene Clean procedure according to the manufacturer (Bio 101, Inc., La Jolla, CA) specifications. 100 ng aliquots of each probe was labelled by mixing with 1µg of random hexamers (Pharmacia, Piscataway, NJ) in a total volume of 13 µl and subsequently boiled for 3 minutes before being incubated with 2.5 µl of a dCTP, dTTP and dGTP mixture (each at a concentration of 0.5M), 2.3 µl Klenow 10X buffer, 1.5 µl Klenow enzyme (2u/µl) and 5µl of ³²P-a-dATP (3000 Ci/mmol, 10 mCi/ml, NEN, Boston, MA) at RT for 4 hours. The reaction was stopped by adding 100 µl of Stop solution (IBI Prime Time kit). 25 µl of each probe was heat denatured (100°C) for 3 minutes before being incubated overnight at 65°C with the previously described nylon membrane in a total volume of 50 ml of prehybridization solution. The nylon membrane was subsequently washed at 65°C in 6xSSC, 0.1% SDS and 50 mM Na Pyrophosphate solution for 2 hours. Viral DNA restriction fragments complementary to the radiolabelled DNA probes were identified by autoradiography.

### 3. Identification and cloning of the restriction fragment containing the E3 region.

The HindIII fragment A (4.0 Kbp) was identified as the shortest well isolated restriction fragment recognized by both PVIII and Fiber probes, suggesting that it may contain the entire CAV2 E3 region. This fragment was isolated using Gene Clean procedure as previously described and subsequently subcloned into the HindIII site of the vector pBluescript SK+ (Stratagene, La Jolla, CA) generating plasmid pLF027.

### 4. Characterization of the CAV2 E3 region.

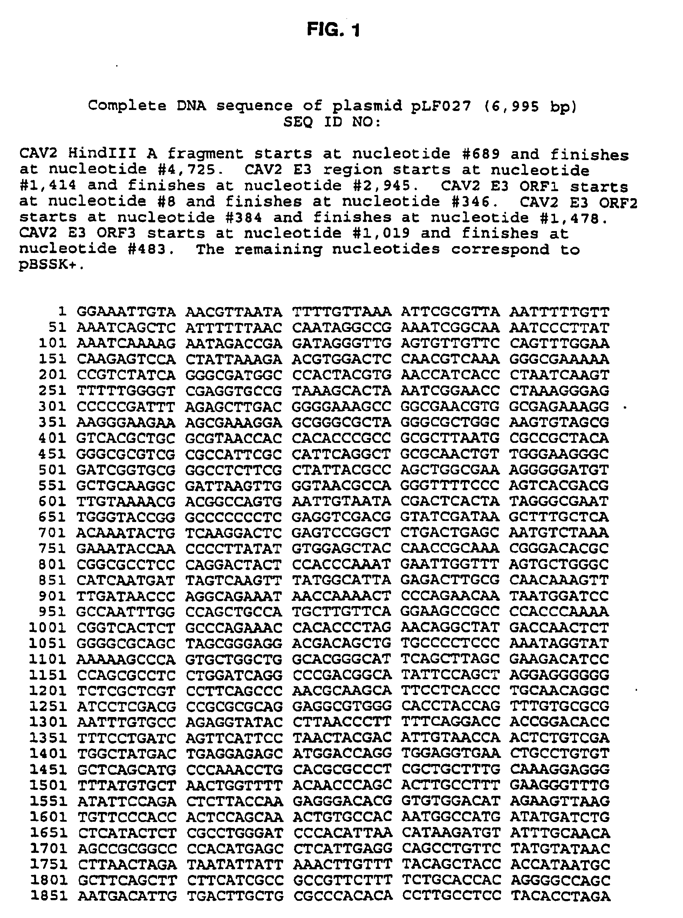

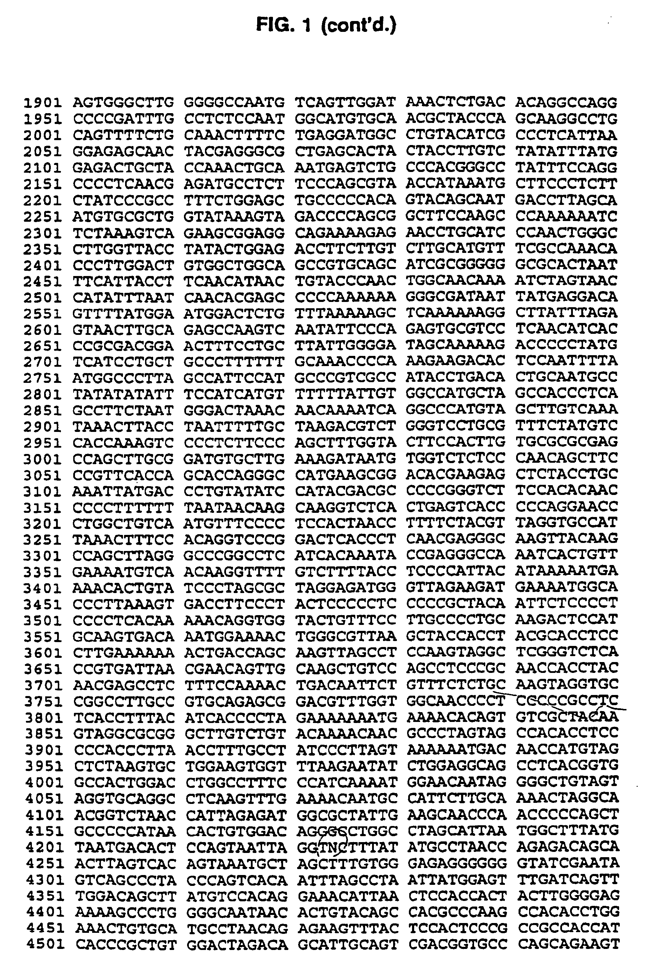

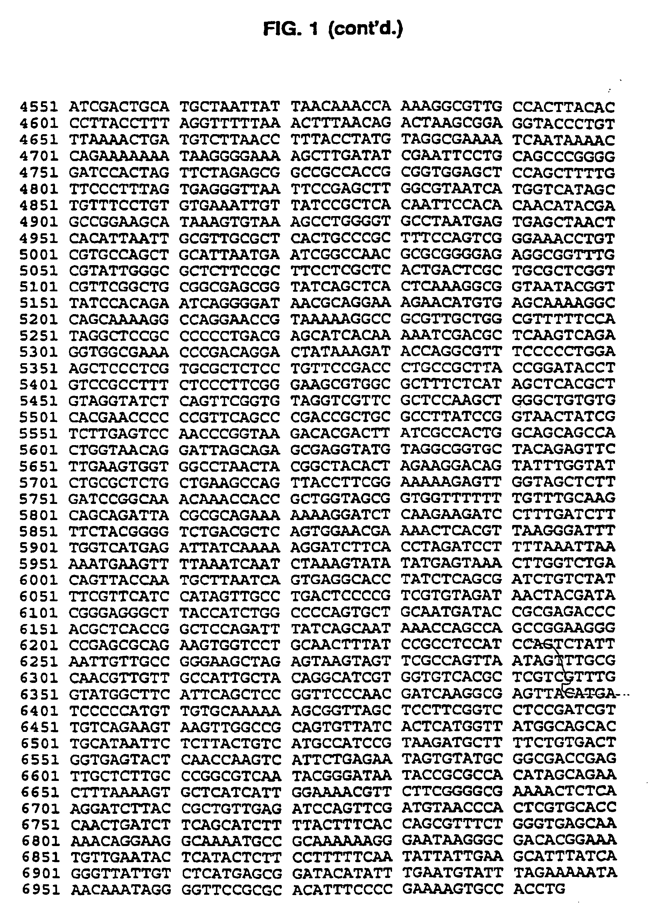

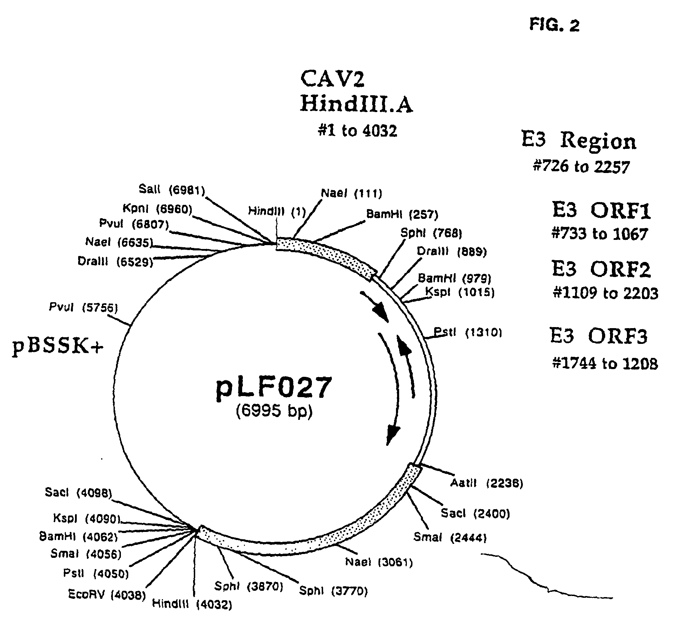

The CAV2 E3 region was analyzed by restriction digestion of pLF027 and by sequencing pLF027 according to Sequenase 2.0 kit instructions (US Biochemical, Cleveland, OH). Sequence analysis was performed using the MacVector software (Eastman Kodak, Rochester, NY). The pLF027 restriction map is shown in Figure 2. The corresponding sequence of the pLF027 including the CAV2 E3 region [defined as the DNA stretch between the PVIII stop codon (#1,413 in pLF027) and the fiber ATG initiation codon (#2,945 in pLF027)] is represented in Figure 1. Analysis of sequencing data revealed that the CAV2 E3 1,533 bps were 100% homologous with the previously identified CAV2 (Manhattan strain) E3 region (Linne, 1992). Analysis of the amino acid sequence deduced from the nucleotide sequence revealed that the rightward coding strand of the CAV2 E3 region encodes two potential polypeptides (ORF1 and ORF2) whereas the leftward coding strand encodes a single potential polypeptide (ORF3). The characteristics of these ORFs are presented in Table 2.

### EXAMPLE 6 - Generation of donor plasmid pLF086.

### 1. Introduction of BglII and MluI restriction sites in the middle of the CAV2 E3 sequence.

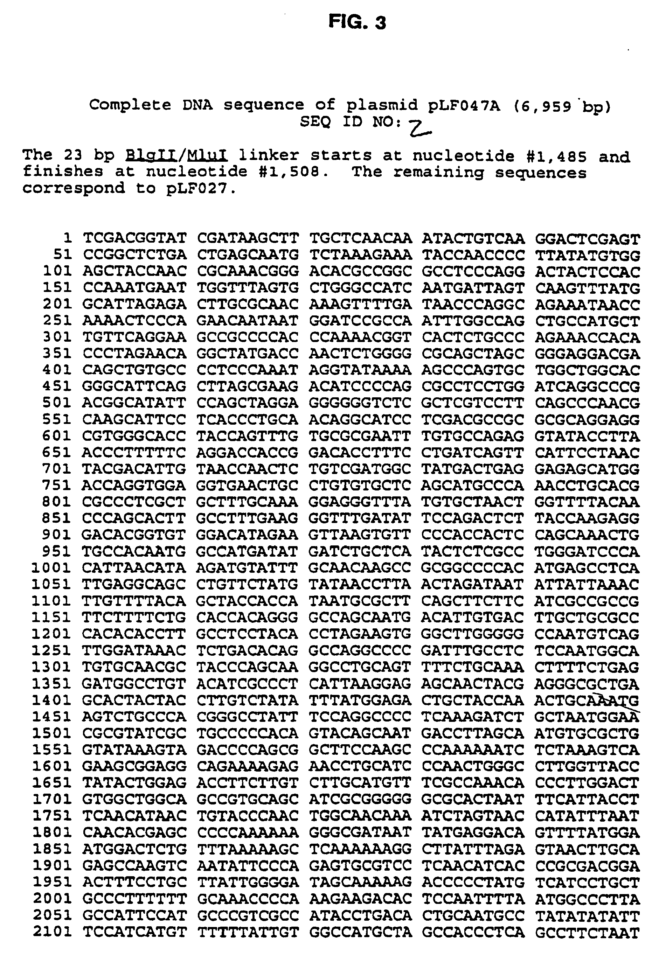

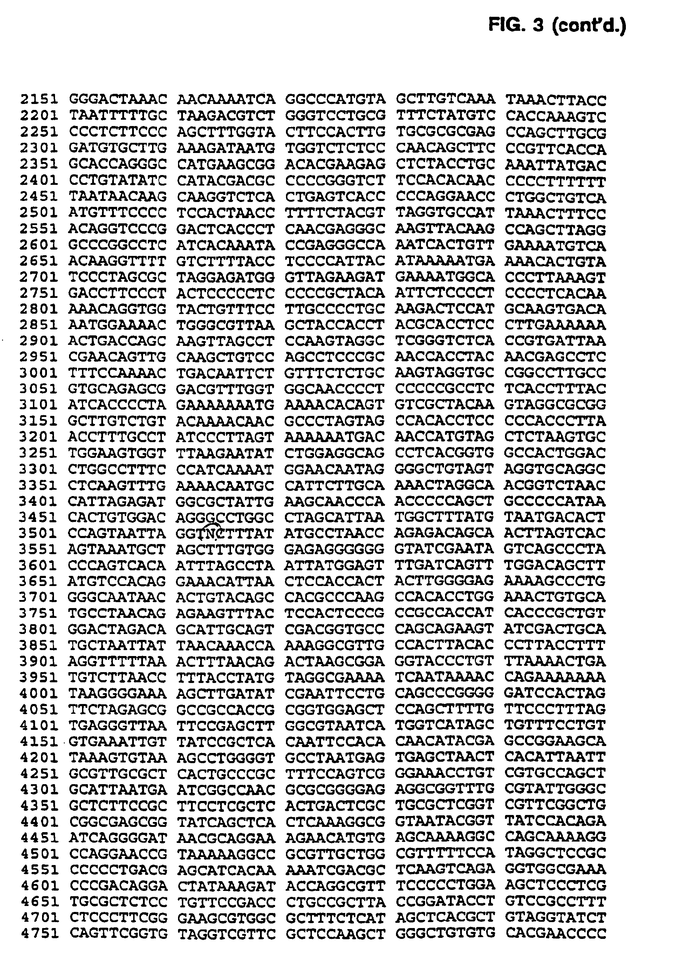

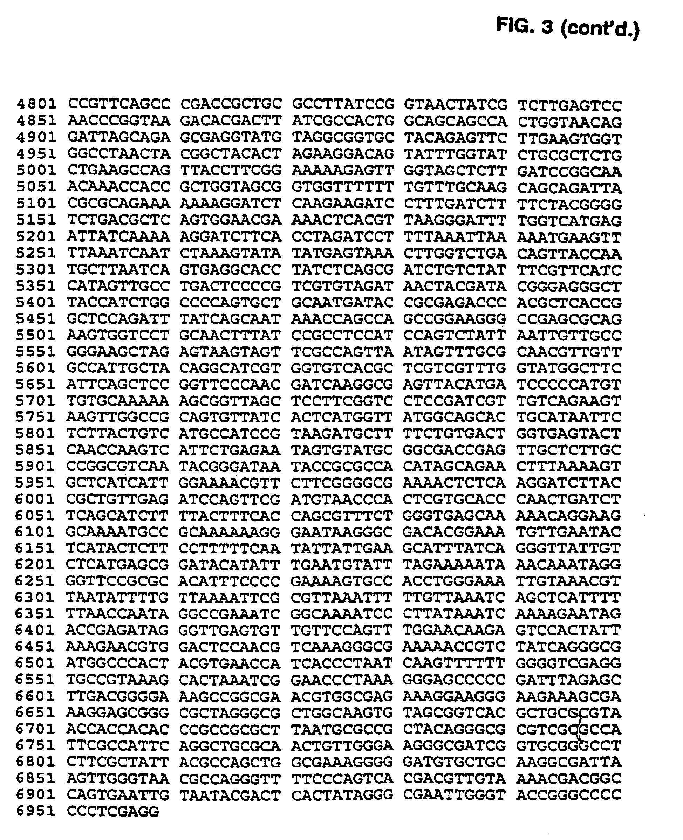

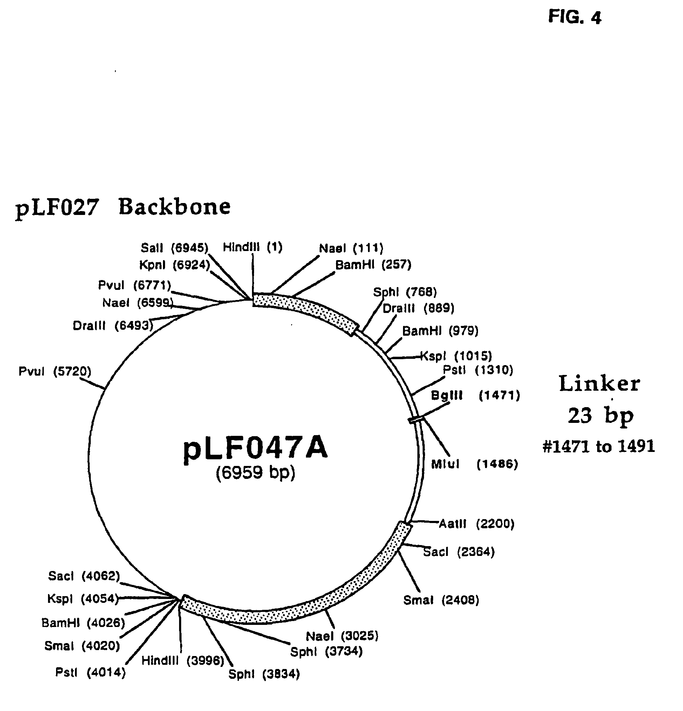

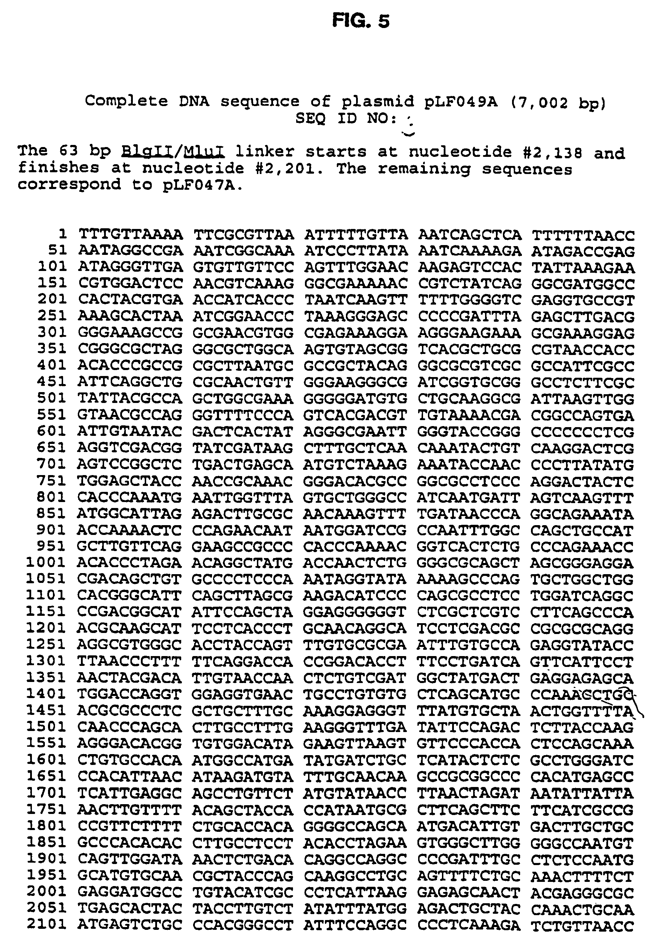

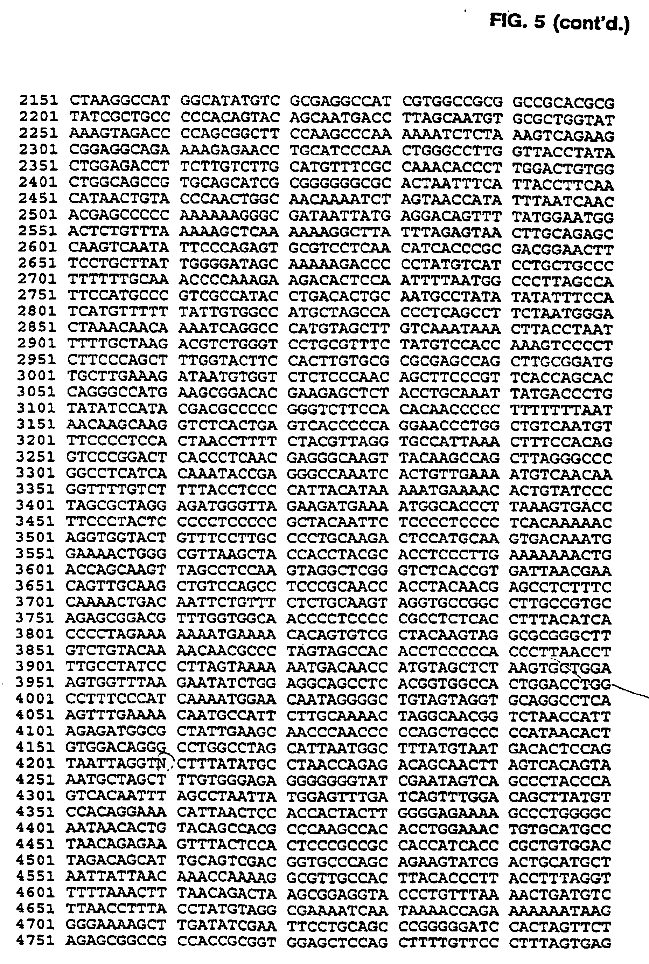

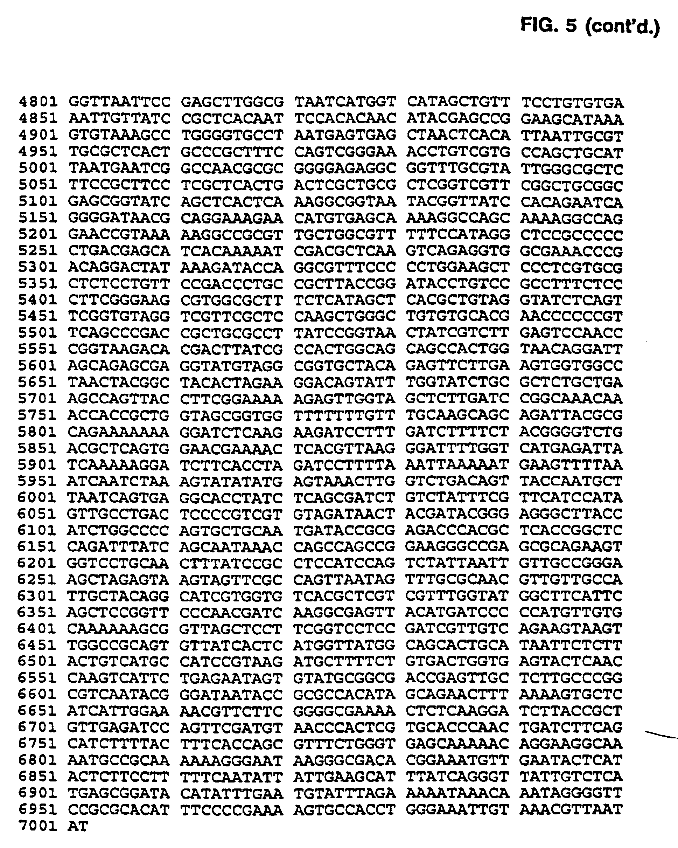

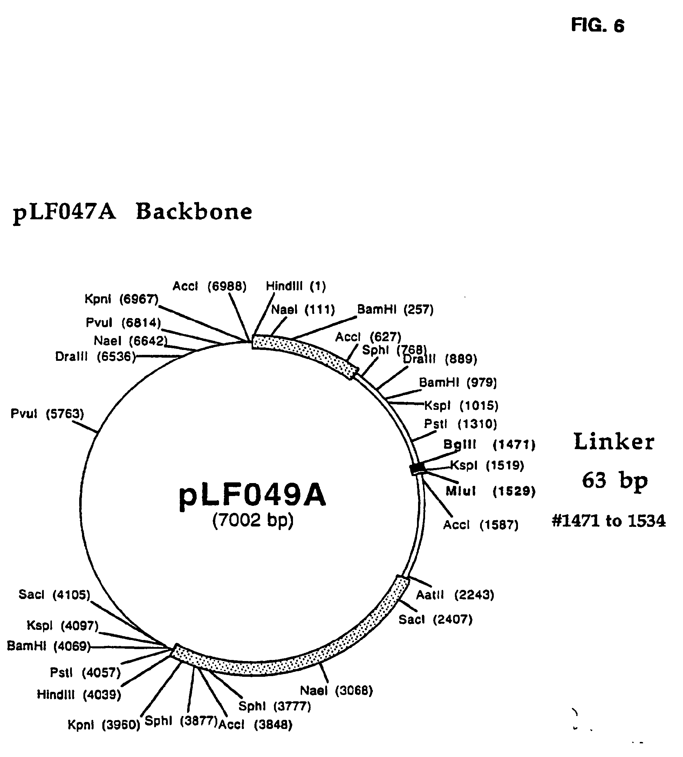

In order to facilitate further manipulations, a 24 bp DNA linker (5'-GATACGCGTTCCATTAGCAGATCT-3') (SEQ ID NO: 30) containing unique BglII and MluI restriction sites were introduced between nucleotide #1487 and #1966 of the CAV2 E3 region (as described in Figure 1) by a double round PCR amplification procedure. Initial PCR amplifications was performed using pLF027 DNA as template and using the following primer couples [LF327(5'-GGACACCTTTCTGATCAGTTCATT-3')/LF324(5'-GATACGCGTTCCATTA GCAGATCTTTGAGGGGCCTGGAAATAGGC-3') (SEQ ID NO: 31, 32)] and [LF326(5'-GGTTGTGTGGAAGACCCGGGGGCG-3')/LF325(5'-AGATCTGCTAATGGAA CGCGTATCGCTGCCCCCACAGTACAGCAA-3') (SEQ ID NO: 33, 34)], to generate two partially overlapping DNA fragments of 838 bp and 956 bp, respectively. The second round of PCR amplification was performed in the presence of both partially overlapping purified DNA fragments and both external primers LF327 and LF326. The resultant 1,794 bp DNA fragment was digested with PstI and AatI and the resultant 890 bp PstI/AatII fragment was purified and ligated with the 6,069 bp PstI/AatII DNA fragment of pLF027, generating pLF047A (Figures 3 and 4). All PCR amplifications were performed using the conditions previously described. The 6,944 bp MluI/BglII pLF047A was subsequently ligated with preannealed oligonucleotides LF328 (5'-GATCTGTTAACCCTAAGGCCATGGCATATGTCGCGAGGCCATCGTGGCCGCGGCCGC A-3 ') (SEQ ID NO: 35) and LF329 (5'-CGCGTGCGGCCGCGGCCACGATGGCCTCGCGACATATGCCATGGCCTTAGGGTTAAC A-3') to (SEQ ID NO: 36) generate pLF049A (Figures 5 and 6).

This manipulation results in the exchanging of 60 bp of the CAV2 E3 region with a 60 bp BglII/MluI polylinker DNA fragment. The size of the E3 region has not been modified and E3 ORF1 remained unaffected. However, sequences corresponding to E3 ORF2 have been disrupted and those of the E3 ORF3 were completely eliminated.

### 2. Generation of donor plasmid pLF086.

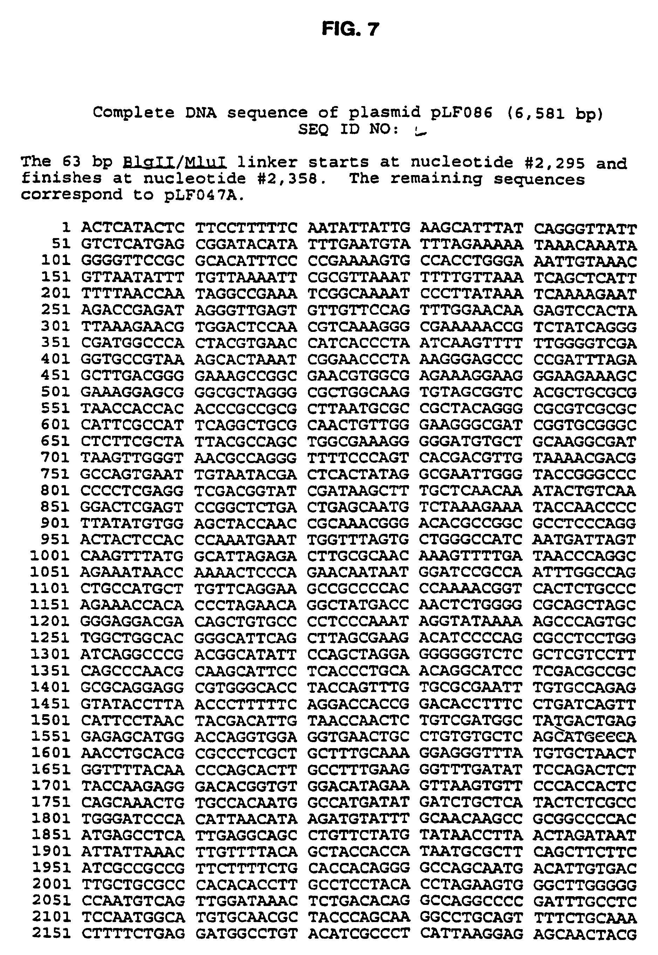

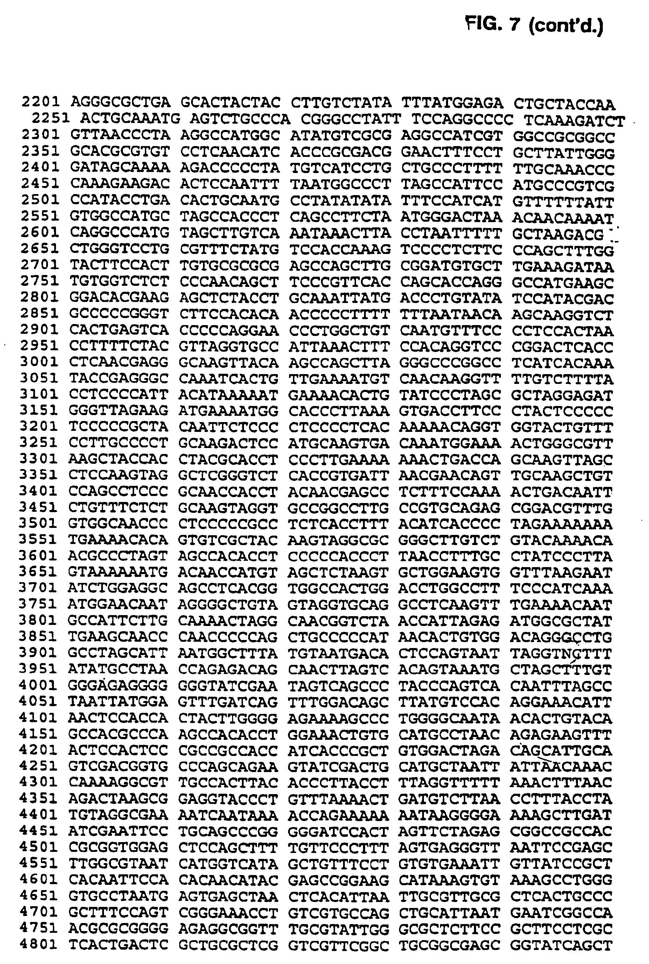

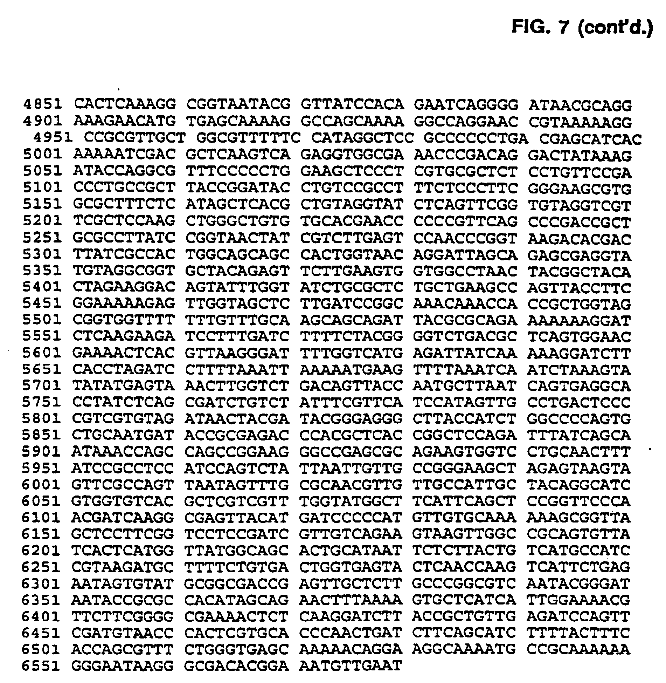

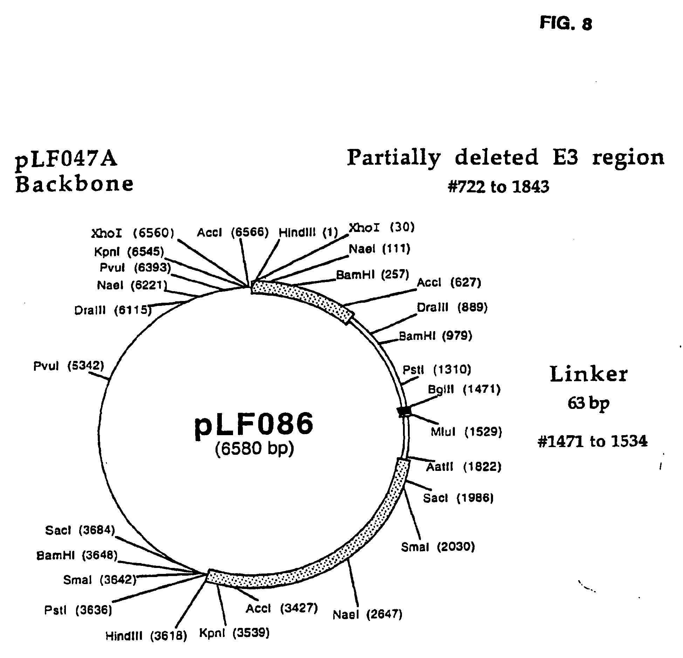

In order to delete part of the CAV2 E3 region a 428 bp deletion was engineered 3' of the pLF049A MluI site. A 537 bp DNA fragment was generated by PCR as previously described using the pLF027 template and the primers pair LF361(5'-CTAGTCATCTTAACGCGTGTCCTCAACATCACCCGCGA-3')/LF334(5'-CTT GCTTGTTATTAAAAAAAG-3') (SEQ ID NO: 37, 38). This 551 bp fragment was subsequently digested with MluI and AatI before being purified and ligated with the 6,284 bp MluI/AatII DNA fragment of pLF049A, generating pLF086 (Figures 7 and 8). This manipulation, which introduces a 27% (428 bp) deletion of the E3 region, further expands the deletion of E3 ORF2 towards its 3'end but does not interfere with E3 ORF1 coding sequence.

### EXAMPLE 7 - Cloning and characterization of the restriction fragment containing the right end of the viral genome

### 1. Cloning of the restriction fragment containing the right end of the viral genome.

Previously published restriction maps of the CAV2 (Glasgow strain) genome indicated the presence of a unique SalI restriction site located at 84.0 map units (Spibey and Cavanagh 1989). SalI digestion of CAV2 DNA (30 µg) generated the predicted 3.2 kbp and 29 kbp DNA fragments. The CAV2 DNA SalI B fragment (3.2 kbp) was gel purified using Gene Clean procedure as previously described and resuspended in 20 µl of H₂O. Approximatively 3 µg of purified SalI B fragment was denatured by the addition of 2 µl of 1 N NaOH in a total volume of 22 µl for 90 minutes at RT to eliminate the known protein moiety (Robinson et al., 1973) which is covalently linked to the 5' termini of adenovirus genome. The DNA was subsequently renatured by the addition of 1.3 µl of 2M Tris HCl pH 7.5 and incubated successively at 65°C for 1 hour and at RT for 1 hour before being ligated with the 2.919 bp SalI/SmaI fragment of pBluescript SK+ to generate pLF056.

### 2. Characterization of the restriction fragment containing the right end of the viral genome.

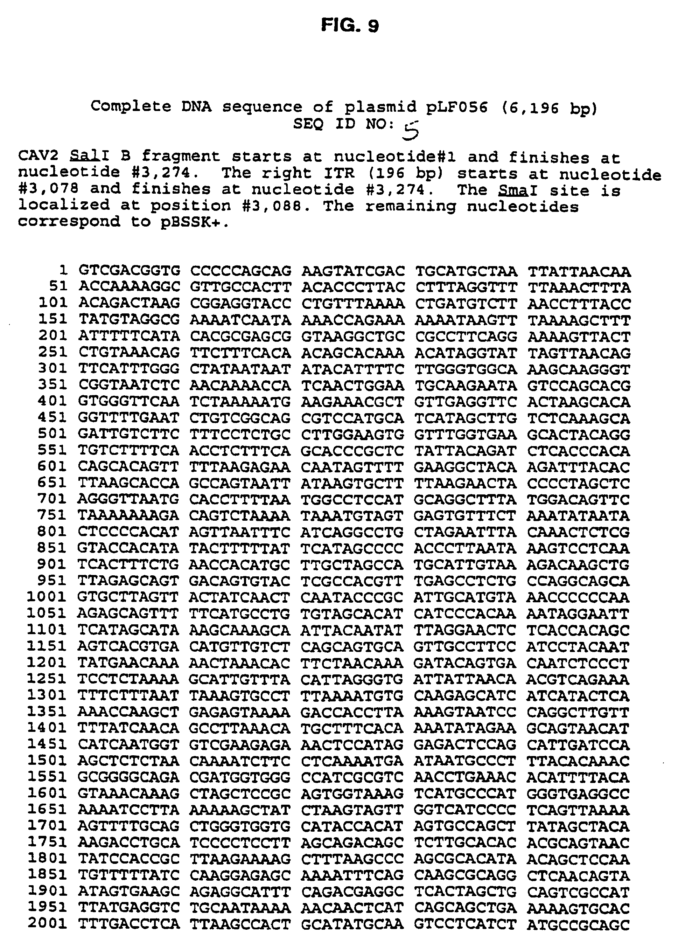

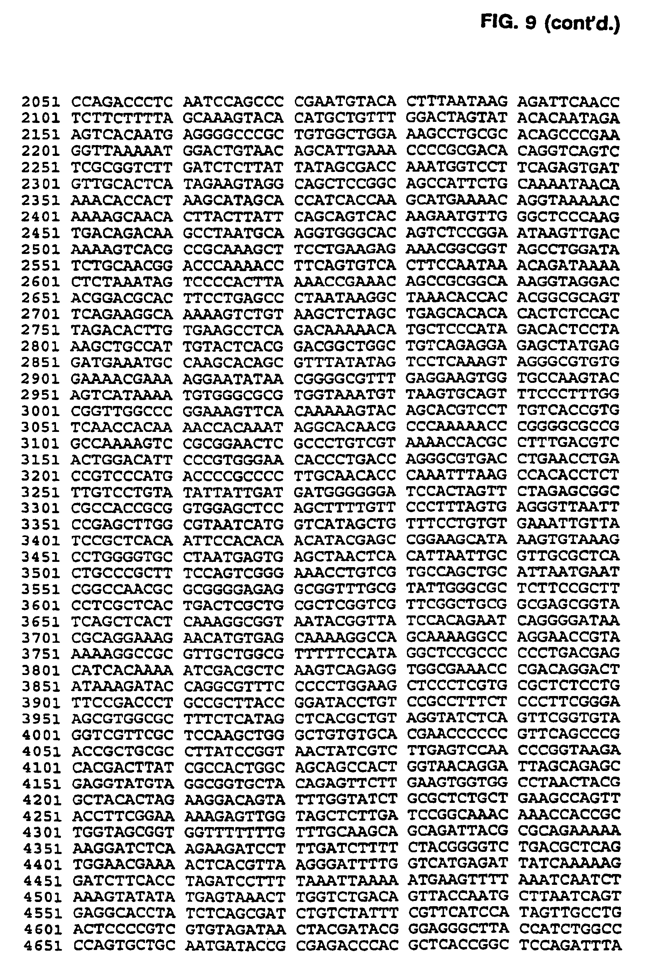

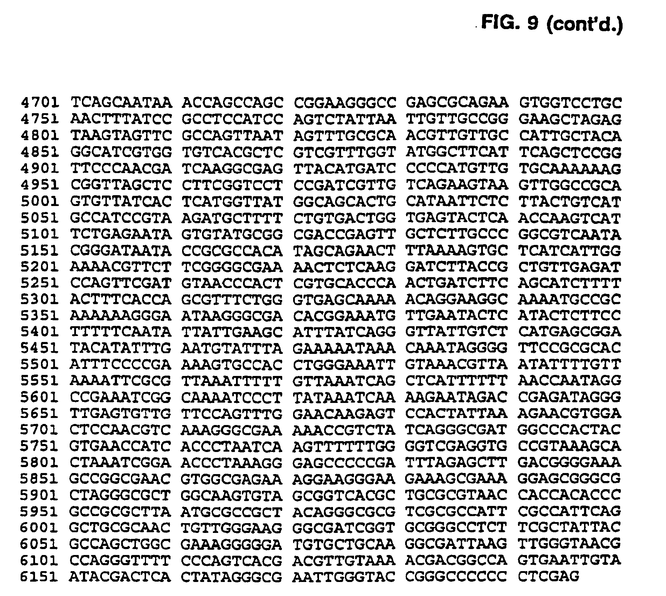

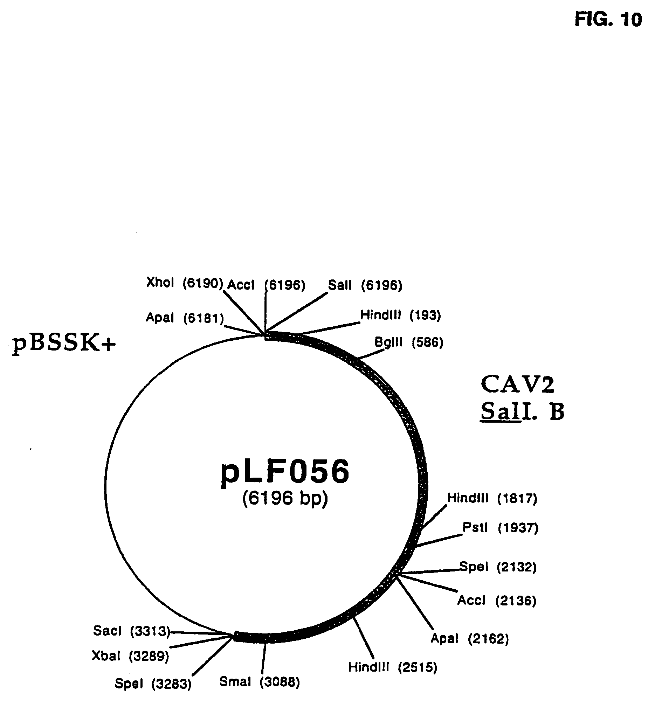

The 3.2K bp right end of the CAV2 genome was analyzed by restriction digestion of pLF056 and by sequencing of the same plasmid according to Sequenase 2.0 kit instructions. Sequence analysis was performed using the MacVector software. The pLF056 restriction map is shown in Figure 10, and Figure 9 shows the DNA sequence. Sequencing data revealed that the CAV2 DNA SalI B fragment is 3,274 bp in length. Two unique restriction sites within the CAV2 genome have been localized within the CAV2 DNA SalI B fragment: BglII at position #587 and SpeI at position #2,133. The 196 bp ITR (Figure 9) nucleotide sequence of CAV2 situated at the right termini is 100% homologous with the CAV2 right and left ITR sequences previously published for the CAV2 Vaxitas and Glasgow strains, respectively (Cavanagh et al. 1991). Analysis of the remainder of the CAV2 SalI-B fragment DNA versus the DNA sequence of the previously mentioned CAV2 strains shows significant divergence with only 45 % homology.

### EXAMPLE 8 - Generation of pLF061

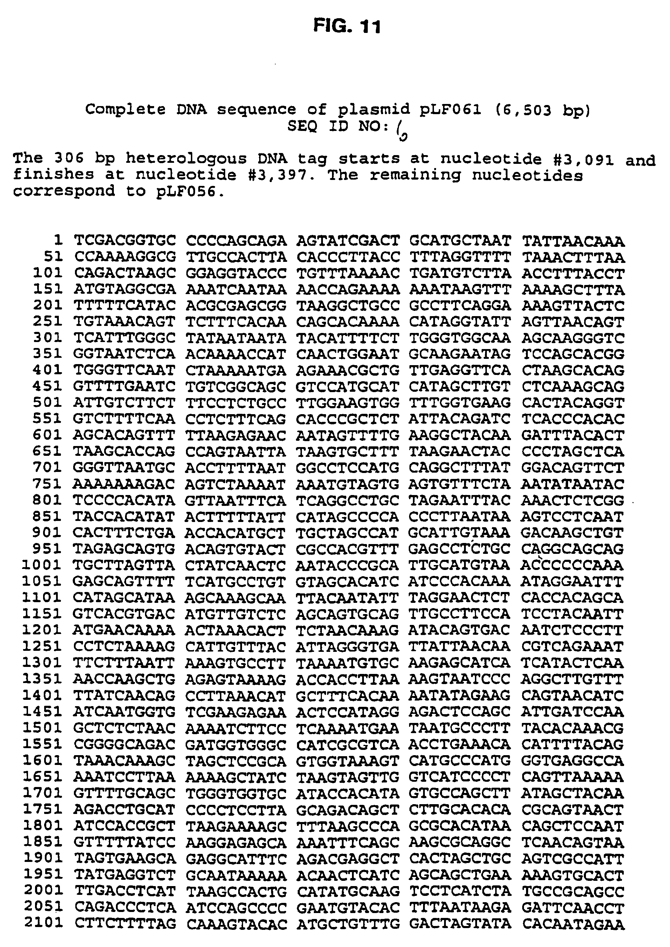

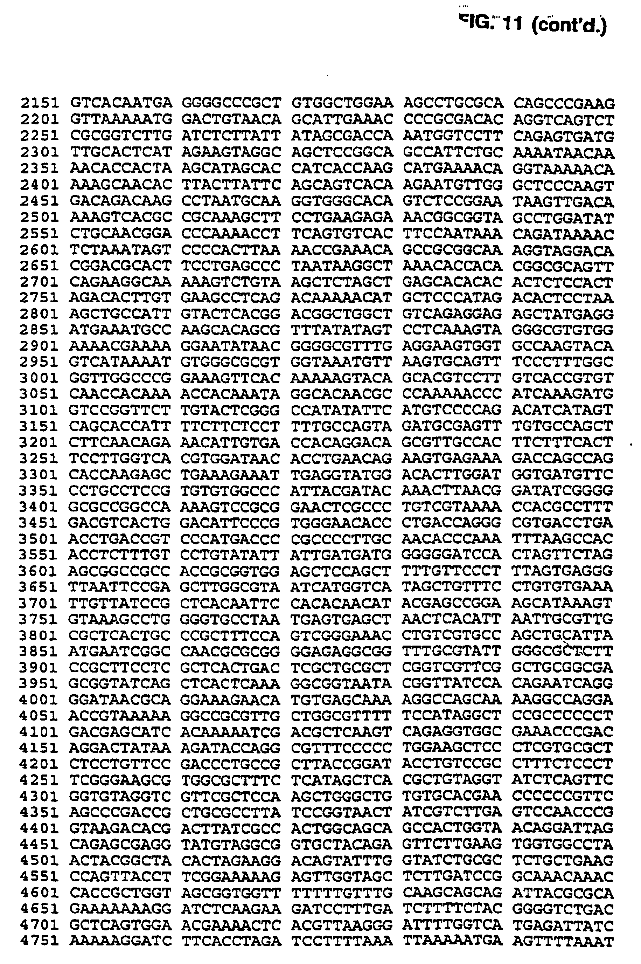

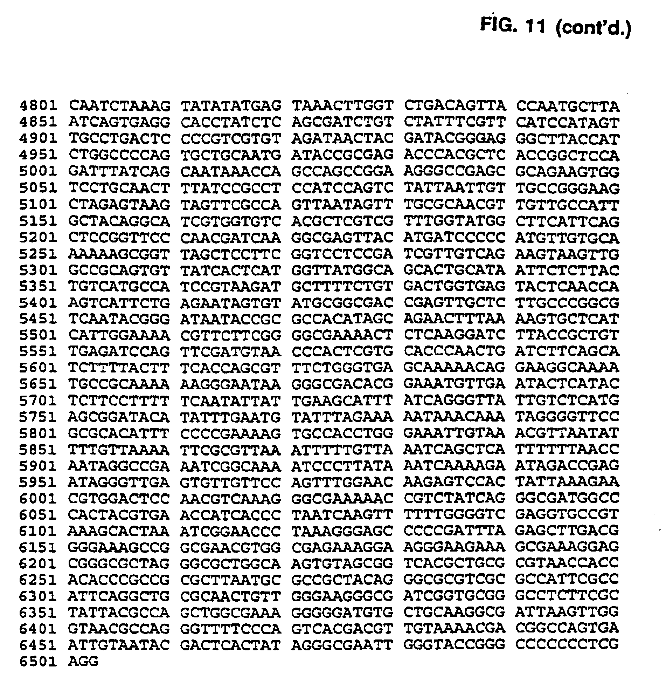

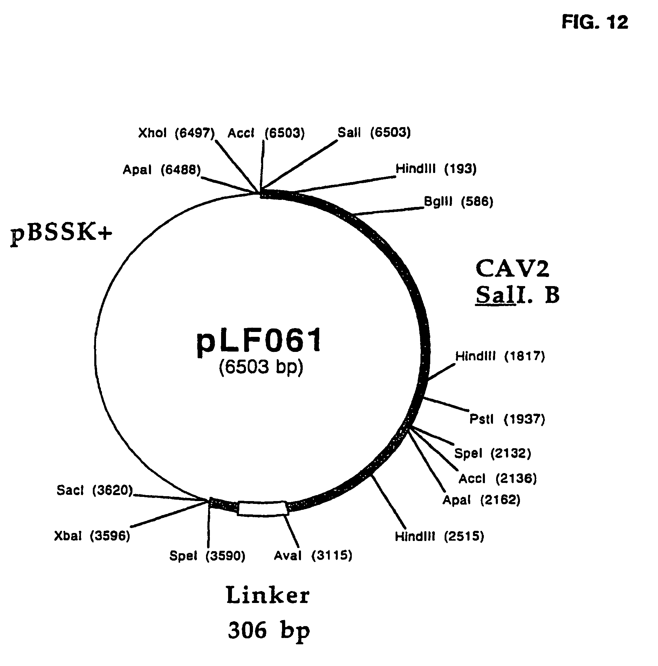

A NruI/EcoRV 312 bp tag DNA fragment (Figure 11) was ligated with SmaI linearized pLF056 to generate pLF061 (Figure 11; restriction map shown in Fig. 12).

### EXAMPLE 9 - Transfection of purified viral DNA into MDCK cells

Solution A was prepared by mixing 5 µg of purified CAV2 DNA with serum free MEM, supplemented as previously described, to a final volume of 300 µl. Solution B was prepared by adding 40 µl of Lipofectamine reagent (Gibco) to 260 µl of supplemented but serum free MEM medium. Solutions A and B were mixed together and incubated at RT for 30 minutes. The CAV2 DNA/liposome complexes were gently mixed with 2.4 ml of supplemented MEM medium (serum free) before being added to MDCK cell monolayer that was 75% confluent. After 24 hour incubation at 37°C in presence of 5% CO2, the serum free medium was removed and replaced by 3 ml of supplemented MEM medium containing 5% CO₂. The culture was incubated at 37°C in presence of 5% CO₂ for 8 days with 2 ml of supplemented MEM medium being added to it on the third day. No CPE could be evidenced during this incubation. On day 8 the transfected MDCK cells were scraped off and harvested in a total volume of 5 ml. After 2 rounds of 2 minutes sonication on ice, 2 ml of the transfected culture were used to infect a 100% confluent MDCK monolayer in a 150 mm diameter tissue culture dish for 1 hour at 37°C in presence of 5% CO₂. The culture was subsequently overlaid with medium containing 0.6% agarose. Plaques are appearing after 5 days at 37°C in the presence of 5% CO₂. Typically, a yield of at least 2,000 pfu/10 µg of purified DNA is observed.

### EXAMPLE 10 - Generation of recombinant CAV2 virus vCA1

### 1. In vitro generation of a recombinant CAV2 genome.

20 µg of purified CAV2 DNA was digested with 30 U of SalI overnight at 37°C. The digested DNA was phenol chloroform extracted and ethanol precipitated before being resuspended in H₂0 to a concentration of 370 ng/µl. 5 µg of SalI digested CAV2 DNA were in vitro ligated with 5 µg of the 3,557 bp SalI/SacI pLF061 DNA fragment overnight at 15°C in the presence of 400 U of ligase (NEB, Beverly, MA) in a total volume of 50 µl.

### 2. Isolation of CAV2 recombinant virus vCA1.

The whole ligation reaction was subsequently used to transfect a 75% confluent MDCK monolayer as previously described. 4 ml of the harvested transfected culture were used to infect two 150 mm diameter tissue culture dishes. A total of 8 plaques became apparent after 10 days of incubation. All plaques were picked and resuspended in 1 ml of supplemented MEM medium before being sonicated for 2 x 2' on ice. The clarified culture medium was serially diluted and used to infect 100% confluent MDCK cells monolayer in 60 mm diameter tissue culture dishes. After 6 days of culture the agarose overlay was discarded and the infected monolayer was blotted onto nitrocellulose filters following the procedure described in Perkus et al. 1993. The filters were processed and subsequently hybridized with a labelled NruI/EcorV 312 bp tag DNA fragment following classical procedures previously described. Autoradiography experiments demonstrated that five out the initially detected 8 plaques contain recombinant CAV2 viruses. One well isolated plaque identified by plaque hybridization was picked and submitted to four additional rounds of plaque purification on MDCK cells. Hybridization with the probe was confirmed after each round of purification. The plaque purified recombinant CAV2 virus was named vCA1.

### 3. Characterization of vCA1.

To further characterize vCA1 a small scale DNA purification was performed. Briefly, purified vCA1 recombinant virus was used to infect a 100% confluent MDCK monolayer (10⁶ cells). After 5 days, when CPE were completed, the infected culture was scraped and harvest. The sonicated and clarified culture medium was treated with proteinase K (500 µg/ml final concentration) for 2 hours at 42°C. The enzyme was inactivated by heating the reaction at 65°C for 20 minutes and the total DNA was subsequently phenol chloroform extracted and ethanol precipitated before being resuspended in H₂O. Purified total DNA was subsequently treated with RNase T1, phenol chloroform extracted and ethanol precipitated before being resuspended in H₂O to a final concentration of 1.2 µg/ml. 5 µg aliquots of purified vCA1 were independently digested with BglII and SpeI. Since those two sites are unique within the CAV2 genome a 29 kbp and 3 kbp fragments are expected from the BalII digestion, whereas a 30.5 kbp and a 1.5 kbp fragments are expected from the SpeI digestion. These restriction fragments are indeed observed demonstrating that vCA1 is a recombinant CAV2 virus which has incorporated 300 bp of heterologous DNA within the right end of its genome.

To further demonstrate that vCA1 has indeed incorporated the expected tag DNA fragment, the VCA1 DNA was analyzed by Southern blotting; and, this confirmed that vCA1 indeed incorporated the tag DNA fragment.

To confirm that the CAV2 SmaI has been used as the insertion site, a 1.9 kbp DNA fragment was amplified from purified vCA1 DNA with the couple of primers LF379 (5'-TCACGCCCTGGTCAGGGTGTT-3') (SEQ ID NO: 39) and LF407 (5'-GCCATCGCGTCAACCTGA-3') (SEQ ID NO: 40) using the conditions previously described. A partial sequence analysis of 1.940 bp DNA fragment conducted using primers LF63 (5'-ATGATGTCTGGGGACATG-3') (SEQ ID NO: 41), LF379 (5'-TCACGCCCTGGTCAGGGTGTT-3') (SEQ ID NO: 42) and LF384 (5'-ACCACGCGCCCACATTTT-3') (SEQ ID NO: 43) confirmed that the heterologous tag DNA was indeed inserted into the CAV2 SmaI site to yield vCA1.

### EXAMPLE 11 - Generation of recombinant CAV2 virus vCA2

Ten µg of pLF086 were digested with HindIII and the resulting 3.6 kbp DNA fragment was isolated using Gene Clean procedure as previously described and resuspended in H₂O to a concentration of 100 ng/µl. MDCK cells were transfected using the Lipofectamine based procedure previously described. Solution A was prepared by mixing 0.5 µg of 3.600 bp HindIII DNA fragment with 3 µg of purified CAV2 DNA. Solution A total volume was brought to 300µl with supplemented serum free MEM medium. Transfected cells were harvested after 8 days and plate out on 150 mm diameter tissue culture dishes as previously described. Plaques were lifted as previously described and hybridized with 5' end labelled oligonucleotide LF328. Five viral plaques crossreacting with the probe were picked and subsequently submitted to 4 rounds of plaque purification as previously described. The plaque purified recombinant CAV2 virus was named vCA2. (Note that plaque purification is a use of the recombinant for replication of the DNA, or for replication of the virus, i.e., a vector use of the recombinant, thereby showing that there is no restriction or limit on the exogenous DNA).

### 2. Characterization of vCA2.

To characterize vCA2, a small scale DNA purification was performed as previously described for vCA1. Purified vCA2 DNA and wild-type CAV2 DNA were independently digested by HindIII and the restricted DNAs were subsequently fractionated by electrophoresis through a 1% agarose gel. A 3.6 kbp HindIII fragment was visualized in the vCA2 sample whereas a 4.0 kbp fragment was present in the wild-type CAV2 sample, proving that the E3 region has been deleted of 428 bp in vCA2 genome.

To further demonstrate that the expected tag (oligonucleotides LF328/LF329) has indeed been incorporated into the vCA2 E3 region, Southern blot was performed and this confirmed incorporation of the tag.

This result indicates that the complete CAV2 E3 ORF2 is not necessary in tissue culture. It also demonstrates that part of the CAV2 E3 ORF2 sequences can be exchanged with heterologous DNA and thus validates a second insertion site within the CAV2 genome. This results also proves that part of the CAV2 E3 region can be deleted to compensate for the introduction of foreign DNA into the SmaI site previously described in the derivation of vCA1.

### EXAMPLE 12 - Generation of subfragment promoters, reduced or modified polyadenylation cassettes, promoters with 5' UTR replaced, and plasmids and recombinants containing same

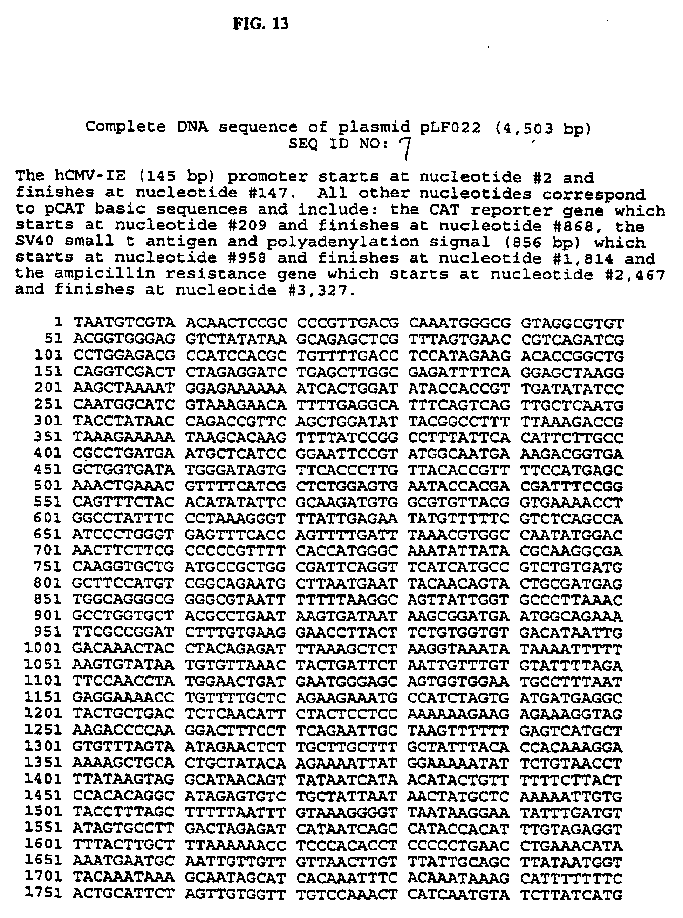

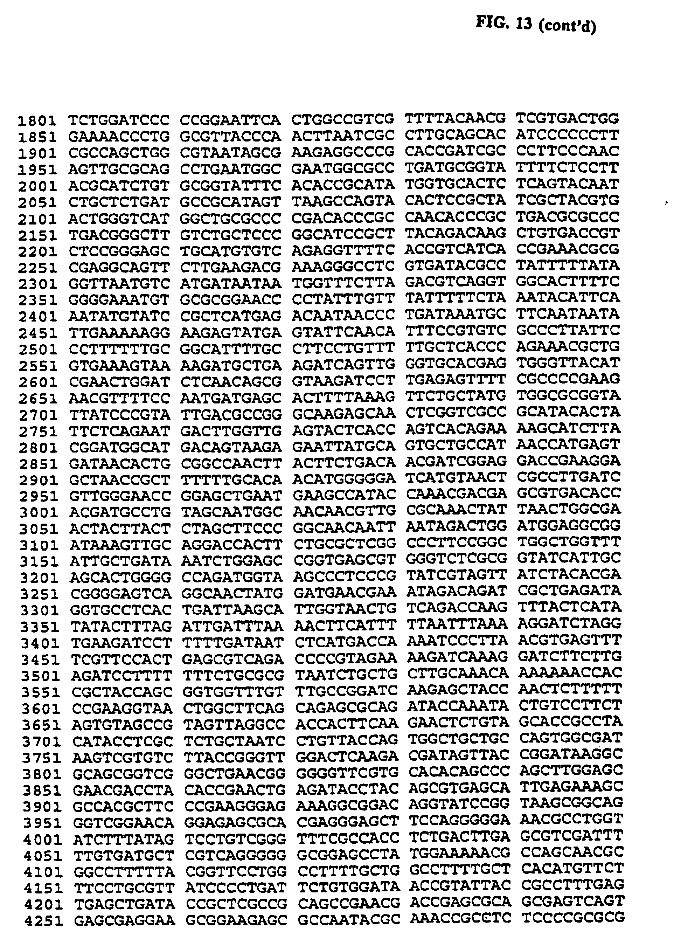

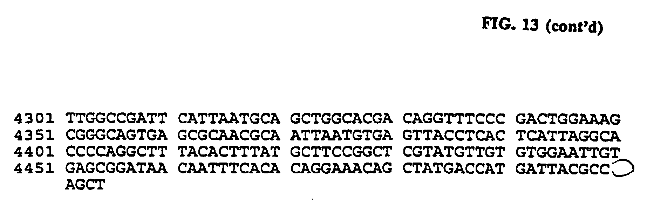

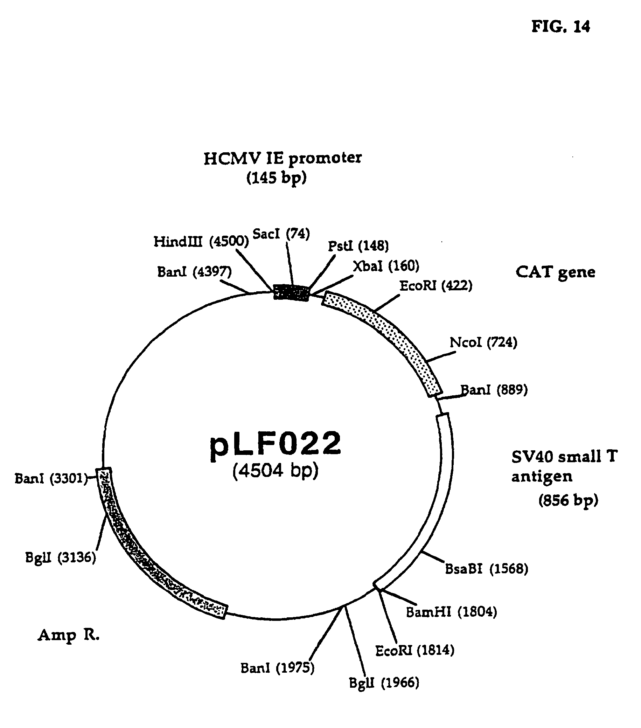

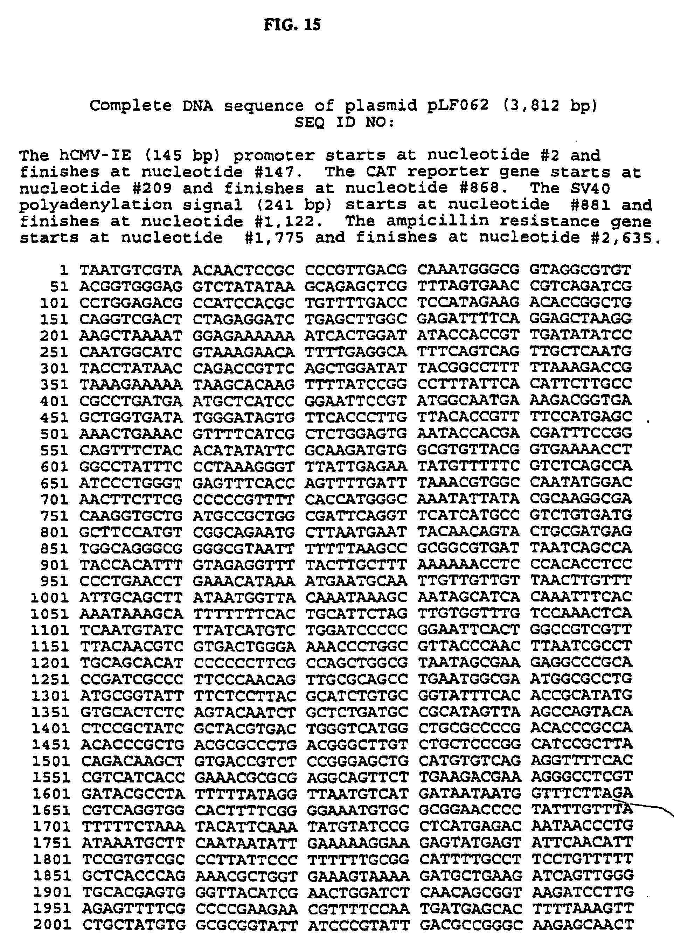

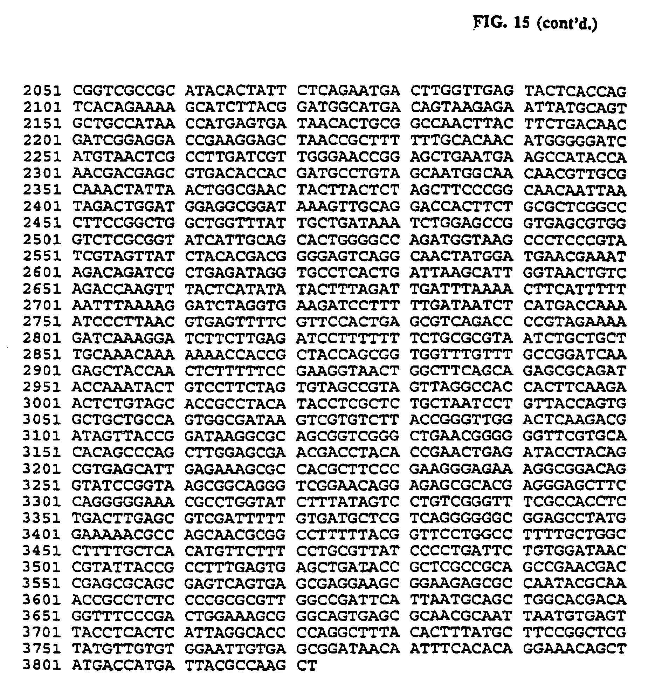

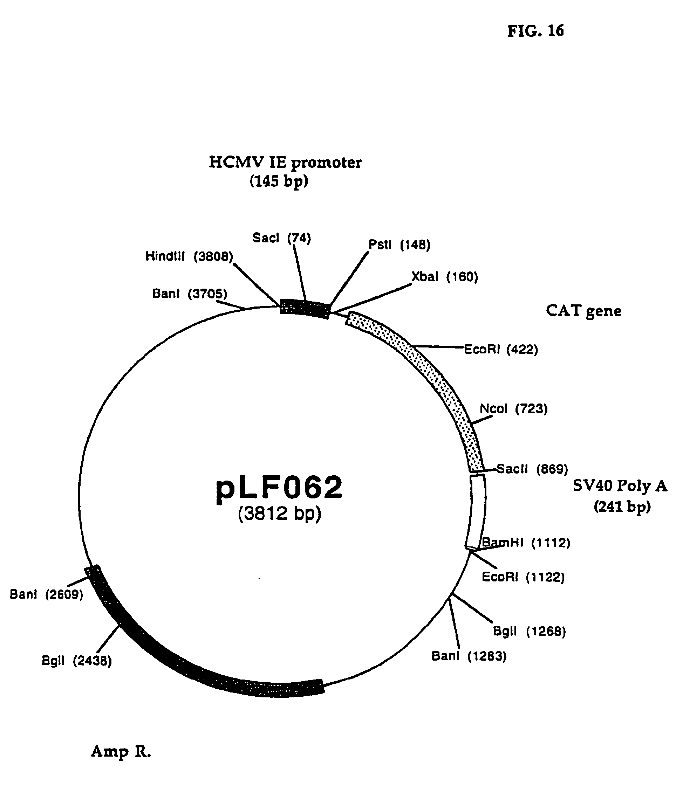

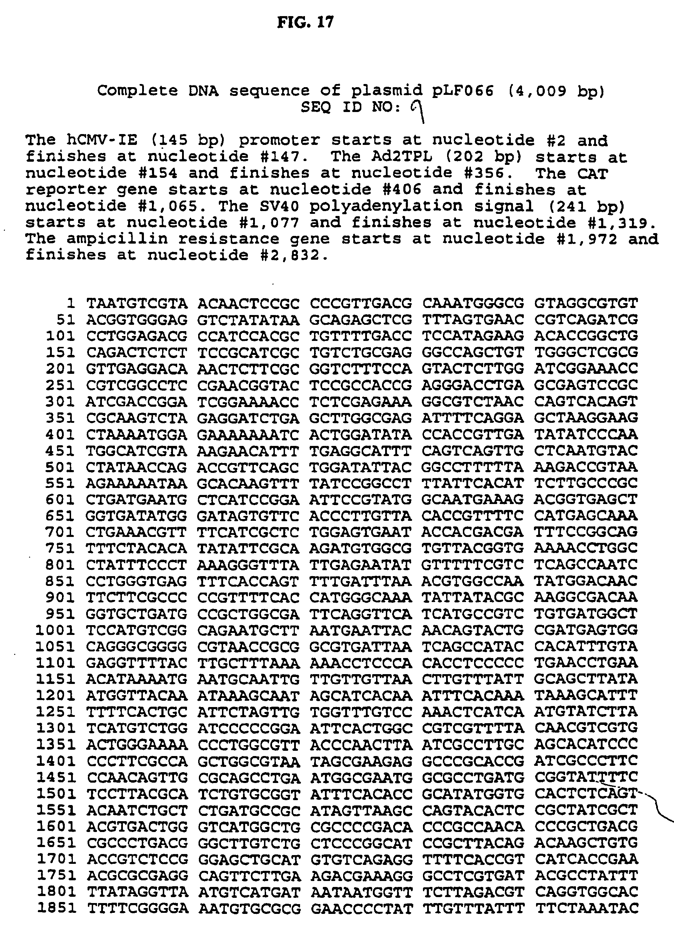

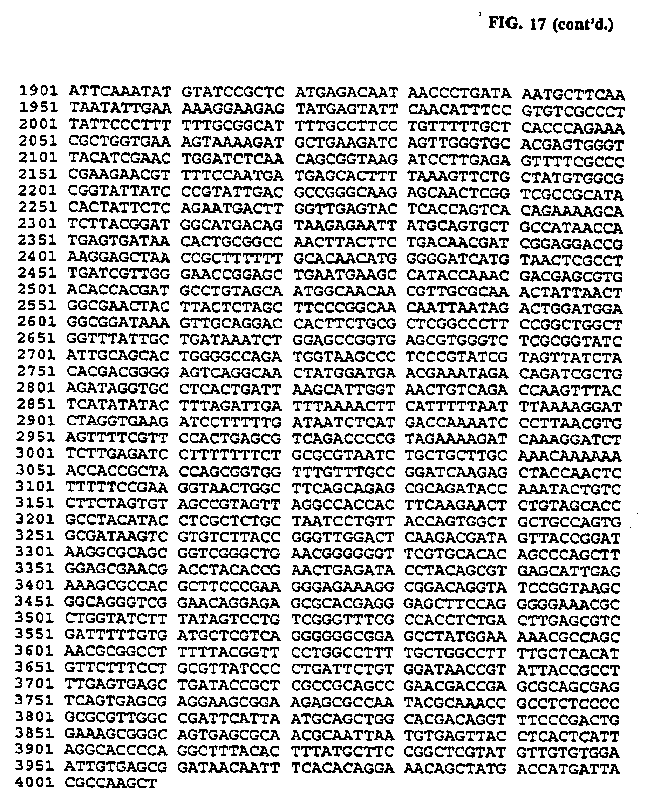

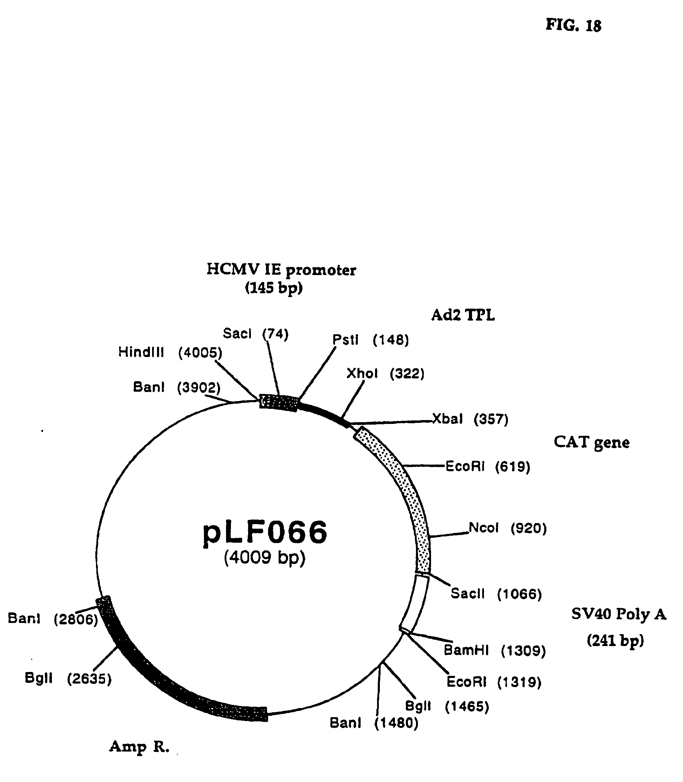

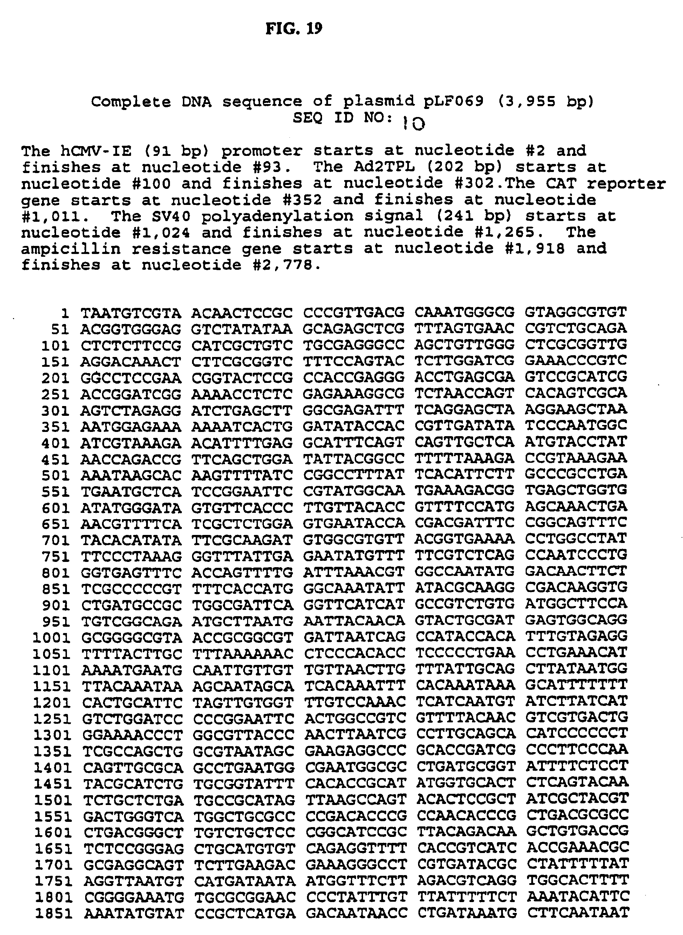

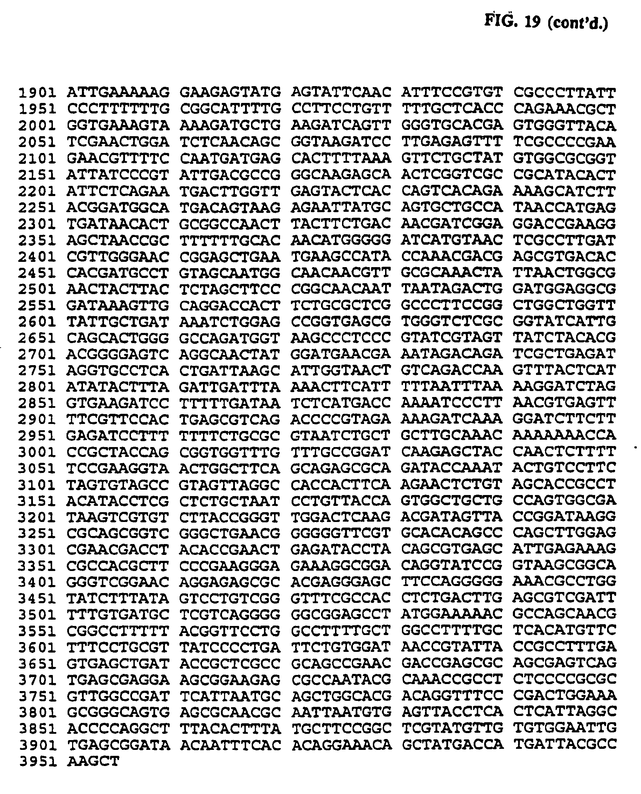

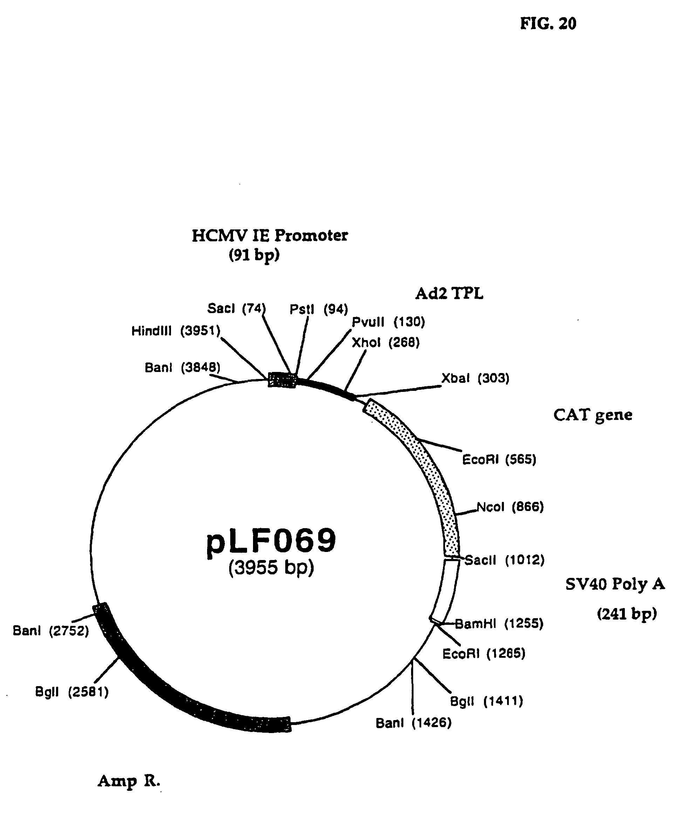

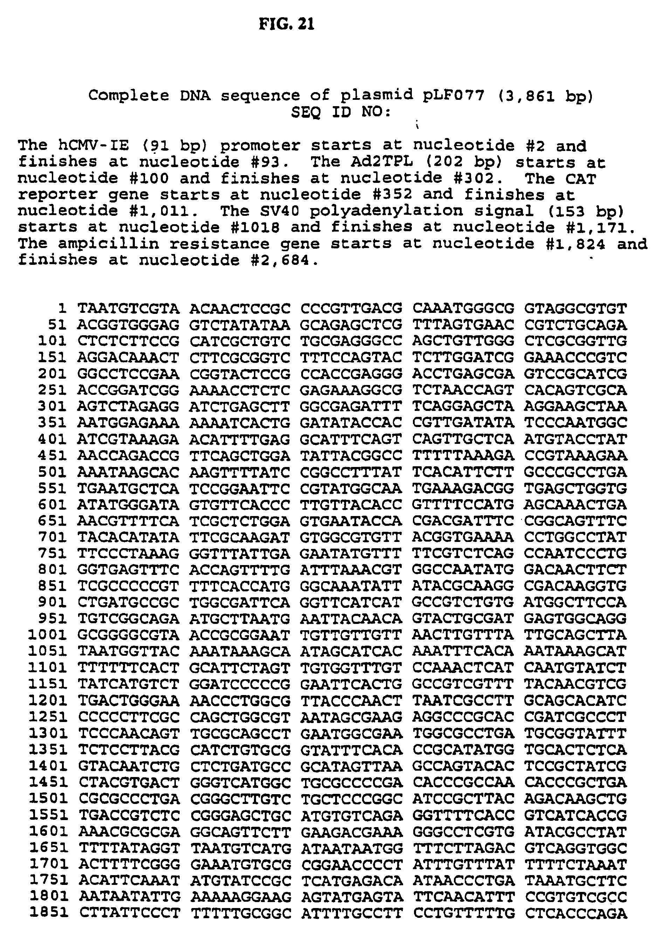

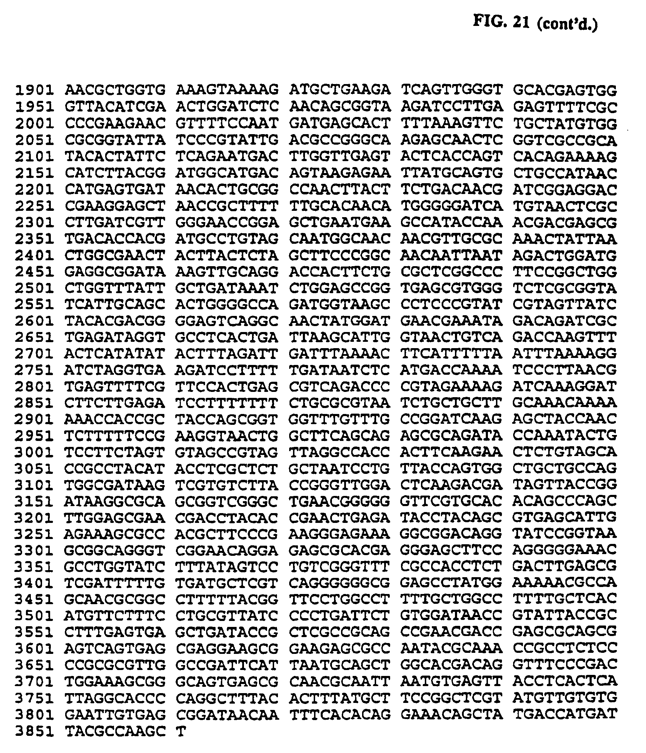

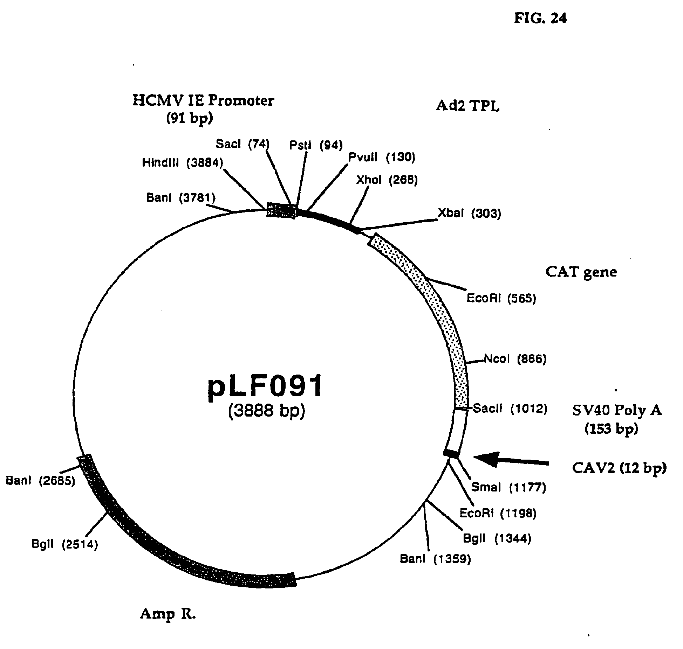

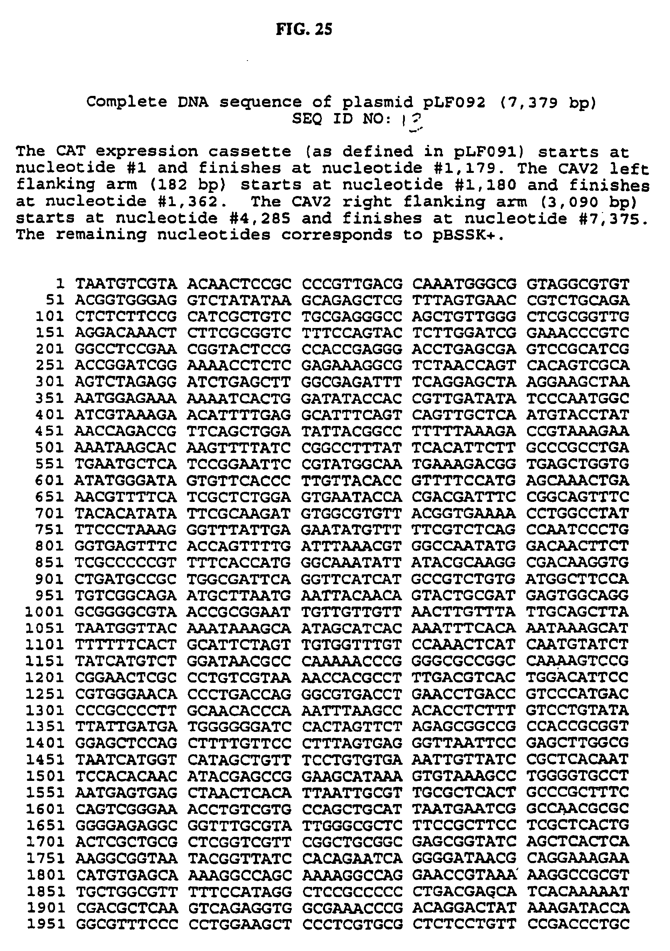

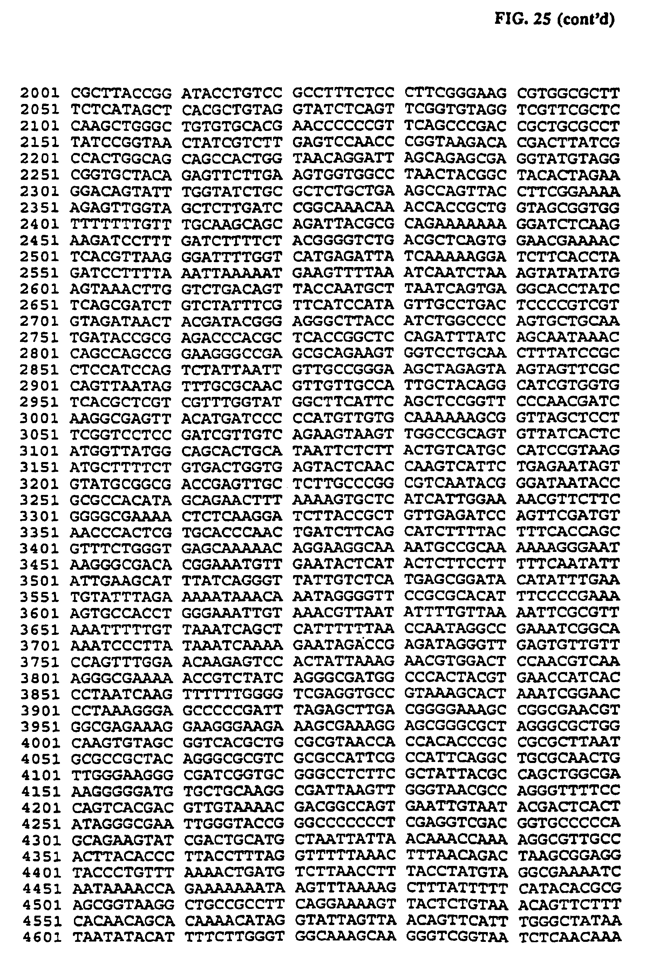

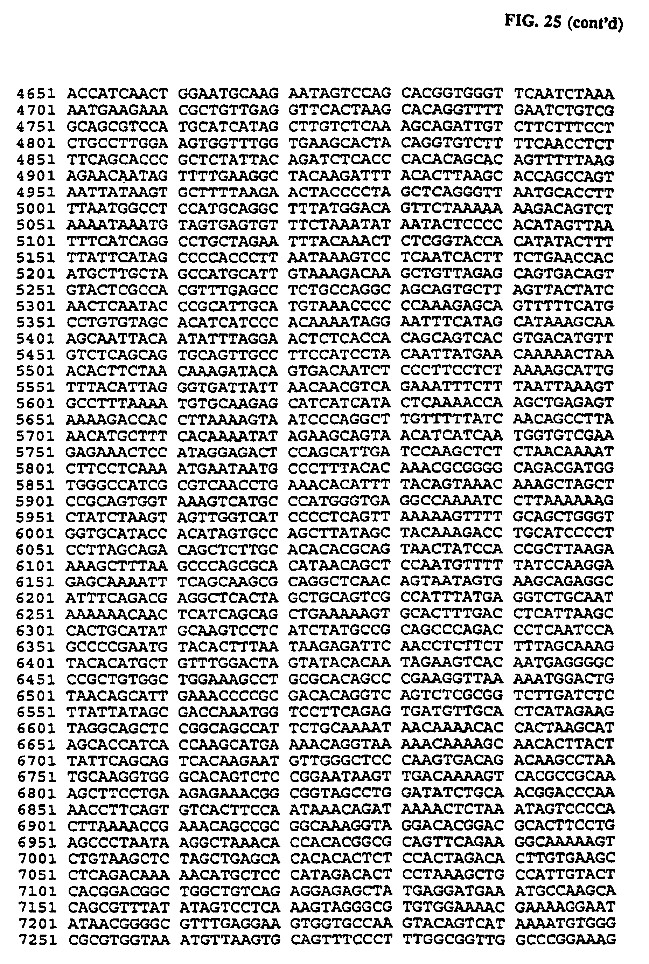

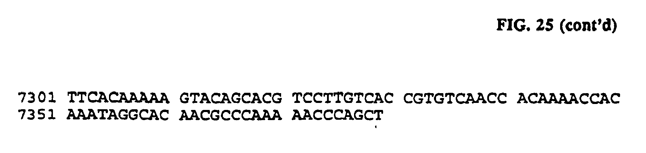

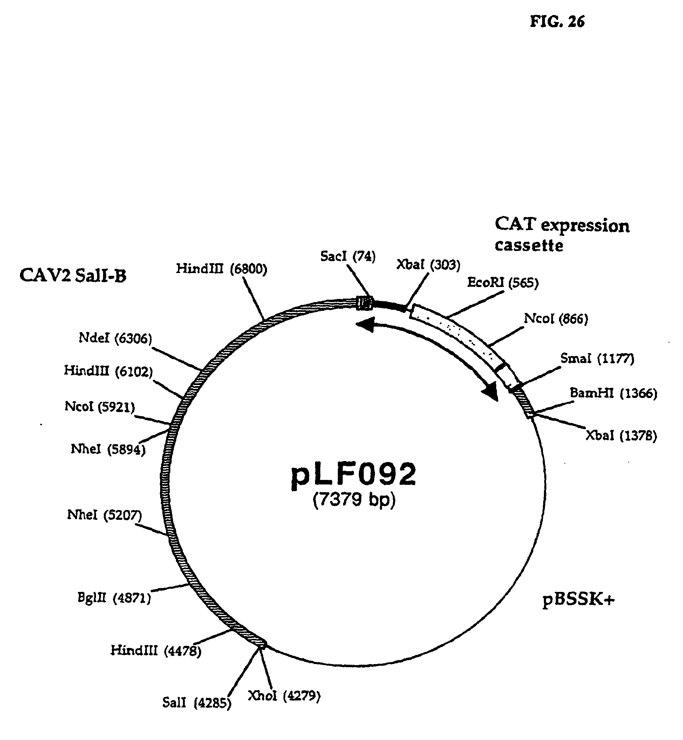

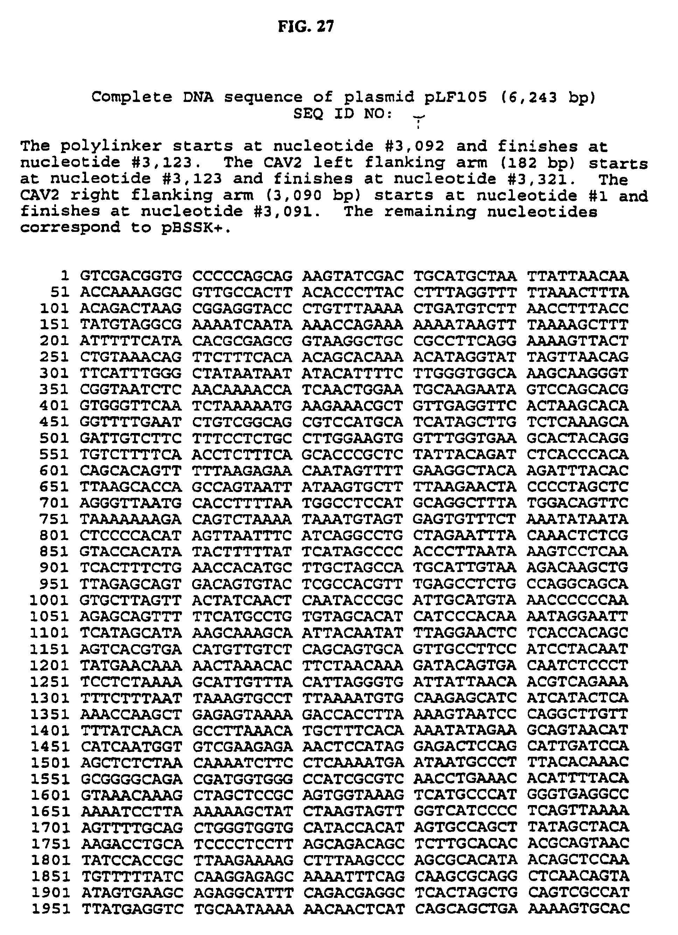

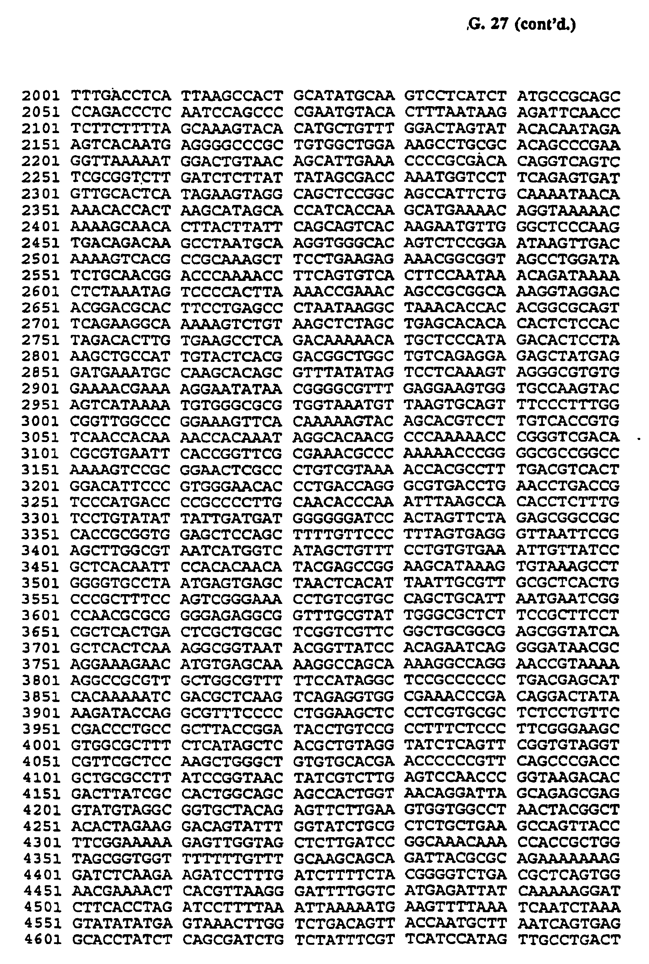

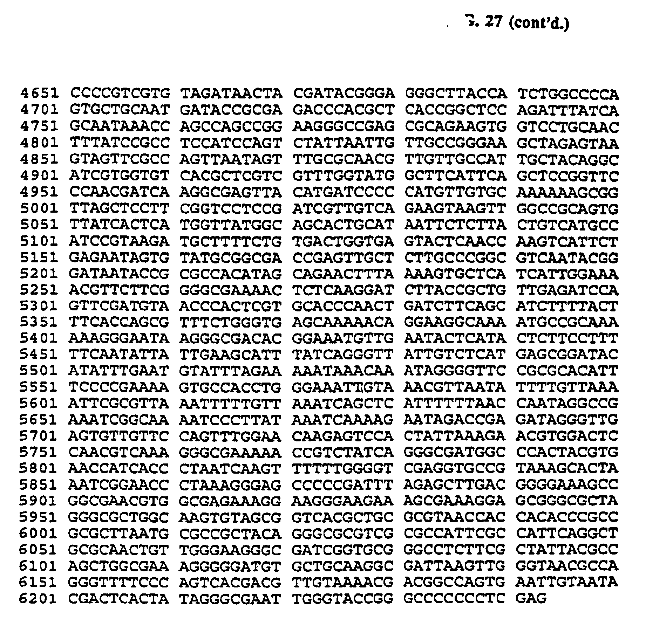

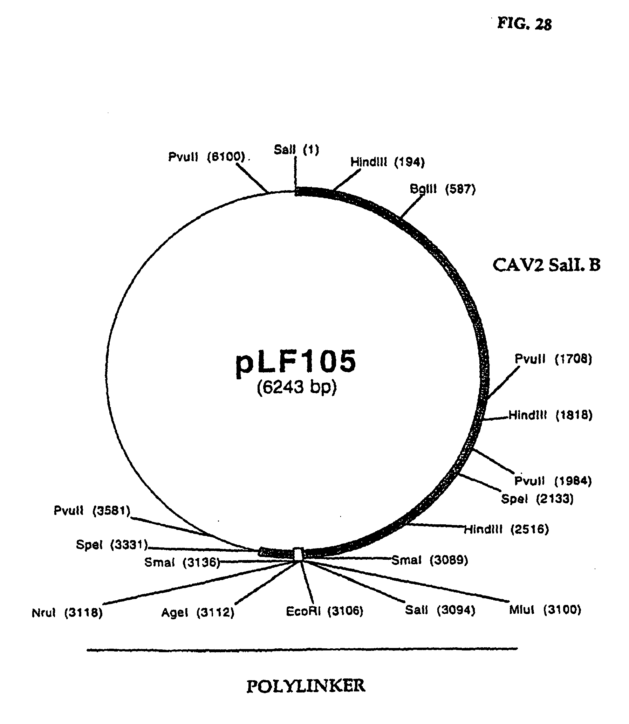

1.1 Generation of pLF022, an expression vector in which the CAT reporter gene has been placed under the control of a subfragment (145 bp) of the HCMV-IE promoter: DNA from human cytomegalovirus (hCMV) (Towns strain) was prepared as described in Lafemina et al.
   (1989). Amplification of the 3' end of the human cytomegalovirus immediate early promoter (hCMV-IE) was performed by PCR as previously described, using the primers pair LF172 (5'-ATCGTAAAGCTTAATGTCGTAATAACCCCGC-3')/LF159 (5'-TCTACTGCAGCCGGTGTCTTCTATGGAGGTCA-3') and hCMV DNA (10ng) as template. The resulting 166 bp DNA fragment was subsequently digested with PstI and HindIII before being purified using Gene Clean procedure and directly ligated with the 4,348 bp PstI/HindIII DNA fragment of pCAT-Basic Vector (Promega, Madison, WI), generating pLF022 (Figures 13, 14, SEQ ID NO: 7). The regulatory sequences present in the pLF022 expression cassette are a 145 bp fragment of the hCMV IE promoter and a 856 bp cassette containing the SV40 small t antigen and polyadenylation signal.
1.2 Generation of pLF062, a derivative of pLF022 in which the SV40 polyadenylation cassette has been reduced to 241 bp:
   In order to reduce the size of the SV40 small t antigen and polyadenylation signal cassette (856 bp) of pLF022, the following manipulations were performed. A 170 bp DNA fragment was amplified by PCR using primers LF377 (5'-TCTTCGCCCCCGTTTTCACCATGG-3') and LF378 (5'-ATCACGCCGCGGCTTAAAAAAATTACGCCCCGCCCT-3') and pLF022 DNA (10ng) as template. The purified amplified fragment was resuspended in 18 ml H₂O and incubated with 1U of Klenow enzyme (Boehringer Mannheim, Indianapolis, IN) for 30 minutes at room temperature in the presence of 800 µM dNTPs. The modified DNA fragment was phenol-chloroform extracted and recovered by ethanol precipitation before being digested with NcoI. The resulting 136 bp fragment was ligated with the 3,655 bp NcoI/BsaBI DNA fragment of pLF022, generating pLF062 (Figures 15, 16, SEQ ID NO: 8). pLF062 contains two repeats of the consensus polyadenylation signal AATAAA downstream of the CAT gene. The size of the CAT expression cassette in pLF062 is 1,119 bp as compared to 1,804 bp in pLF022. Regulatory sequences in pLF062 expression cassette are a 145 bp fragment of the hCMV-IE promoter and a 241 bp cassette containing the SV40 polyadenylation signal.
1.3 Generation of pLF066, a derivative of pLF062 in which the Ad2 TPL has been cloned downstream of the HCMV-IE promoter:
   In order to allow the expression of the reporter gene after the onset of CAV2 replication, pLF062 CAT expression cassette was modified by cloning the human Ad2 tripartite leader (Ad2 TPL) downstream of the hCMV-IE promoter transcription start site.
   Oligonucleotides SPH6ETr1 (5'-AATTCGGTACCAAGCTTCTTTATTCTATACTTAAAAAGTGAAAATAAATACAAAGGT TCTTGACTCTCTTC-3', SPH6ETr2 (5'-CGCATCGCTGTCTGCGAGGGCCAGCTGTTGGGCTCGCGGTTGAGGACAAACTCTTCG CGGTCTTTCCAGT-3'), SPH6ETr3 (5'-ACTCTTGGATCGGAAACCCGTCGGCCTCCGAACGTACTCCGCCACCGAGGGACCTGA GCGAGTCCGCATC-3'), SPH6ETr4 (5'-GACCGGATCGGAAAACCTCTCGAGAAAGGCGTCTAACC AGTCACAGTCGCAAGCCCGGGT-3'), SPH6ETr5 (5'-CTTTGTATTTATTTTCAC TTTTTAAGTATAGAATAAAGAAGCTTGGTACCG-3'), SPH6ETr6(5'GAAGAGT TTGTCCTCAACCGCGAGCCCAACAGCTGGCCCTCGCAGACAGCGATGCGGAAGAGAG TCAAGAAC-3'), SPH6ETr7 (5'-GCTCAGGTCCCTCGGTGGCGGAGTACGTTCGGAGGCCGACGGGTTTCCGATCCAAGA GTACTGGAAAGACCGC-3'), and SPH6ETr8 (5'-CTAGACCCGGGCTTGCGACTGTGACTGGTTAGACGCCTTTCTCGAGAGGTTTTCCGA TCCGGTCGATGCGGACTC-3,) were kinased and annealed and the 271 bp product was gel purified.
   The complete Ad2 TPL was subsequently amplified by PCR using primers LF394 (5'ATCGTCCTGCAGACTCTCTTCCGCATCGCTGTCTGC-3') and LF395 (5'-GCTCTAGACTTGCGACTGTGACTGGTTAG-3') and the gel purified annealed oligonucleotides as template.
   The resulting 220 bp DNA fragment was subsequently digested by PstI and XbaI before being purified using Gene Clean procedure as previously described and directly ligated with the 3,800 bp PstI/XbaI pLF062 fragment, generating pLF066 (Figures 17, 18, SEQ ID NO: 9). Regulatory sequences in pLF066 expression cassette are a 145 bp fragment of the hCMV-IE promoter in which the 5'UTR has been replaced by the 202 bp Ad2 TPL and a 241 bp cassette containing the SV40 polyadenylation signal.
1.4 Generation of pLF069, a derivative of pLF066 in which the HCMV-IE 5'UTR has been replaced by the Ad2 TPL: The HCMV-IE promoter 5'UTR (54 bp) present in pLF062 was deleted using the following procedure. Annealed oligonucleotides LF397 (5'-CGTTTAGTGAACCGTCTGCA- 3') and LF398 (5'-GACGGTTCACTAAACGAGCT-3') were ligated with the 3,936 bp DNA fragment of pLF062, generating pLF069 (Figure 19, 20, SEQ ID NO: 10). Regulatory sequences in pLF069 expression cassette are a 91 bp fragment of the HCMV-IE promoter in which the 5'UTR has been replaced by the 202 bp Ad2 TPL and a 241 bp cassette containing the SV40 polyadenylation signal.
1.5 Generation of pLF077, a derivative of pLF069 in which the SV40 polyadenylation cassette has been reduced to 153 bp:
   A 160 bp subfragment of SV40 polyadenylation sequences was amplified by PCR using oligonucleotides M13R (5'-GTAAAACGACGGCCAGT-3') and LF409 (5'-ATCGTCCCGCGGAATTGTTGTTGTTAACTTGTT-3') and pCAT Basic DNA (10ng) as template. The resulting 145 bp DNA fragment was subsequently digested by KspI and BamHI before being purified using Gene Clean procedure and directly ligated with the 3,716 bp KsoI/BamHI DNA fragment of pLF069, generating pLF077 (Figure 21, 22, SEQ ID NO: 11). The CAT expression cassette size in pLF077 is 1,161 bp as compared to 1,804 bp in pLF022 (36% reduction). Regulatory sequences in pLF069 expression cassette are a 91 bp fragment of the HCMV-IE promoter in which the 5'UTR has been replaced by the 202 bp Ad2 TPL and a 153 bp cassette containing part of the SV40 polyadenylation signal.
1.6 Generation of pLF091, a derivative of pLF077 in which the 3' end of the polyadenylation signal has been modified:
   The 12 bp (5'-TTTTTGGGCGTT-3') which are localised upstream of SmaI site at the 5' end of the right ITR sequence in the CAV2 genome were introduced downstream of the pLF077 polyadenylation cassette using the following procedure. A 1,000 bp DNA fragment was amplified by PCR using oligonucleotides LF423 (5'-ACGACCCGTAGAGGGCGTTGGACAGCAACTTGGCCTCGCGGTTGAGGACAAACTCTT -3') and LF432 (5'-ATCGTCCCCGGGTTTTTGGGCGTTATCCAGACATGATAAGATACA-3') and pLF077 DNA (10 ng) as template. The 1,000 bp PCR DNA fragment was Gene Clean purified and modified by Klenow treatment before being digested by NcoI. The PCR reaction was electrophoresed through a 1.2% agarose gel and the 295 bp fragment was subsequently isolated using Gene Clean procedure.
   pLF077 was digested by BamHI and subsequently modified by the action of Klenov enzyme before being digested by NcoI. The digestion reaction was electrophoresed through a 1% agarose gel and the 3,567 bp restriction fragment was isolated using Gene Clean procedure, before being ligated with the aforementionned 295 bp DNA fragment, resulting in pLF091 (Figures 23, 24, SEQ ID NO: 12).
1.7 Generation of pLF092, a CAT expression cassette donor plasmid:
   The 1,180 bp HindIII/SmaI DNA fragment of pLF091, which contains the entire CAT expression cassette, was modified by the action of Klenov enzyme and subsequently ligated with the 6.2 kbp SmaI linearized pLF056 to generate pLF092 (Figures 25, 26, SEQ ID NO: 13). This plasmid corresponds to a donor plasmid for the insertion of the CAT expression cassette into an insertion site 12 bp upstream of the SmaI site at the CAV2 genome 5' end.
1.8 Generation of pLF105, a donor plasmid for the insertion of foreign DNA 12 bp upstream of the SmaI site at the 5' end of the right ITR sequence in the CAV2 genome:
   A polylinker [NruI-AgeI-EcoRI-MluI-SalI-SmaI] constituted of preanneled oligonucleotides LF446 (5'-GGGTTTTTGGGCGTTTCGCGAACCGGTGAATTCACGCGTGTCGACCCC-3') and LF447 (5'-CCCAAAAACCCGCAAAGCGCTTGGCCACTTAAGTGCGCACAGCTGGGG-3') was ligated with the 6.2 kbp SmaI linearized pLF056 to generate pLF105 (Figures 27, 28, SEQ ID NO: 14).
1.9 Generation of recombinant CAV2 virus vCA3, which contains a CAT expression cassette inserted into the right terminal end of the CAV2 genome:
   Ten(10) µg of pLF092 were digested with HindIII and BamHI and the resulting 4.3 kbp DNA fragment was isolated using Gene Clean procedure and resuspended in H₂O to a concentration of 100 ng/µl. MDCK cells were transfected using the Lipofectamine based procedure. Solution A was prepared by mixing 0.4 µg of 4.3 kbp HindIII/BamHI pLF092 fragment with 4.4 µg of purified CAV2 DNA. Solution A total volume was brought to 300µl with supplemented serum free MEM medium. Transfected cells were harvested after 8 days and plated out on 150 mm diameter tissue culture dishes as previously described. A probe specific for the CAT reporter gene was generated by PCR using pCAT Basic DNA (10 ng) as template and primers pair LF218 (5'-ATCGTACATATGGAGAAAAAAATCACTGGATAT-3')/ LF231 (5'-ATCGTAGATATCCTCGAGTTACGCCCCGCCCTGCCACTC-3'). The resultant 660 bp DNA fragment was labelled by random priming using a procedure previously described and subsequently hybridized with nitrocellulose membrane to lift viral plaques, as previously described. A plaque crossreacting with the probe was picked and subsequently submitted to 4 rounds of plaque purification, as previously described. The plaque purified recombinant CAV2 virus was named vCA3.

### 2. Characterization of vCA3.

### 2.1. Analysis of CAT gene expression by recombinant virus vCA3.

### 2.1.1. Detection of CAT enzymatique activity in vCA3 infected MDCK cells lysates.

Purified vCA3 recombinant virus and wild-type CAV2 were used to independently infect 100% confluent MDCK monolayer (10⁶ cells) at a M.O.I. of 10. After 24 hours at 37°C in the presence of 5% CO2, the infected cultures were scraped and harvested. Cells pellets were washed 3 times with prewarmed (37°C) PBS (Ca²⁺ and Mg²⁺ free) before being resuspended in 1ml of 40 mM Tris-HCl, pH 7.5, 1 mM EDTA, pH 8.0 and 150 mM NaCl and incubated for 5 minutes at room temperature. The cells were subsequently centrifuged at 12 Kg for 30 seconds at 4°C and the resulting pellet was resuspended in 100 ml of 0.25M Tris-HCl, pH 8.0 before being subjected to 3 rapid freeze/thaw cycles with vigorous vortexing after each thaw cycle. Endogenous deacetyl activity was inactivated by incubating the lysates at 65°C for 10 minutes. The supernatants of a 12 Kg centrifugation for 2 minutes at RT were assayed in a chloramphenicol acetyltransferase (CAT) assay as follows. Twenty-five ml of cell lysate was incubated for 2 hours at 37°C with 3 ml of [¹⁴C] chloramphenicol (0.005 mCi/ml)(NEN, Boston, MA), 5 ml of n-Butyryl Coenzyme A (5 mg/ml) and 92 ml of 0.25 M Tris-HCl, pH 8.0. The reaction was terminated by adding 500 ml of ethyl acetate (Sigma, St Louis, MO) per tube. The reaction was vortexed with the mixed xylenes for 30 seconds and subsequently centrifuged at 12 K g for 1 minute. The upper, organic phase was transferred to a fresh tube and evaporated to dryness. The residue was resuspended in 25 ml of n-Butyryl Coenzyme A (5 mg/ml) and 10 ml of the resuspended material was subsequently dotted onto a silica gel thin layer chromatography (TCL) silica plate (Baker, Philisburg, NY). The slica plate chromatography was run in a closed chamber for approximately 1 hour, until the solvent was half-way up the plate. The silica plate was subsequently dried and autoradiogramed. Butyrylated chloramphenicol was clearly detected in the vCA2 sample whereas no modified chloramphenicol could be evidenced in the control wild-type CAV2 sample. This result demonstrates that the recombinant virus vCA3 expresses a functional CAT activity and thus validates both the expression cassette we have engineered and the insertion site we have selected.

### 2.1.2. Detection of CAT protein by radioimmnuprecipitation from vCA3 infected MDCK cells lysates.

Radioimmunoprecipitation analyses were performed as previously described (Pincus et al., 1992) using [³⁵S] methionine (1000 Ci/mmol, NEN)-labelled lysates derived from vCA3-infected MDCK cells and CAT rabbit polyclonal serum (5'3'Inc, Boulder, CO). The immunoprecipitated CAT polypeptide was resolved by SDS-PAGE and visualized by fluorography using sodium salicylate.

Analysis of vCA3 genomic organisation by restriction enzyme activity:
vCA3 DNA was purified as previously described. Purified total DNA was subsequently resuspended in H₂O to a final concentration of 1.3 µg/ml. 2 µg aliquots of purified vCA3 were independently digested with BglII and SalI. Since those two sites are unique within the CAV2 genome a 28.2 kbp and 3.8 kbp fragments are expected from the BglII digestion, whereas a 27.8 kbp and a 4.2 kbp fragments are expected from the SalI digestion. These restriction fragments are indeed observed demonstrating that vCA3 is a recombinant CAV2 virus which has incorporated 1,000 bp of the CAT expression cassette within the right end of its genome.

### EXAMPLE 13 - Generation of donor plasmid pLF102

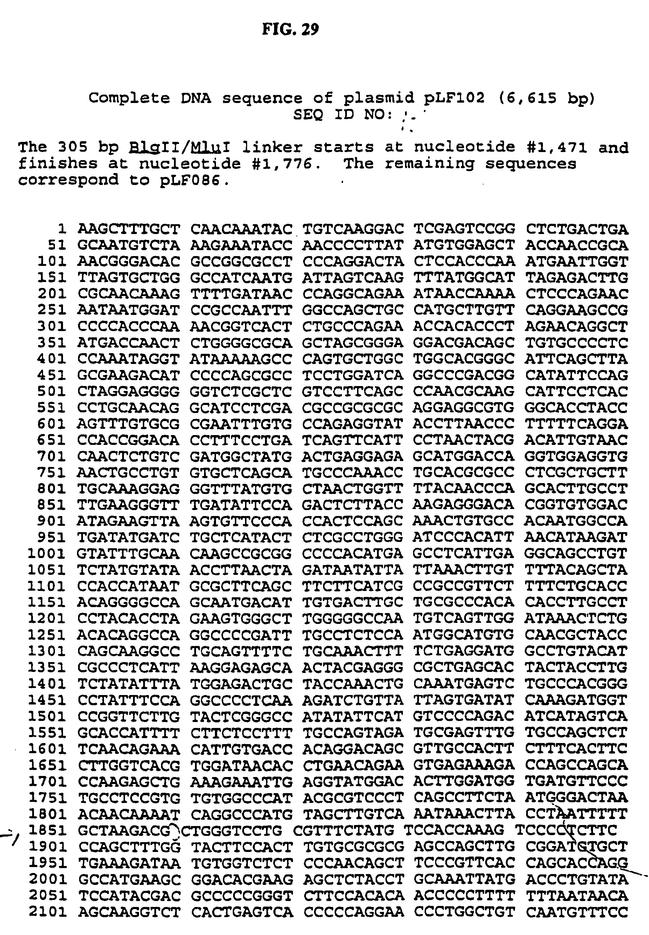

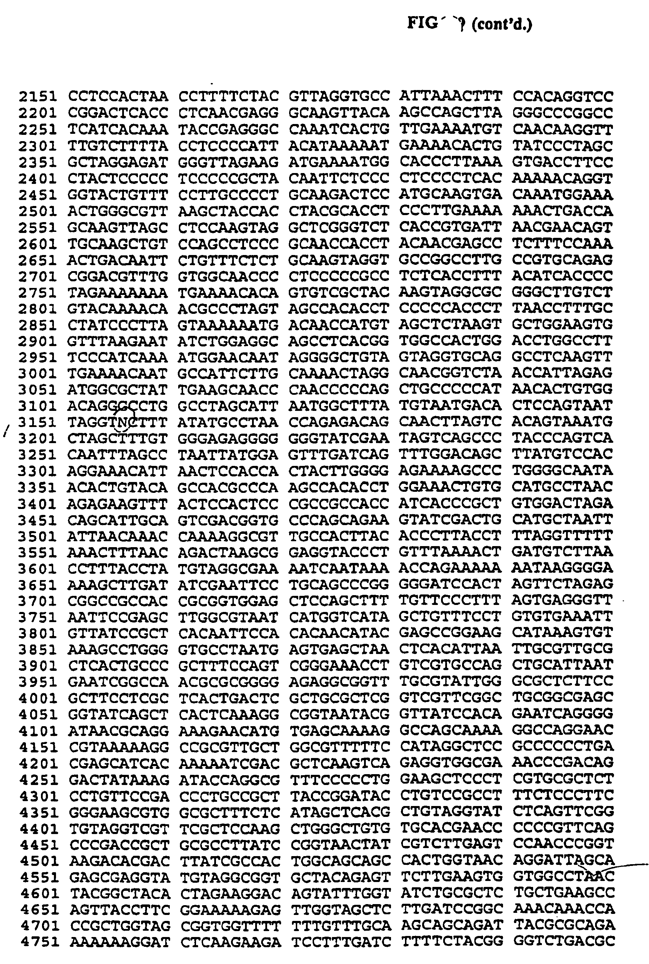

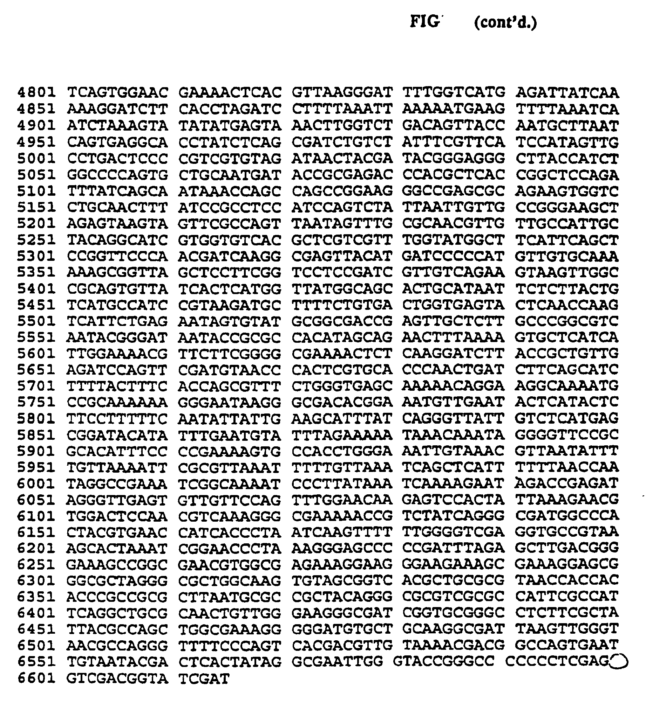

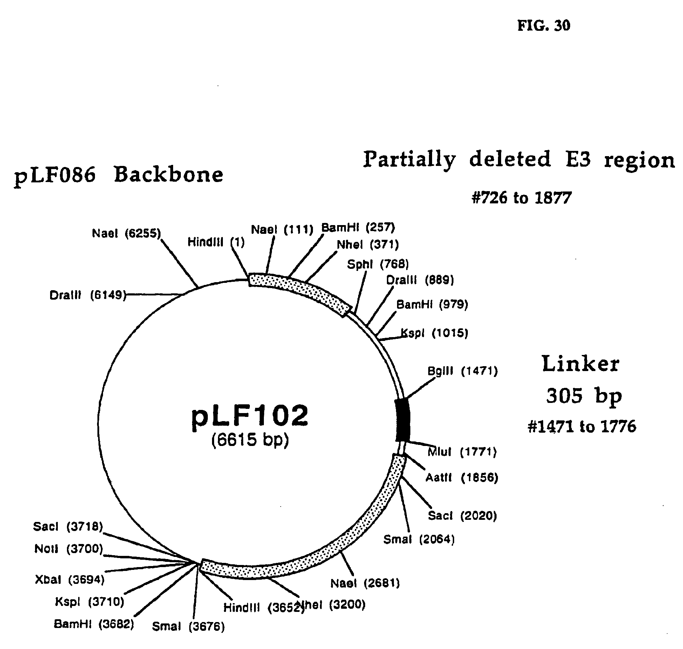

In order to delete the 3' end of the E3 ORF2 without modifying the E3 ORF1, the following procedure was developed.
A PCR amplification was set up using pLF027 DNA as a template and the primers pair LF437 (5'ATCTTAACGCGTCCCTCAGCCTTCTAATGGGAC 3') and LF334 (5' CTTGCTTGTTATTAAAAAAAG 3') as previously described. The 329 bp amplified DNA fragment was purified using the previously described Gene Clean procedure before being digested by MluI and SmaI. The resultant 287 bp MluI/SmaI DNA fragment was gel purified before being ligated with the 6,079 bp MluI/SmaI DNA fragment of pLF086, generating pLF095. The pLF095 63 bp BglII/MluI linker was subsequently swapped with a 305 bp BglII/MluI linker of unrelated foreign DNA using the following procedure. A 305 bp DNA fragment [nucleotide sequence described in Figures 29 and 30, see below] was obtained by digesting an unrelated plasmid with MluI and BglII. The MluI and BglII digested DNA fragment was gel purified and subsequently ligated with the 6,315 bp MluI/BglII DNA fragment of pLF095, generating pLF102 (Figure 29, SEQ ID NO: 15).

The engineering of pLF102 results in the exchange of a 688 bp fragment of CAV2 E3 (which represents 45% of the total E3 size) with 305 bp of foreign DNA and is useful to further define the limits of non-essential subdomains within CAV2 E3 region.

### EXAMPLE 14 - Generation of donor plasmid pLF116A

In order to delete a pLF027 EcoRV/AatII 1.8 kbp DNA fragment which contains two SphI restriction sites [at positions #3,770 and #3,870], the pLF027 EcoRV/AatII 5,163 bp fragment was gel purified and subsequently treated with Klenow enzyme before being religated on itself to generate pLF094.

A 24 bp DNA linker (5'-GATACGCGTTCCATTAGCAGATCT-3') containing unique BglII and MluI restriction sites was introduced into the pLF094 intergenic sequence between E3 ORF1 and E3 ORF2 by a double round PCR amplification procedure. Initial PCR amplifications were performed using pLF027 DNA as template and the following primer couples [LF243(5' CGCGCACAAACTGGTAGGTGC 3')/LF436(5' AGATCTGCTAATGGAACGCGTATCAAGTTTAATAATATTATC 3')] and [LF435(5' GATACGCGTTCCATTAGCAGATCTGTTTTACAGCTACCA 3')/LF277(5' GTACAGTTATGTTGAAGG 3')], to generate two partially overlapping DNA fragments of 487 bp and 698 bp, respectively. The second round of PCR amplification was performed in the presence of both partially overlapping purified DNA fragments and both external primers LF243 and LF277. The amplified 1,185 bp DNA fragment was digested with SphI and PstI and the resultant 566 bp PstI/SphI fragment was purified and ligated with the 4,653 bp SphI/PstI partial digest of pLF094, generating pLF093. All PCR amplifications were performed using the conditions previously described.

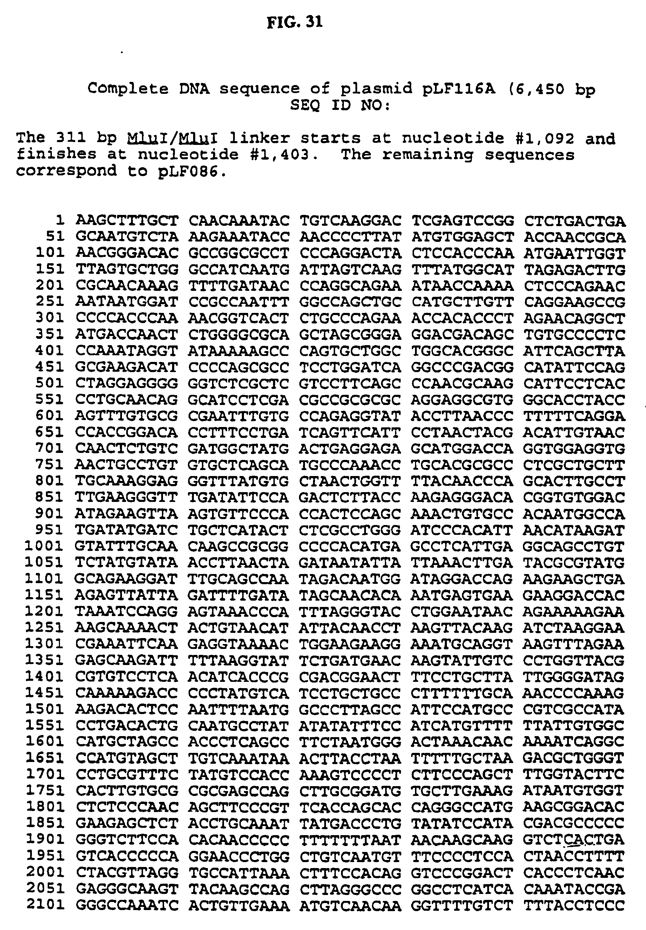

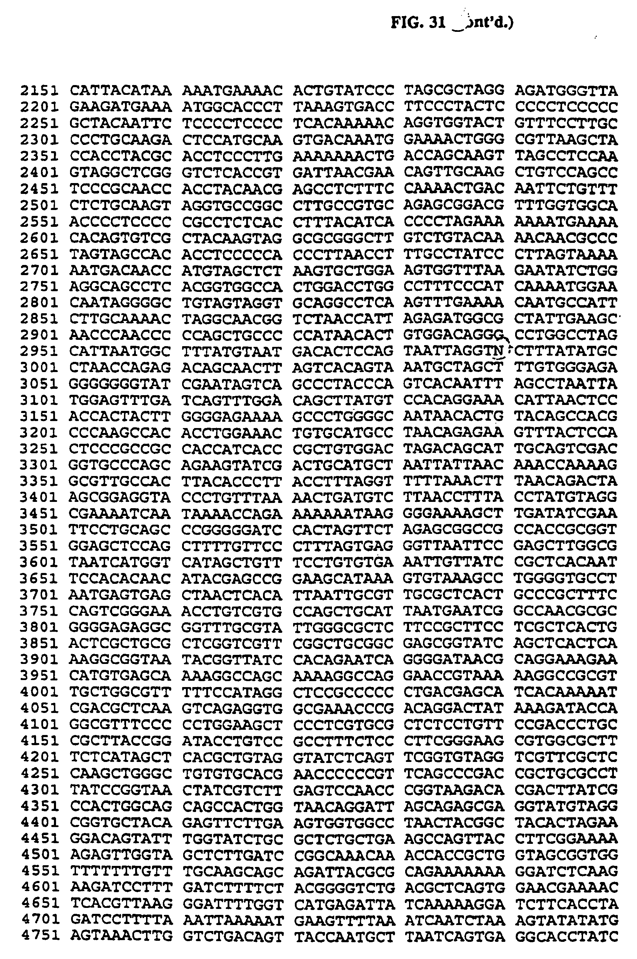

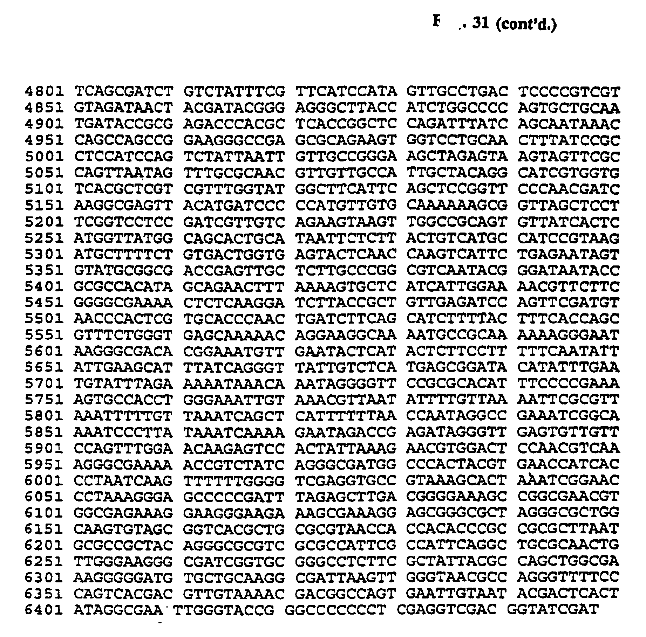

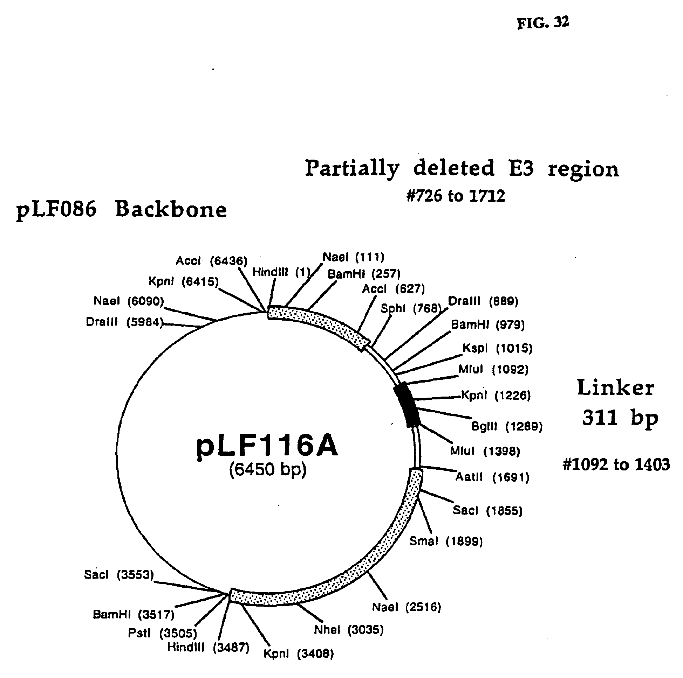

A deletion of the 5' end of E3 ORF2 without modifying E3 ORF1 was engineered by the following procedure. The pLF093 XhoI/MluI 1,062 bp fragment was gel purified and subsequently ligated with the 5,081 bp XhoI/MluI fragment of pLF086, generating pLF115. MluI linearized pLF115 DNA was subsequently ligated with a 311 bp MluI/MluI fragment of unrelated foreign DNA, generating pLF116A and B. The complete DNA sequence of pLF116A including the sequence of the unrelated 311 bp MluI/MluI fragment of foreign DNA is presented in Figure 31 (SEQ ID NO: 16), with the restriction map shown in Figure 32.

The engineering of pLF116A results in the exchange of a 876 bp fragment of CAV2 E3 (which represents 57% of the total E3 size) with 311 bp of foreign DNA and is useful to further define the limits of non-essential subdomains within CAV2 E3 region.

### EXAMPLE 14 - Generation of donor plasmid pLF100

In order to delete simultaneously the 5' end of the E3 ORF2, the 3' end of the E3 ORF1 and the complete E3 ORF3, a 634 bp fragment was deleted between the MluI(#1529) and DraIII(#889) restriction sites of pLF086 (Figures 7 and 8) and subsequently exchanged with a 302 bp fragment of unrelated foreign DNA using the following procedure.

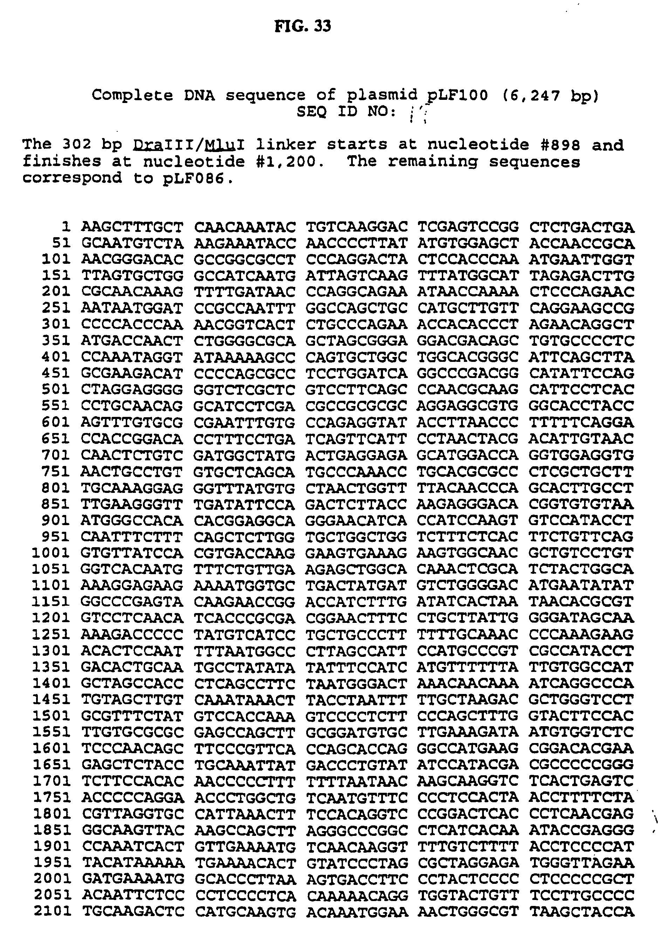

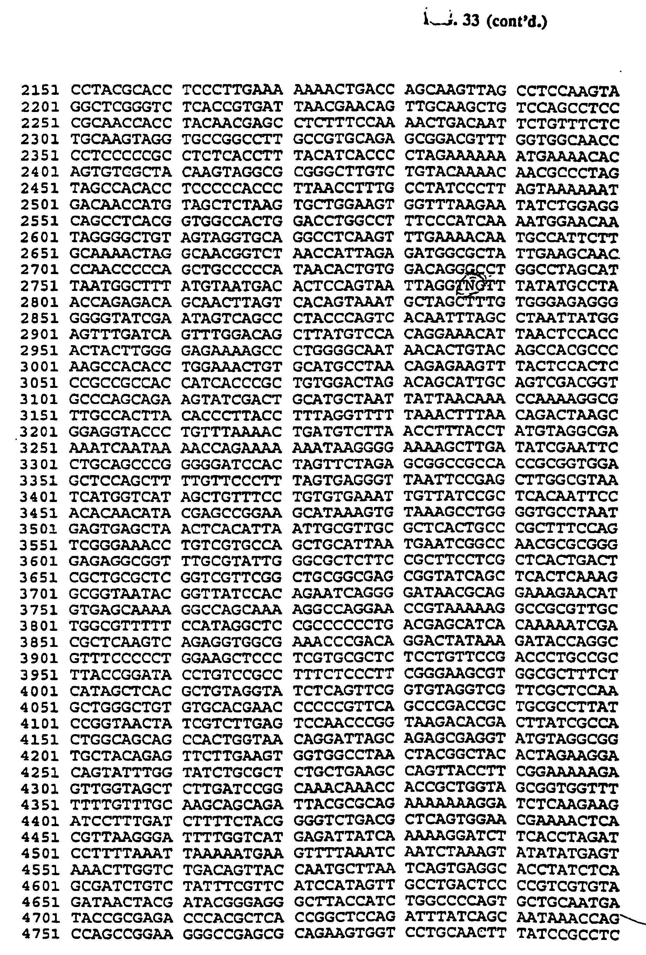

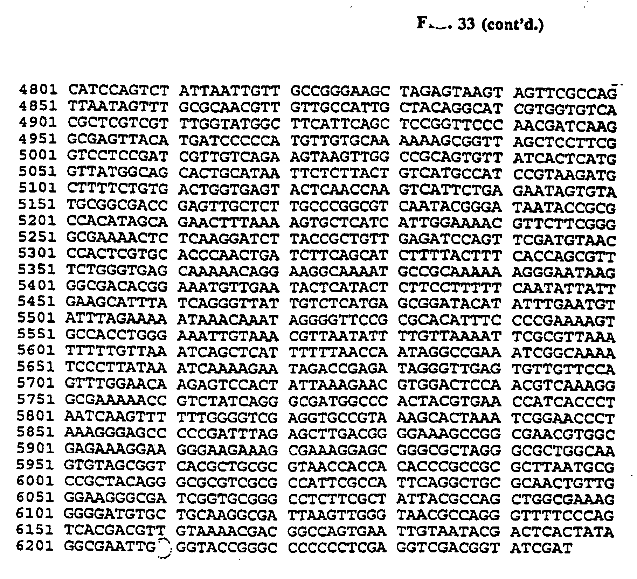

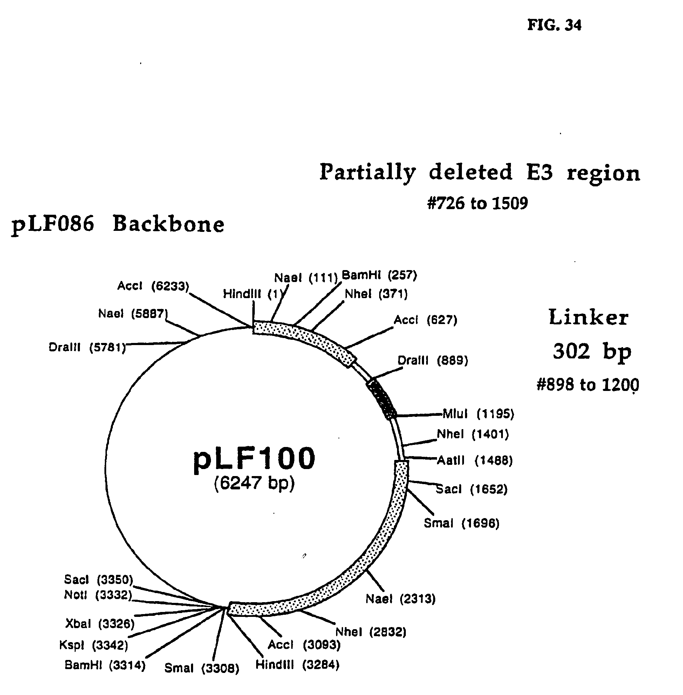

The 302 bp DNA fragment was obtained by digesting an unrelated plasmid with MluI and DraIII. The MluI and DraIII digested DNA fragment was gel purified and subsequently ligated with the 5,946 bp MluI/DraIII DNA fragment of pLF086, generating pLF100 (Figures 33, 34 SEQ ID NO: 17). The nucleotide sequence of the 302 bp fragment is shown in Figure 33, and the restriction map is shown in Figure 34.

The engineering of pLF100 results in the exchange of a 1,060 bp fragment of CAV2 E3 (which represents 69% of the total E3 size) with 302 bp of foreign DNA and is useful to further define the limits of non-essential subdomains within CAV2 E3 region.

### EXAMPLE 15 - Generation of donor plasmid pLF120

In order to delete simultaneously the 3' end of the E3 ORF1, the almost complete E3 ORF2 and the complete E3 ORF3, a 882 bp fragment was deleted between the MluI(#1,771) and DraIII(#889) restriction sites of pLF102 and subsequently exchanged with a 311 bp fragment of unrelated foreign DNA using the following procedure.

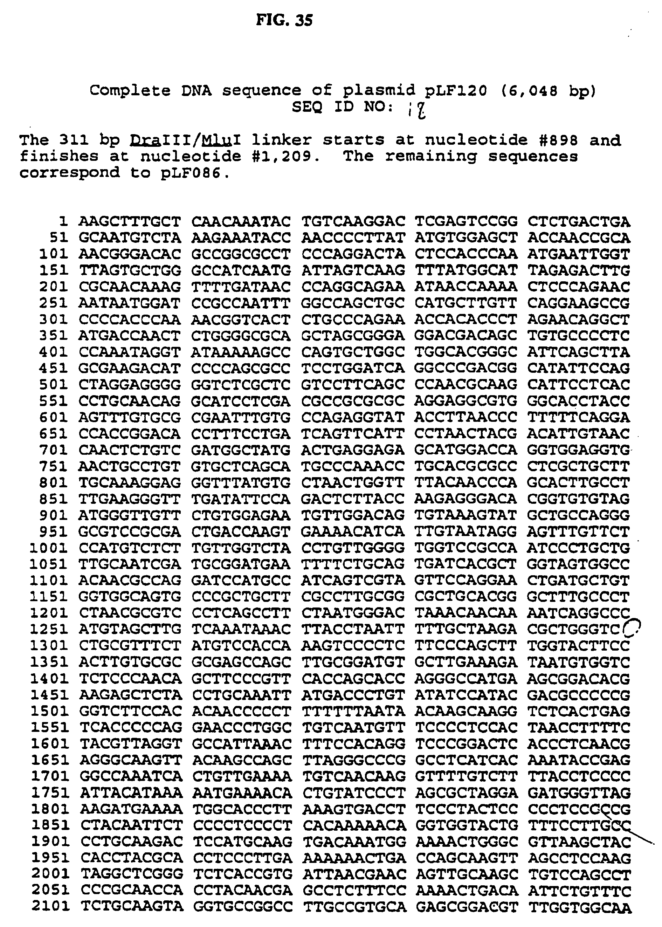

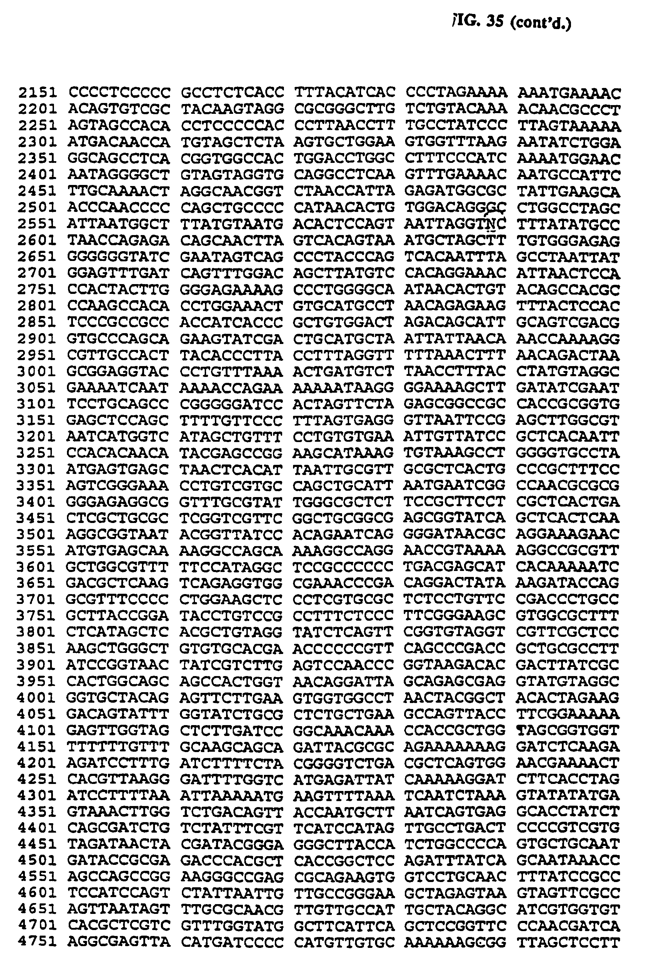

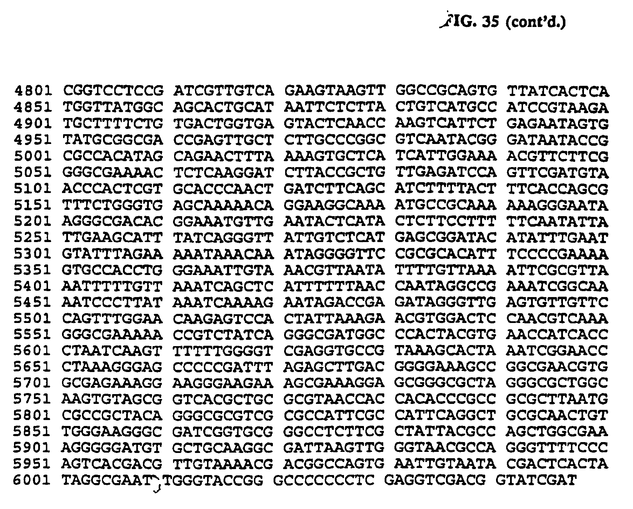

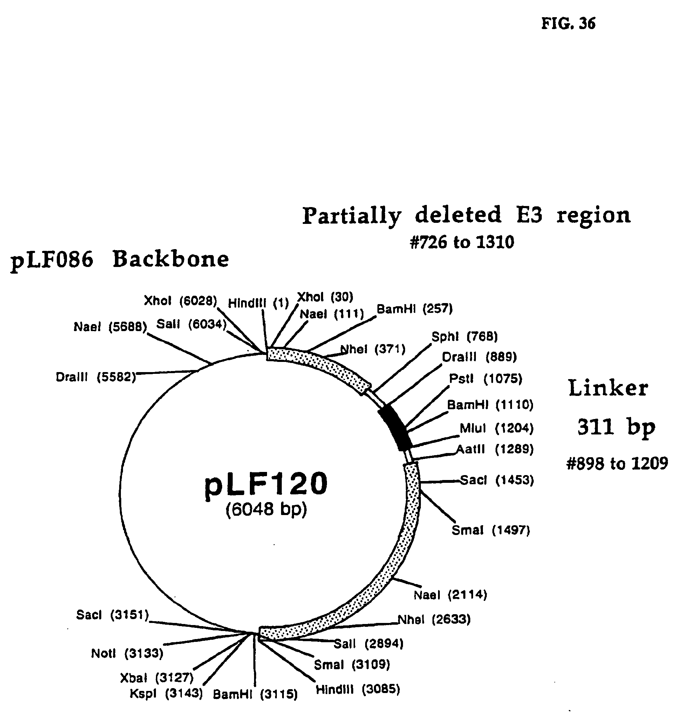

pLF102 DNA was linearized by MluI and subsequently partially digested with DraIII. The resultant 5,733 bp MluI/DraIII was subsequently ligated with a 311 bp MluI/DraIII fragment of unrelated foreign DNA, generating pLF120 (Figures 35, 36, SEQ ID NO: 18). The nucleotide sequence of the 311 bp MluI/DraII fragment of unrelated foreign DNA is shown in Figure 35, and the restriction map is shown in Figure 36.

The engineering of pLF120 results in the exchange of a 1,261 bp fragment of CAV2 E3 (which represents 82% of the total E3 size) with 311 bp of foreign DNA and is useful to further define the limits of non-essential subdomains within CAV2 E3 region. This is the largest deletion and indicates that practically all of the E3 region, e.g., about 80% to about 100%, such as up to about 80 to about 95% or up to about 80% to 90% or up to about 80% to 85% of the E3 region can be deleted.

### EXAMPLE 16 - Generation of pLF043, a pBSSK+ which contains the canine distemper virus (CDV) hemagglutinin (HA) coding sequence

### 1. Generation of plasmid pSDCDVHA.

The Onderstepoort strain of canine distemper virus (CDV) was obtained from Dr. M. Appel (cornell University, Ithaca, NY). RNA was harvested from CDV infected Vero cells and cDNA was prepared in the following manner.

RNA from CDV infected Vero cells was isolated by the guanidium isothiocyanate-cesium chloride method of Chirgwin, et al., (1979). First strand cDNA was synthesized with AMV reverse transcriptase (Life Sciences, St. Petersburg, FL), the oligonucleotide primer CDVFSP (SEQ ID NO: 44) (5'-CCAGGACATAGCAAGCCAACAGGTC-3'), and RNA from CDV infected cells. CDVFSP (SEQ ID NO: 44) primes 80 bp upstream of the CDV fusion (F) start codon, yielding a positive sense single stranded cDNA product which contains the F and hemagglutinin (HA) coding sequences.

The HA-specific open reading frame (ORF) was amplified from the first strand cDNA product by polymerase chain reaction (PCR) as previously described. Oligonucleotide primers CDVHA1 (SEQ ID NO: 45) (5'-CGATATCCGTTAAGTTTGTATCGTAATGCTCCCCTACCAAGAC-3') and CDVHA2 (SEQ ID NO: 46) (5'-GGGATAAAAATTAACGGTTACATGAGAATCTTATACGGAC-3') were used in a PCR with the CDVFSP derived first strand cDNA as template. CDVHA1 contains the 3' most region of the vaccinia virus H6 promoter (Perkus, et al., 1989) followed by a sequence which primes from the translation initiation codon into the CDV HA ORF. CDVHA2 (SEQ ID NO: 46) primes from the stop codon of the HA ORF toward the CDV HA 5' end. The resultant 1.8 kbp PCR product was treated with the Klenow fragment from the *E. coli* DNA polymerase, in the presence of 20mM dNTPs, to blunt end the fragment. The 1.8 kbp blunt-ended fragment was inserted between the NruI site within the H6 promoter, and the SmaI site 3' of the H6 promoter in pSD554 (see below). The resultant plasmid pCDVHA should have contained the H6 promoted CDV HA ORF, but there was an unexpected deletion at the CDV HA 5' end. Repair of the deletion is described below.

Plasmid pSD554 contains the vaccinia K1L host range gene (Gillard et al., 1986) and vaccinia H6 promoter followed by insertion sites, within flanking vaccinia arms. The flanking vaccinia arms replace the ATI region: open reading frames A25L and A26L (Goebel et al., 1990a,b). pSD554 was prepared in the following manner.

Left and right vaccinia flanking arms were constructed by PCR using the template pSD414 which contains vaccinia SalI B (Goebel et al., 1990a,b). The left arm was synthesized using oligonucleotide primers MPSYN267 (SEQ ID NO: 47) (5'-GGGCTGAAGCTTGCTGGCCGCTCATTAGACAAGCGAATGAGGGAC-3') and MPSYN268 (SEQ ID NO: 48) (5'-AGATCTCCCGGGCTCGAGTAATTAATTAATTTTTATTACACCAGAAAAGACGGCTTG AGA T C-3') in a PCR with template pSD414. The right arm was synthesized using oligonucleotide primers MPSYN269 (SEQ ID NO: 49) (5'-TAATTACTCGAGCCCGGGAGATCTAATTTAATTTAATTTATATAACTCATTTTTTGA ATA T ACT-3') and MPSYN270 (SEQ ID NO: 50) (5'-TATCTCGAATTCCCGCGGCTTTAAATGGACGGAACTCTTTTCCCC-3') in a PCR with template pSD414. The two PCR-derived fragments containing the left and right arms were combined in a PCR. The resultant PCR product was digested with EcoRI and HindIII and a 0.9kbp fragment was isolated. The 0.9kb fragment was inserted between the pUC8 EcoRI and HindIII sites. The resultant plasmid pSD541 received the K1L gene, and additional insertion sites, in the following manner.

Plasmid pSD541 was digested with BglII and XhoI and ligated with annealed complementary oligonucleotides MPSYN333 (SEQ ID NO: 51) (5'-GATCTTTTGTTAACAAAAACTAATCAGCTATCGCGAATCGATTCCCGGGGGATCCGG TACC C-3') and MPSYN334 (SEQ ID NO: 52) (5'-TCGAGGGTACCGGATCCCCCGGGAATCGATTCGCGATAGCTGATTAGTTTTTGTTAA CAA A A-3'), generating plasmid pSD552. pSD452 (Perkus et al., 1990) contains the K1L gene. pSD452 was digested with HpaI and partially digested with BglII and the resultant 1kbp fragment containing the K1L gene was inserted between the pSD552 BglII and HpaI sites. The resultant plasmid pSD553 was digested with NruI and a SmaI/NruI fragment containing the vaccinia H6 promoter (Perkus et al., 1989) was inserted. The resultant plasmid, pMP553H6, contains the vaccinia H6 promoter downstream from the K1L gene within the A26L insertion locus.

Plasmid pMP553H6 was digested with NruI and BamHI and ligated with annealed synthetic oligonucleotides MPSYN347 (SEQ ID NO: 53) (5'-CGATATCCGTTAAGTTTGTATCGTAATCTGCAGCCCGGGGGGG-3') and MPSYN348 (SEQ ID NO: 54) (5'-GATCCCCCGGGCTGCAGATTACGATACAAACTTAACGGATATCG-3'). The resultant plasmid pSD554 contains the K1L gene and the H6 promoter followed by insertion sites, within flanking vaccinia sequences which replace the ATI region.

The vaccinia virus H6 promoter and 5' end of the CDV HA ORF were added to pCDVHA as a PCR derived fragment. The ATG of the regulatory region H6 overlaps the CDV HA translation initiation codon in the PCR derived fragment. The vaccinia virus H6 promoter has been described in Perkus, et al., 1989.

pEIVC5L contains the modified H6 promoter and a nonpertinent gene. pEIVC5L was used in a polymerase chain reaction with oligonucleotide primers H65PH (SEQ ID NO: 55) (5'-ATCATCAAGCTTGATTCTTTATTCTATAC-3') and CDVHAH6 (SEQ ID NO: 56) (5'-GTCTTGGTAGGGGAGCATTACGATACAAACTTAACG-3') to generate a 156bp fragment. CDVHAH6 contains the 5' 18 base pairs of CDV HA followed by a sequence which primes from the translation initiation codon toward the H6 promoter 5' end. H65PH (SEQ ID NO: 55) contains a HindIII site followed by a sequence which primes from the H6 promoter 5' end toward the 3' end. The 156 base pair PCR-derived H65PH/CDVHAH6 (SEQ ID NO: 55/SEQ ID NO: 56) product contains the H6 promoter and the 5' 18 base pairs of the CDV HA coding sequence.

The CDVFSP (SEQ ID NO: 44) first strand cDNA product was used in a PCR with oligonucleotide primers CDVHAATG (SEQ ID NO: 57) (5'-ATGCTCCCCTACCAAGAC-3') and CDVHAECO (SEQ ID NO: 58) (5'-GTAATTAGTAAAATTCACCTTG-3') to generate a 459 base pair fragment. CDVHAATG (SEQ ID NO: 57) primes from the translation initiation codon toward the CDV HA 3' end. CDVHAECO (SEQ ID NO: 58) primes from position 583 of the following H6 promoted CDV HA sequence toward the CDV HA 5' end. The 156 base pair and 459 base pair PCR-derived fragments were pooled and used in a PCR with H65PH (SEQ ID NO: 55) and CDVHAECO (SEQ ID NO: 58) to generate a 597 base pair fragment. The PCR-derived product was digested with HindIII and EcoRI, generating a 520 base pair fragment which contains the H6 promoter and 5' most 387 base pairs of the CDV HA coding sequence. The 520 base pair HindIII/EcoRI digested PCR fragment was inserted between the HindIII and EcoRI sites of pBSSK+ , yielding pBSCDVHA5S. Plasmid pBSCDVHA5S contains the H6 promoted 5' end of the CDV HA ORF in pBSSK+, and the 3' end of the CDV HA ORF was added in the following manner.

Plasmid pCDVHA was digested with SmaI followed by partial digestion with EcoRI to generate a 1.4kbp fragment containing the 3' end of the CDV HA ORF. The 1.4kbp pCDVHA EcoRI/SmaI fragment was inserted between the EcoRI and SmaI sites of pBSCDVHA5S. The resultant plasmid pBSCDVHA was digested with BamHI and partially digested with XhoI to generate a 1.9kbp fragment containing the H6 promoted CDV HA open reading frame. The 1.9kbp BamHI/XhoI pBSCDVHA fragment was inserted between the BamHI and XhoI sites of pSD553 (see above). The resultant plasmid pSDCDVHA contains the H6 promoted CDV HA gene in the ATI insertion site.

### 2. Generation of pLF043.

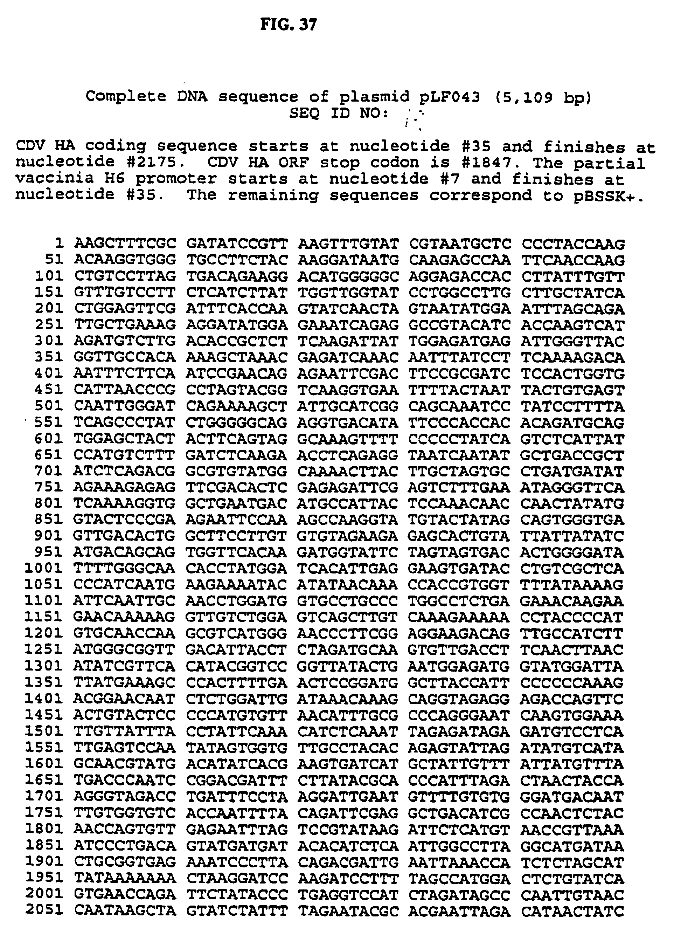

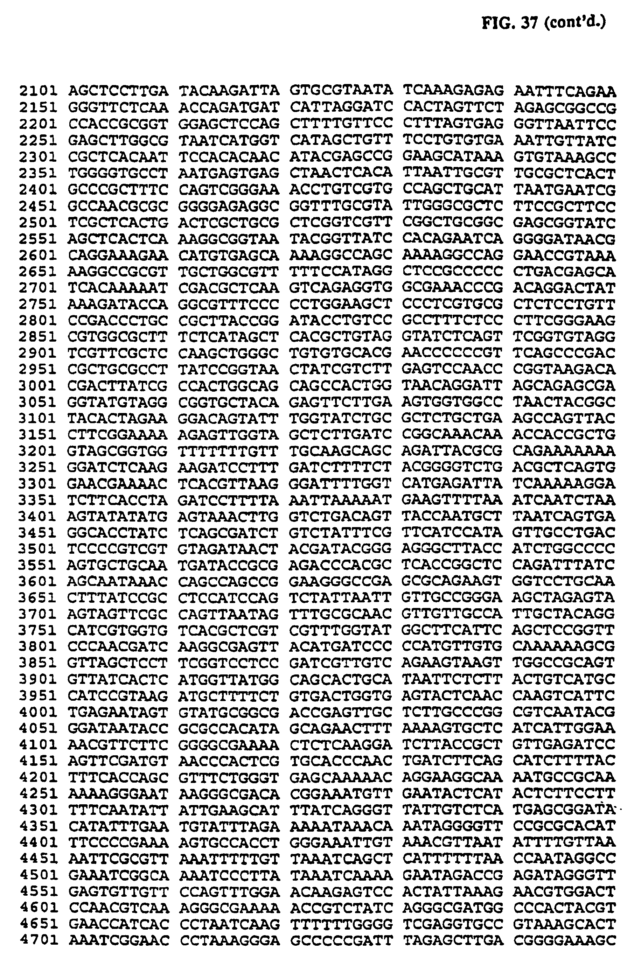

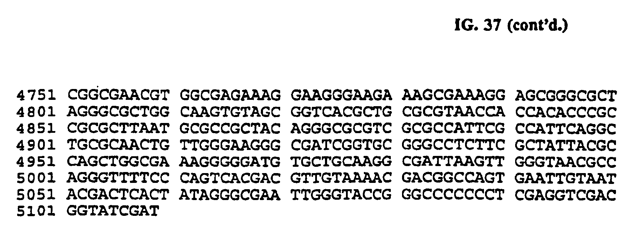

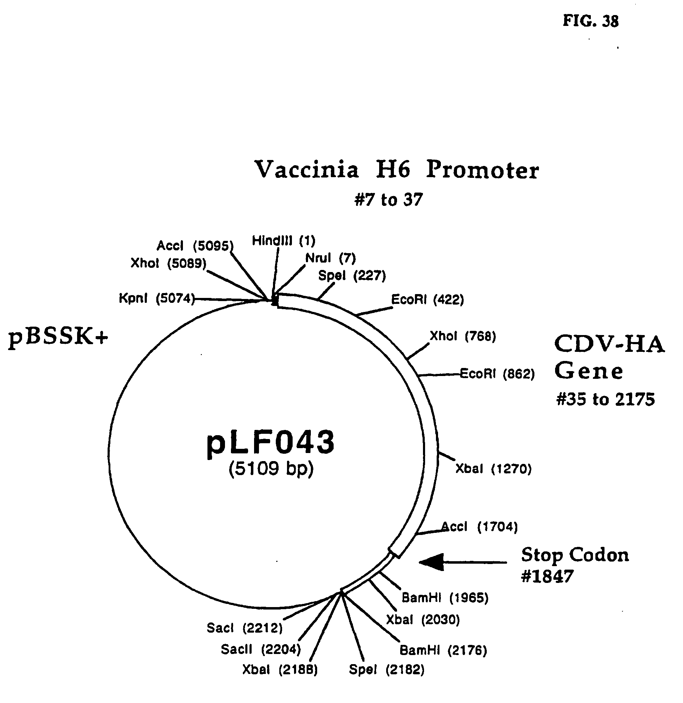

The pSDCDVHA 1,975 bp HindIII/BamHI which contains the CDV HA coding sequence and the 3' most region of the vaccinia virus H6 promoter, was gel purified and subsequently inserted between the corresponding restrictions sites of pBSSK+, generating pLF043 (Figures 37 and 38) (SEQ ID NO: 19).

### EXAMPLE 17 - Generation of pLF098, which contains a complete CDV HA expression cassette

A XbaI restriction site was engineered immediately upstream of the CDV HA initiation codon (ATG) in the following manner. A 409 bp DNA fragment was amplified by PCR using pLF043 DNA as a template and the primers pair LF412 (5' CTGATCTCTAGAATGCTCCCCTACCAAGACAAG 3') (SEQ ID NO: 59) and LF413 (5' TGGAGATCGCGGAAGTCG 3') (SEQ ID NO: 60) as previously described. The PCR amplified fragment was isolated using the Gene Clean procedure as previously described before being treated with the Klenow fragment from the *E. coli* DNA polymerase, in the presence of 20mM dNTPs and digested by SpeI and EcoRI. The resultant blunt-ended/SpeI 192 bp DNA fragment was subsequently ligated with the 4,891 bp NruI/SpeI fragment of pLF043, generating pLF096.

A KspI restriction site was engineered immediately downstream of pLF096 CDV HA stop codon (TAA) in the following manner. A 204 bp DNA fragment was amplified by PCR using pLF043 as a template and the primers pair LF438 (5' TGTTTATGACCCAATCG 3') (SEQ ID NO: 61) and LF439 (5' ATGCTCCCGCGGTTAACGGTTACATGAGAATCT 3') (SEQ ID NO: 62) as previously described. The PCR amplified fragment was isolated using the Gene Clean procedure as previously described before being digested with KspI and AccI. The resultant 143 bp DNA fragment was gel purified and subsequently ligated with the 4,594 bp KspI/AccI fragment of pLF096, generating pLF097.

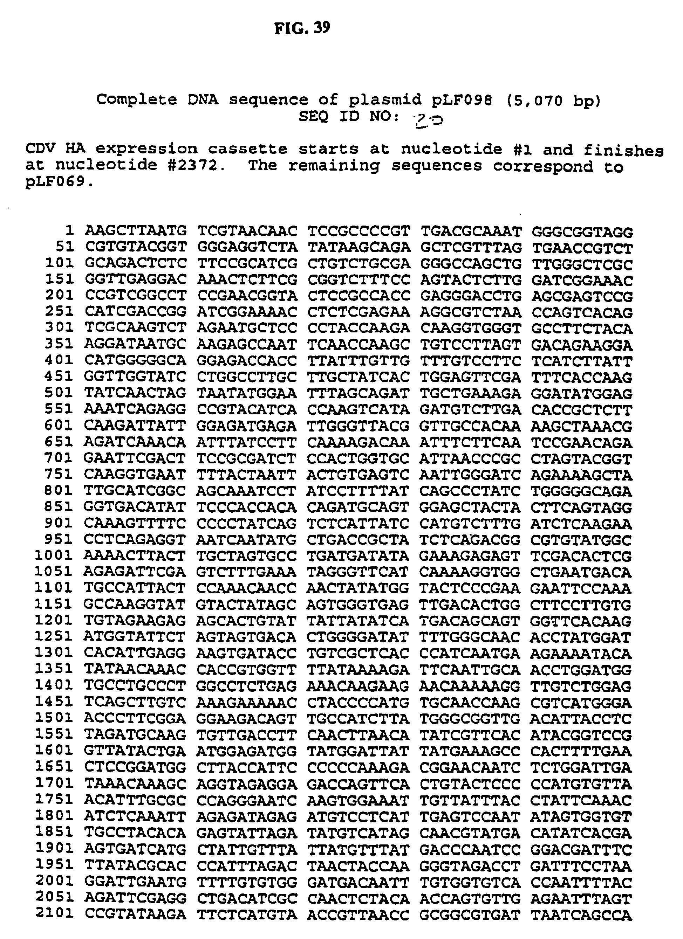

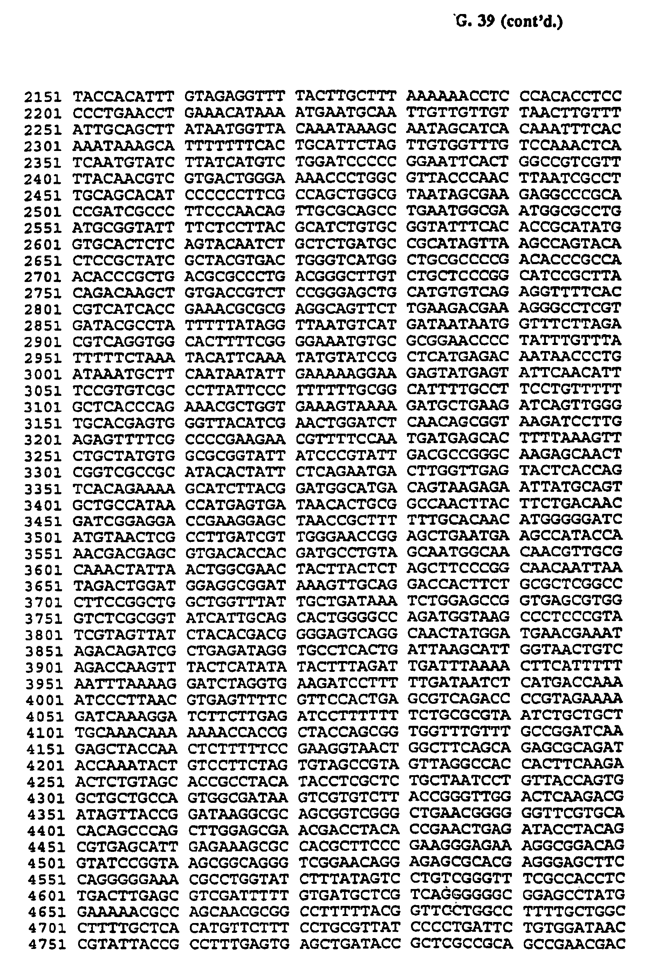

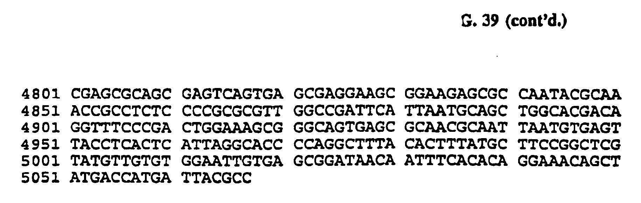

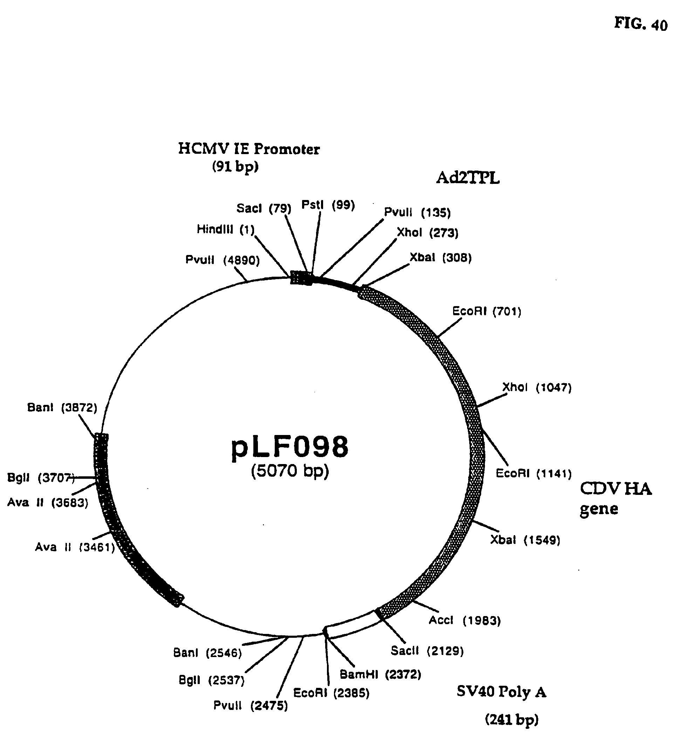

The 1,821 bp pLF097 KspI/XbaI fragment which contains the CDV HA coding sequence was subsequently ligated with the 3,246 bp KspI/XbaI fragment of pLF069, generating pLF098 (Figures 39, 40) (SEQ ID NO: 20).

### EXAMPLE 18 - Generation of pLP099A, a donor plasmid for the insertion of CDV XA expression cassette 12 bp upstream of the SmaI site at the CAV2 genome right end

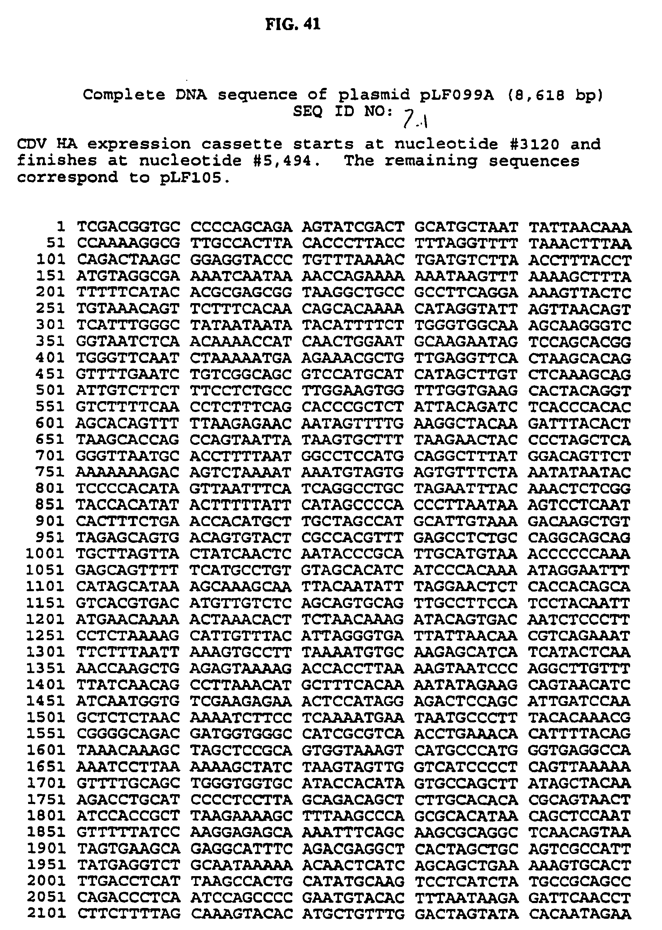

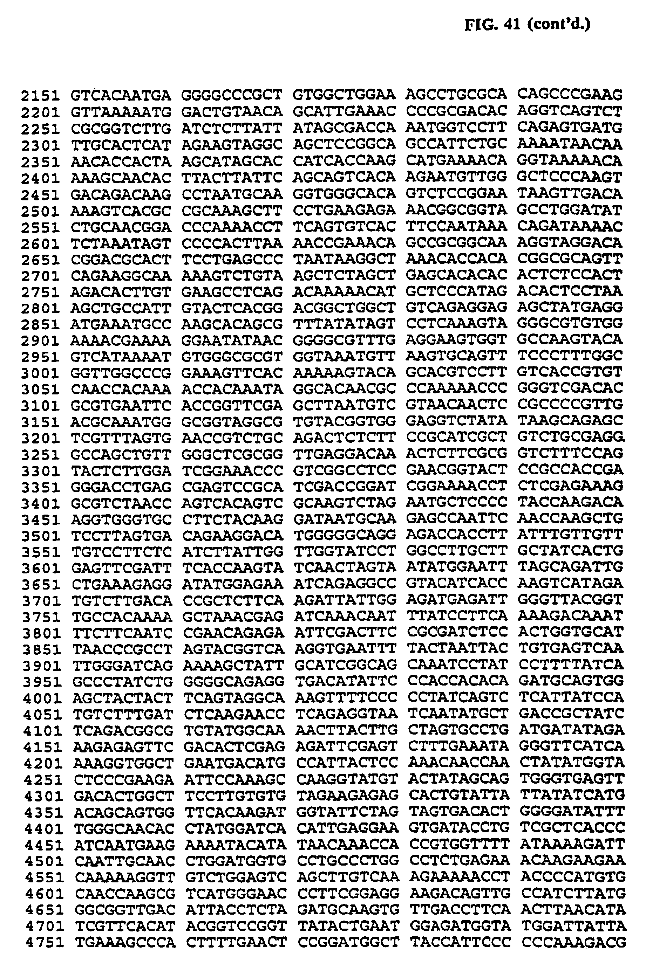

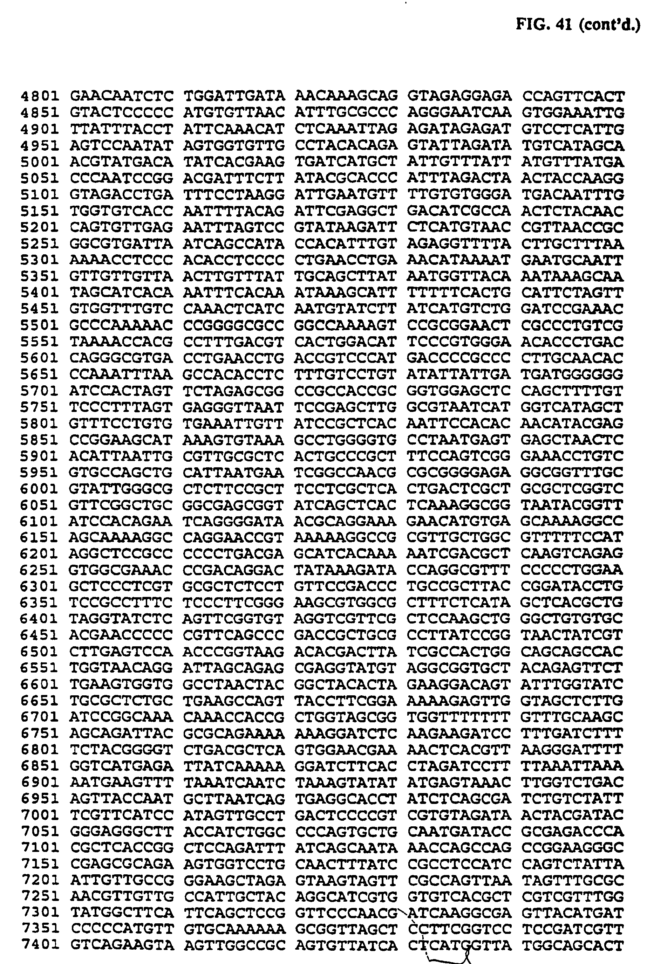

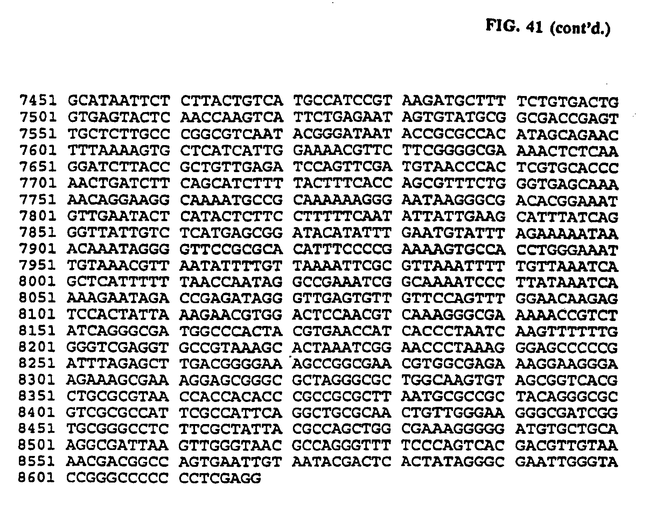

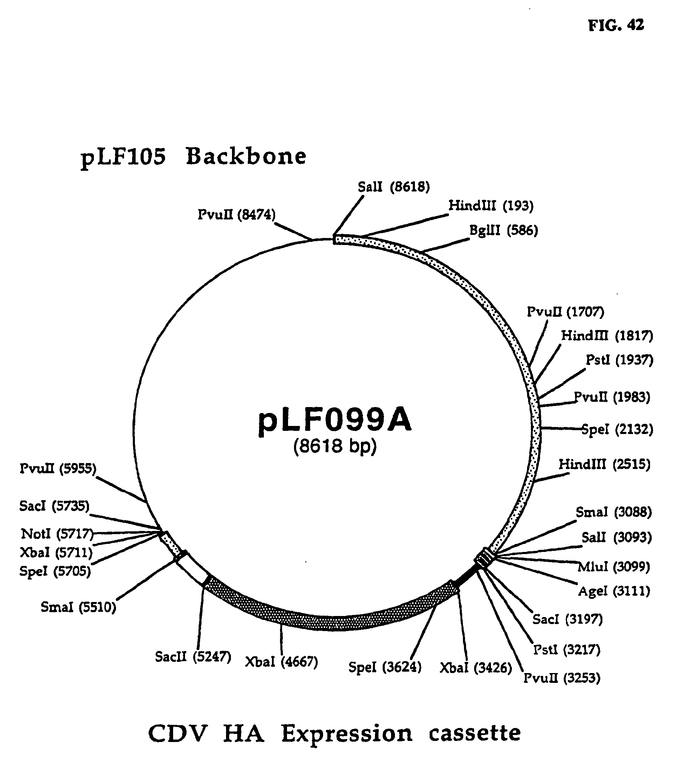

The 2,372 bp BamHI/HindIII pLF098 fragment which contains the CDV HA coding sequence coupled to the regulatory sequences defined in pLF069 was treated with the Klenow fragment from the *E*. *coli* DNA polymerase before being ligated with the 6,243 bp NruI linearized pLF105, generating pLF099A and pLF099B. pLF099A corresponds to the rightward orientation of the expression cassette (Figures 41, 42) (SEQ ID NO: 21).

### EXAMPLE 19 - Generation and characterization of recombinant CAV2 virus vCA4

### 1. Generation of recombinant CAV2 virus vCA4

Ten µg of pLF102 were digested with HindIII and the resulting 3,652 bp DNA fragment was isolated using Gene Clean procedure as previously described and resuspended in H₂O to a concentration of 100 ng/µl. MDCK cells were transfected using the Lipofectamine based procedure previously described. Solution A was prepared by mixing 0.5 µg of 3.6 kbp HindIII DNA fragment with 3 µg of purified vCA2 DNA. Solution A total volume was brought to 300µl with supplemented serum free MEM medium. Transfected cells were harvested after 8 days and plate out on 150 mm diameter tissue culture dishes as previously described. A probe specific for the 305 bp fragment of foreign DNA inserted into pLF102 was generated by PCR using pLF102 DNA (10 ng) as template and primers pair LF440 (5'-ATCAGTACGCGTATGGGCCACACACGGAGG-3')/ LF441 (5'-ATCAGTAGATCTGTTATTAGTGATATCAAA-3'). The resultant 305 bp DNA fragment was labelled by random priming using a procedure previously described and subsequently hybridized with nitrocellulose membrane used to lift viral plaques as previously described. Five viral plaques crossreacting with the probe were picked and subsequently submitted to 4 rounds of plaque purification as previously described. The plaque purified recombinant CAV2 virus was named vCA4.

### 2. Characterization of vCA4

vCA4 DNA was purified as previously described. Purified total DNA was subsequently resuspended in H₂O to a final concentration of 1.9 µg/ml. 2 µg aliquots of purified vCA4 were digested with HindIII. The expected 3, 667 bp HindIII fragment was visualized in the vCA4 sample whereas a 4.0 kbp fragment was present in the wild-type CAV2 sample, proving that vCA4 genomic DNA contains the partially deleted E3 region described in pLF102. VCA4 DNA was analyzed by Southern Blot which indicated that vCA4 has an E3 region 371 bp shorter than the wild-type E3 region.

This result futher demonstrates non-essential subdomains of CAV2 E3 region. More specifically, the derivation of vCA4 demonstrates that the CAV2 E3 sequences comprised between position #1,470 and position #2,157 [ie 45% of the E3 region], as in pLF027 (see Figure 1, SEQ ID NO: 1) can be exchanged with heterologous DNA. It also further validates the CAV2 E3 as an insertion site within the CAV2 genome. This results also proves that part of the CAV2 E3 region can be deleted to compensate for the introduction of foreign DNA into the right end of CAV2 genome as previously described in the derivation of vCA3.

### EXAMPLE 20 - Generation and characterization of recombinant CAV2 virus vCA5

### 1. Generation of recombinant CAV2 virus vCA5

Ten µg of pLF116A were digested with HindIII and the resulting 3,487 bp DNA fragment was isolated using Gene Clean procedure as previously described and resuspended in H₂O to a concentration of 100 ng/µl. MDCK cells were transfected using the Lipofectamine based procedure previously described. Solution A was prepared by mixing 0.5 µg of 3.5 kbp HindIII DNA fragment with 3 µg of purified vCA2 DNA. Solution A total volume was brought to 300µl with supplemented serum free MEM medium. Transfected cells were harvested after 8 days and plate out on 150 mm diameter tissue culture dishes as previously described. A probe specific for the 311 bp fragment of foreign DNA inserted into pLF116A was generated by PCR using pLF116A DNA (10 ng) as template and primers pair LF453(5'-ATCGTCATTGCCACGCGTATGGCAGAAGGATTTGCAGCCAAT-3')/ LF454 (5'-ATCGTCATTGCCACGCGTAACCAGGGACAATACTTGTTCATC-3'). The resultant 311 bp DNA fragment was labelled by random priming using a procedure previously described and subsequently hybridized with nitrocellulose membrane used to lift viral plaques as previously described. Five viral plaques crossreacting with the probe were picked and subsequently submitted to 4 rounds of plaque purification as previously described. The plaque purified recombinant CAV2 virus was named vCA5.

### 2. Characterization of vCA5

vCA5 DNA is purified as previously described. Purified total DNA is subsequently resuspended in H₂O to a final concentration of 1.9 µg/ml. 2 µg aliquots of purified vCA5 are digested with HindIII. The expected 3,487 bp HindIII fragment is visualized in the vCA5 sample whereas a 4.0 kbp fragment is present in the wild-type CAV2 sample, proving that vCA5 genomic DNA contains the partially deleted E3 region described in pLF116A.

This result futher demonstrates non-essential subdomains of CAV2 E3 region. More specifically, the derivation of vCA5 demonstrates that the CAV2 E3 sequences comprised between position #1,088 and position #1,964 [ie 57% of the E3 region], as described in pLF027 (see figure 1, SEQ ID NO: 1) can be exchanged with heterologous DNA. It also further validates the CAV2 E3 as an insertion site within the CAV2 genome. This result also proves that part of the CAV2 E3 region can be deleted to compensate for the introduction of foreign DNA into the right end of CAV2 genome as previously described in the derivation of vCA3.

### EXAMPLE 21 - Generation and characterization of recombinant CAV2 virus vCA6

### 1. Generation of recombinant CAV2 virus vCA6

Ten µg of pLF100 were digested with HindIII and the resulting 3,284 bp DNA fragment was isolated using Gene Clean procedure as previously described and resuspended in H₂O to a concentration of 100 ng/µl. MDCK cells were transfected using the Lipofectamine based procedure previously described. Solution A was prepared by mixing 0.5 µg of 3.3 kbp HindIII DNA fragment with 3 µg of purified vCA2 DNA. Solution A total volume was brought to 300µl with supplemented serum free MEM medium. Transfected cells were harvested after 8 days and plate out on 150 mm diameter tissue culture dishes as previously described. A probe specific for the 311 bp fragment of foreign DNA inserted into pLF100 was generated by PCR using pLF100 DNA (10 ng) as template and primers pair LF442(5'-ATCAGTCACGGTGTGTAAATGGGCCACACACGGAGG-3')/ LF443 (5'-ATCAGTACGCGTGTTATTAGTGATATCAAA-3'). The resultant 302 bp DNA fragment was labelled by random priming using a procedure previously described and subsequently hybridized with nitrocellulose membrane used to lift viral plaques as previously described. Five viral plaques crossreacting with the probe were picked and subsequently submitted to 4 rounds of plaque purification as previously described. The plaque purified recombinant CAV2 virus was named vCA6.

### 2. Characterization of vCA6

vCA6 DNA is purified as previously described. Purified total DNA is subsequently resuspended in H₂O to a final concentration of 1.9 µg/ml. 2 µg aliquots of purified vCA6 are digested with HindIII. The expected 3,284 bp HindIII fragment was visualized in the vCA6 sample whereas a 4.0 kbp fragment is present in the wild-type CAV2 sample, proving that vCA6 genomic DNA contains the partially deleted E3 region described in pLF100.

This result futher demonstrates non-essential subdomains of CAV2 E3 region. More specifically, the derivation of vCA6 demonstrates that the CAV2 E3 sequences comprised between position #898 and position #1,949 [ie 69% of the E3 region], as described in pLF027 (see Figure 1, SEQ ID NO: 1) can be exchanged with heterologous DNA. It also further validates the CAV2 E3 as an insertion site within the CAV2 genome. This results also proves that part of the CAV2 E3 region can be deleted to compensate for the introduction of foreign DNA into the right end of CAV2 genome as previously described in the derivation of vCA3.

### EXAMPLE 22 - Generation and characterization of recombinant CAV2 virus vCA7

### 1. Generation of recombinant CAV2 virus vCA7

Ten µg of pLF120 were digested with HindIII and the resulting 3,085 bp DNA fragment was isolated using Gene Clean procedure as previously described and resuspended in H₂O to a concentration of 100 ng/µl. MDCK cells were transfected using the Lipofectamine based procedure previously described. Solution A was prepared by mixing 0.5 µg of 3.3 kbp HindIII DNA fragment with 3 µg purified vCA2 DNA. Solution A total volume was brought to 300µl with supplemented serum free MEM medium. Transfected cells were harvested after 8 days and plate out on 150 mm diameter tissue culture dishes as previously described. A probe specific for the 311 bp fragment of foreign DNA inserted into pLF100 was generated by PCR using pLF100 DNA (10 ng) as template and primers pair LF458(5'-ATCCGTACGCGTTAGAGGGCAAAGCCCGTGCAGCAGCGC-3')/ LF459 (5'-ATCCGTCACGGTGTGTAGATGGGTTGTTTTGTGGAGAAT-3'). The resultant 311 bp DNA fragment was labelled by random priming using a procedure previously described and subsequently hybridized with nitrocellulose membrane used to lift viral plaques as previously described. Cross reacivity between the probe and viral DNA has been evidenced.

This result indicates that a deletion of 1,259 bp between position #898 and position #2,157, as described in pLF027 (see Figure 1, SEQ ID NO: 1) is compatible with viral replication in tissue culture, further showing that essentially all of the E3 region can be deleted.

### EXAMPLE 23 - Generation of vCA8

Ten µg of pLF099A were digested with BglII and NotI and the resulting 5,131 bp DNA fragment was isolated using Gene Clean procedure as previously described and resuspended in H₂O to a concentration of 100 ng/µl. MDCK cells were transfected using the Lipofectamine based procedure previously described. Solution A was prepared by mixing 0.5 µg of 5.1 kbp BglII/NotI DNA fragment with 3 µg of purified vCA2 DNA. Solution A total volume was brought to 300µl with supplemented serum free MEM medium. Transfected cells were harvested after 8 days and plate out on 150 mm diameter tissue culture dishes as previously described. The 440 bp EcoRI fragment of pSDCDVHA was labelled by random priming using a procedure previously described and subsequently hybridized with nitrocellulose membrane used to lift viral plaques as previously described. Two viral plaques cross-reacting with the probe were picked and are currently submitted to a plaque purification process as previously described. The plaque purified recombinant CAV2 virus is named vCA8.

### 2. Characterization of vCA8

vCA8 DNA purification, restriction digestion, Southern Blot, and CDV HA expression analysis by radioimmunoprecipitation confirm insertion and expression.

### EXAMPLE 24 - Generation of pLF108, a pBSSK+ derived plasmid which contains the canine distemper virus (CDV) fusion (F1) coding sequence

### 1. Generation of pATICDVF1

The CDV fusion (F) specific open reading frame (ORF) was amplified from cDNA by PCR using oligonucleotide primers CDVATGF1 (SEQ ID NO: 63) (5'-CATAAATTATTTCATTATCGCGATATCCGTTAAGTTTGTATCGTAATGCACAAGGGA ATCCCCAA AAGC-3') and CDVFT (SEQ ID NO: 64) (5'-ATCATCGGATCCATAAAAATCAGTGTGATCTCACATAGGATTTCGAAG-3') with CDVFSP (SEQ ID NO: 44) derived first strand cDNA as the template. CDVATGF1 (SEQ ID NO: 63) contains the 3' most region of the vaccinia virus H6 promoter (Perkus, et al., 1989) followed by a sequence which primes from the CDV F translation initiation codon into the CDV F ORF. CDVFT (SEQ ID NO: 64) contains a BamHI site followed by a sequence which primes from the CDV F stop codon toward the CDV F 5' end. The resultant PCR product was digested with NruI and BamHI, yielding a 2 kbp fragment which was inserted into pSD554 between the NruI and BamHI sites. The resultant plasmid pATICDVF1 contains the H6 promoted CDV F ORF in the vaccinia virus ATI insertion locus.

### 2. Generation of HC5LSP28

The C5 vector plasmid HC5LSP28 was constructed to remove the C5 ORF in the following manner. Oligonucleotide primers C5A (SEQ ID NO: 65) (5'-ATCATCGAATTCTGAATGTTAAATGTTATACTTTG-3') and C5B (SEQ ID NO: 66) (5'-GGGGGTACCTTTGAGAGTACCACTTCAG-3') were used in a PCR with genomic canarypox DNA as the template. The resultant 1.5 kbp fragment was digested at the C5A end with EcoRI and the other end remained blunt for insertion between the EcoRI and SmaI sites of pUC8, yielding plasmid C5LAB. Oligonucleotide primers C5C (SEQ ID NO: 67) (5'-GGGTCTAGAGCGGCCGCTTATAAAGATCTAAAATGCATAATTTC-3') and C5DA (SEQ ID NO: 68) (5'-ATCATCCTGCAGGTATTCTAAACTAGGAATAGATG-3') were used in a PCR with genomic canarypox DNA as template. The resultant 400 base pair fragment was digested at the C5DA end with PstI and the other end remained blunt for insertion between the SmaI and PstI sites of C5LAB, yielding plasmid pC5L. Annealed complementary oligonucleotides CP26 (SEQ ID NO: 69) (5'-GTACGTGACTAATTAGCTATAAAAAGGATCCGGTACCCTCGAGTCTAGAATCGATCC CGGGTTTT TATGACTAGTTAATCAC-3') and CP27 (SEQ ID NO: 70) (5'-GGCCGTGATTAACTAGTCATAAAAACCCGGGATCGATTCTAGACTCGAGGGTACCGG ATCCTTTT TATAGCTAATTAGTCAC-3') were inserted between the pC5L Asp718 and NotI sites. The resultant plasmid HC5LSP28 is a locus C5 vector plasmid.

### 3. Generation of pBSCDVHAVQ

Oligonucleotides RW132 (SEQ ID NO: 71) (5'-AGCTTCCCGGGTTAATTAATTAGTCATCAGGCAGGGCGAGAACGAGACTATCTGCTC GTTAATTA ATTAG-3') and RW133 (SEQ ID NO: 72) (5'-AGCTCTAATTAATTAACGAGCAGATAGTCTCGTTCTCGCCCTGCCTGATGACTAATT AATTAACC CGGGA-3') were annealed to form a double-stranded linker sequence. The RW132/RW133 (SEQ ID NO: 71/SEQ ID NO: 72) double-stranded sequence was inserted into the HindIII site 5' of the H6 promoted CDV HA ORF in pBSCDVHA5S, generating plasmid pBSCDVHAVQ.

### 4. Generation of pC5CDVHAF1

The 2 kbp pBSCDVHAVQ SmaI fragment, which contains the H6 promoted CDV HA ORF, was inserted into the HC5LSP28 SmaI site, generating plasmid pC5LCDVHA. The 2.1 kbp pATICDVF1 HpaI/BamHI fragment, containing the H6 promoted CDV F ORF, was ligated with the pC5LCDVHA SmaI/BamHI 6.5 kbp DNA fragment, generating plasmid pC5LCDVHAF1 which contains the H6 promoted CDV F and H₆ promoted CDV HA ORFs, with their transcripts directed away from each other, in the C5 locus.

### 6. Generation of vector plasmid pC6L

The C6 vector pC6L was constructed to remove the C6 ORF in the following manner. Oligonucleotide primers C6A1 (SEQ ID NO: 73) (5'-ATCATCGAGCTCGCGGCCGCCTATCAAAAGTCTTAATGAGTT-3'), C6B1 (SEQ ID NO: 74) (5'-GAATTCCTCGAGCTGCAGCCCGGGTTTTTATAGCTAATTAGTCATTTTTTCGTAAGT AAGTATTT TTATTTAA-3'), C6C1 (SEQ ID NO: 75) (5'-CCCGGGCTGCAGCTCGAGGAATTCTTTTTATTGATTAACTAGTCAAATGAGTATATA TAATTGAA AAAGTAA-3') and C6D1 (SEQ ID NO: 76) (5'-GATGATGGTACCTTCATAAATACAAGTTTGATTAAACTTAAGTTG-3') were used to construct pC6L. Oligonucleotide primers C6A1 (SEQ ID NO: 73) and C6B1 (SEQ ID NO: 74) were used in a PCR with canarypox DNA template to generate a 380 base pair fragment. A second PCR reaction with the canarypox DNA template, and oligonucleotide primers C6C1 (SEQ ID NO: 75) and C6D1 (SEQ ID NO: 76), generated a 1,155 base pair fragment. The two PCR reaction products were pooled and primed for a final PCR with C6A1 (SEQ ID NO: 73) and C6D1 (SEQ ID NO: 76), yielding a 1,613 base pair fragment. The final PCR product was digested with SacI and KpnI, and inserted between the SacI and KpnI sites of pBSSK+. The resultant C6 insertion plasmid was designated as pC6L.

### 7. Generation of pMM103

pC5LCDVHAF1 was digested with BamHI and treated with the Klenow fragment from the *E*. *coli* DNA polymerase, in the presence of 20 µM dNTPs to blunt end the BamHI site, followed by digestion with SmaI. The 4.2 kbp blunt ended BamHI to SmaI fragment, containing the H6 promoted CDV F and H6 promoted CDV HA ORFs, was inserted into the SmaI site of pC6L, generating plasmid pMM103.

### 8. Generation of pLF108

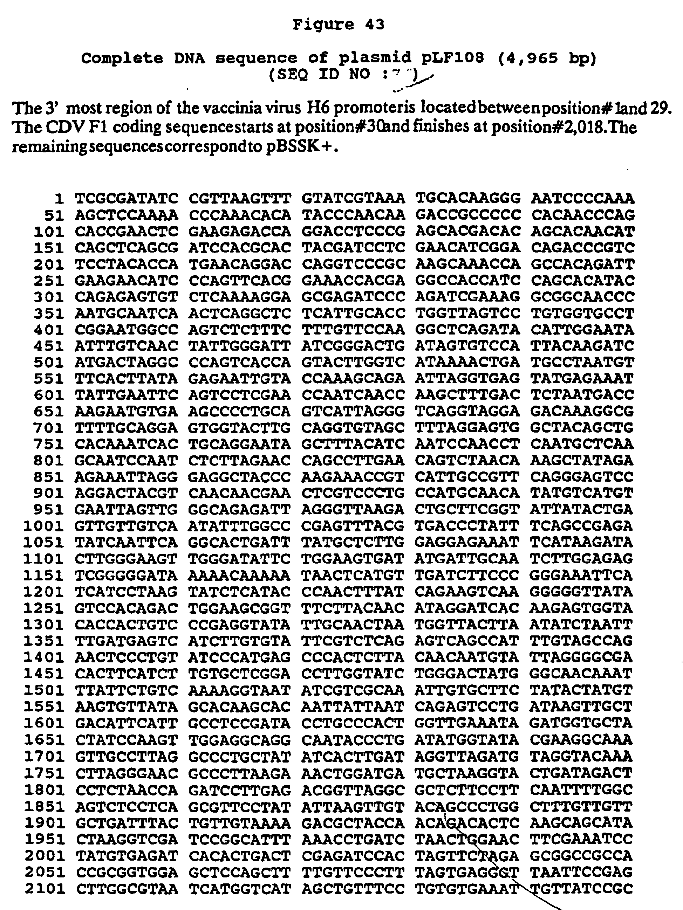

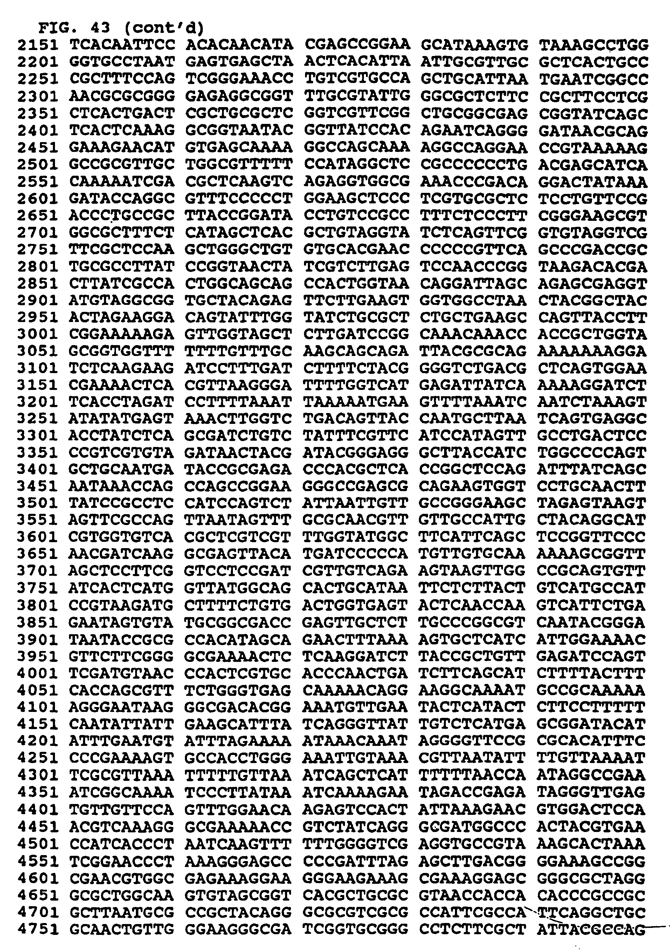

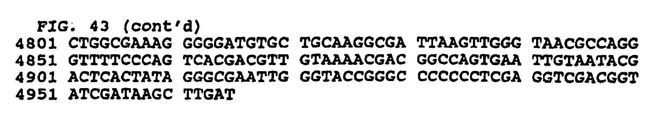

The pMM103 HindIII/BamHI 1,961 bp DNA fragment which contains the CDV F1 coding sequence and the 3' most region of the vaccinia virus H6 promoter, was gel purified and subsequently inserted between the corresponding restrictions sites of pBSSK+, generating pLF108 (Figures 43, 44, SEQ ID NO: 22).

### EXAMPLE 25 - Generation of pLF111, which contains a complete CDV F1 expression cassette

A pLF108 XbaI restriction site was engineered immediately upstream of the CDV F1 initiation codon (ATG) in the following manner. A 473 bp DNA fragment was amplified by PCR using pLF108 DNA as a template and LF448A (5' ACTGTACTCGAGTCTAGAATGCACAAGGGAATCCCCAAAAGC 3') (SEQ ID NO: 77) and RW830 (5' ATTCCAATGTATCTGAGC 3') (SEQ ID NO: 78) as primers. The PCR amplified fragment was isolated using the Gene Clean procedure as previously described before being digested by XhoI and CelII. The resultant XhoI/CelII 136 bp DNA fragment was subsequently ligated with the 4,783 bp XhoI/CelII fragment of pLF108, generating pLF109.

The XbaI (#2,035) was deleted and a KspI restriction site was engineered immediately downstream of pLF108 CDV F1 stop codon (TGA#2,016) in the following manner. A 431 bp DNA fragment was amplified by PCR using pLF109 as a template and LF449 (5'ACTGTACCGCGGTCAGTGTGATCTCACATAGGATTTCGA 3') (SEQ ID NO: 79) and CDV-FG (5' GGTTGAAATAGATGGTG 3') (SEQ ID NO: 80) as the primers. The PCR amplified fragment was isolated using the Gene Clean procedure as previously described before being digested with KspI and BfrI. The resultant 255 bp DNA fragment was gel purified and subsequently ligated with the 4,631 bp KspI/BfrI fragment of pLF109, generating pLF110.

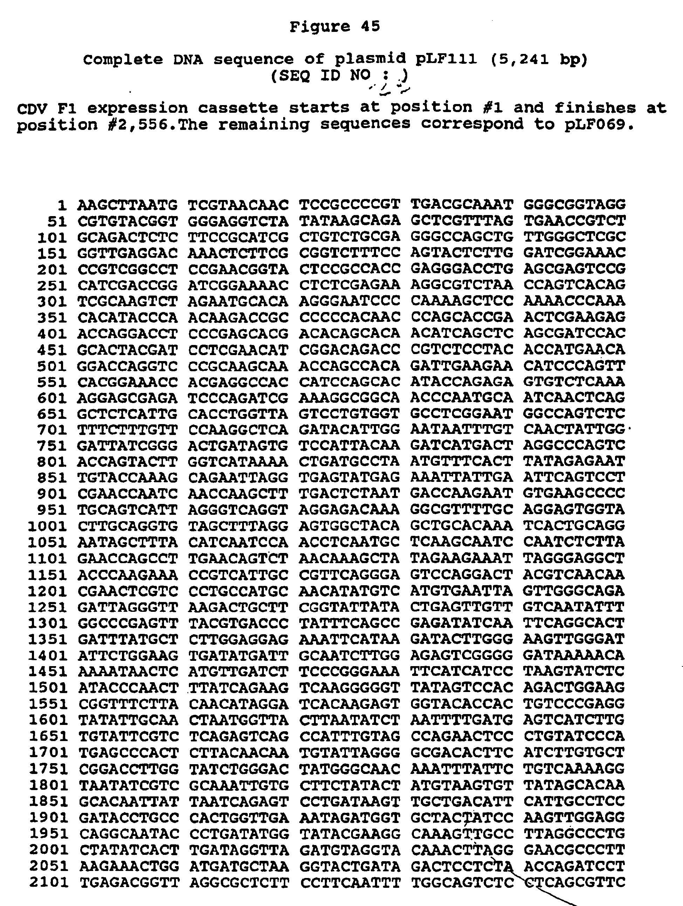

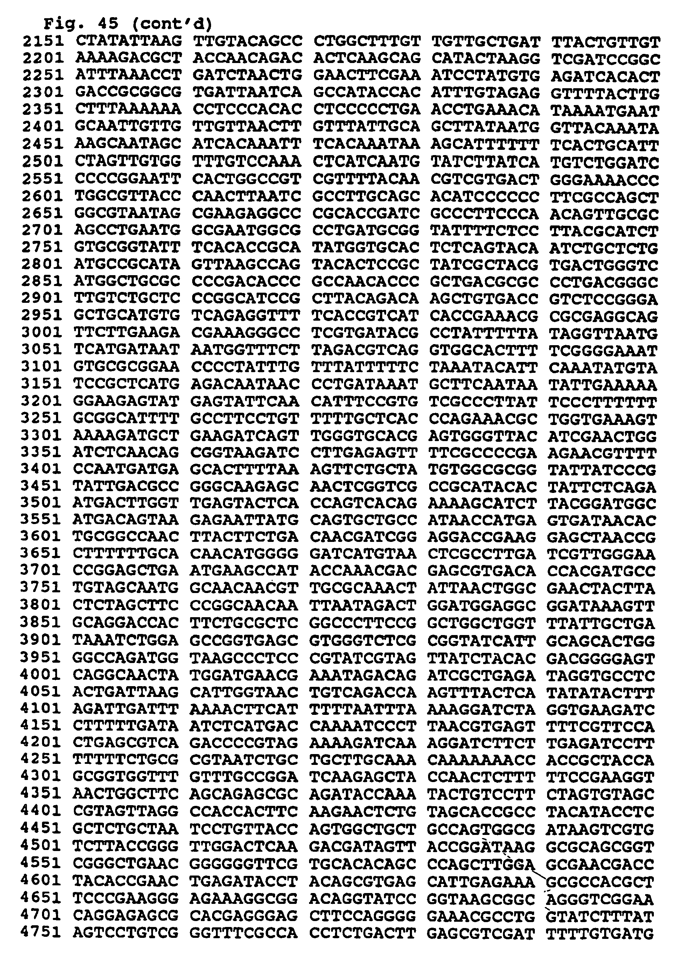

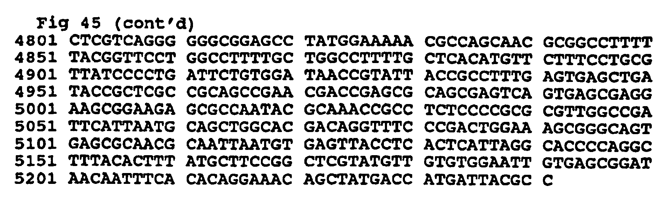

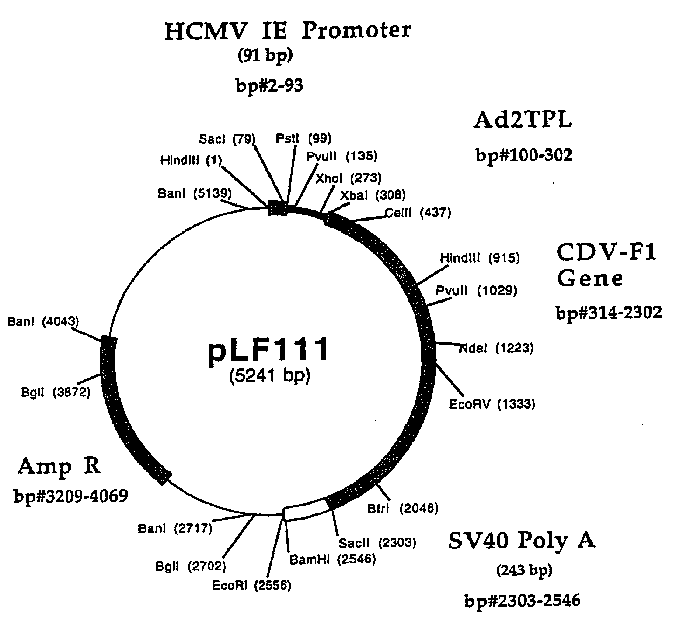

The 1,997 bp pLF110 KspI/XbaI fragment which contains the CDV F1 coding sequence was subsequently ligated with the 3,244 bp KsoI/XbaI fragment of pLF069, generating pLF111 (Figures 45, 46, SEQ ID NO: 23).

### EXAMPLE 26 - Generation of pLF128, which contains a modified complete CDV F1 expression cassette

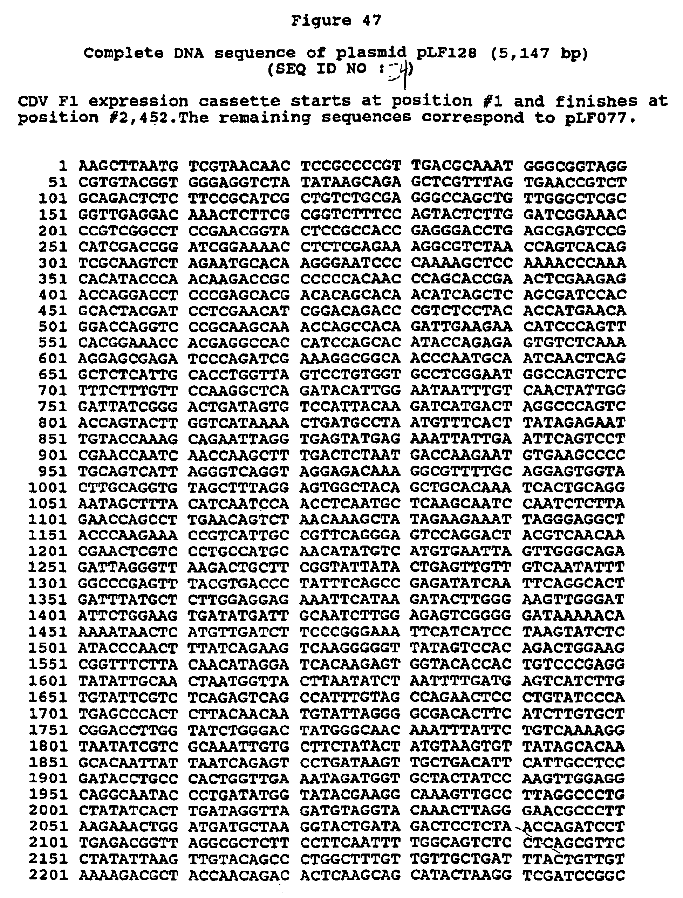

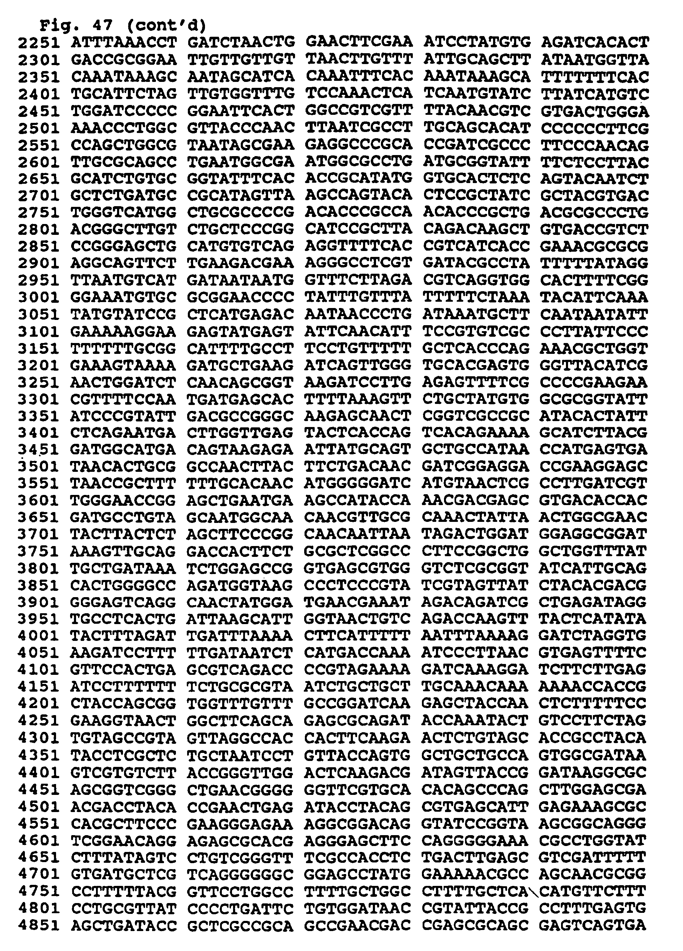

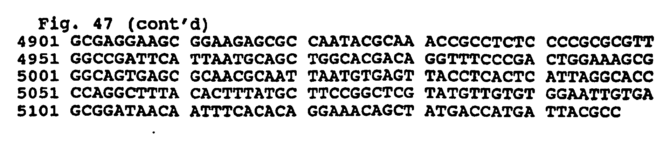

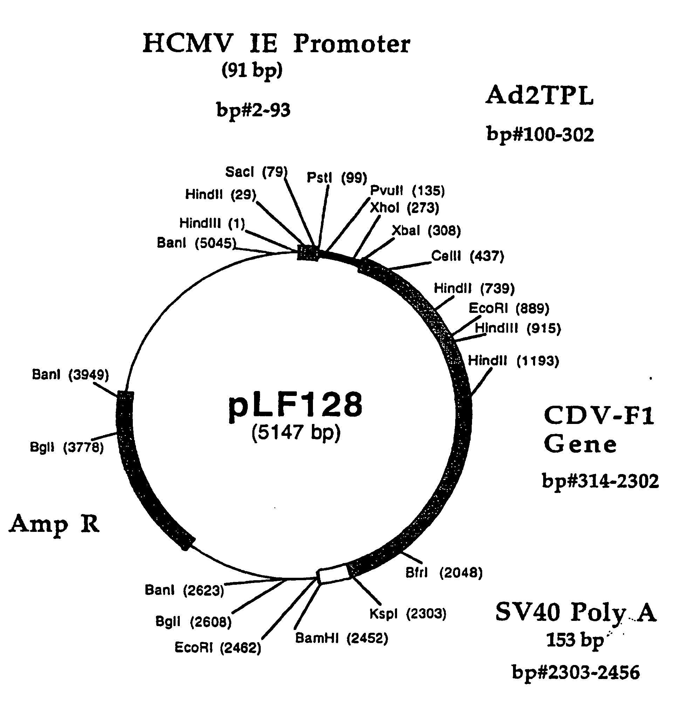

In order to reduce the size of the polyadenylation cassette in the CDV F1 expression cassette from 241 bp to 153 bp, the following manipulations were performed. The pLF077 KspI/BamHI 146 bp fragment was gel purified as previously described and subsequently ligated with the pLF111 KspI/RamHI 5,002 bp fragment in order to generate pLF128 (Figures 47, 48, SEQ ID NO: 24).

### EXAMPLE 27 -Generation of pLF130A, a donor plasmid for insertion of CDV F1 expression cassette 12 bp upstream of SmaI site at CAV2 genome right end

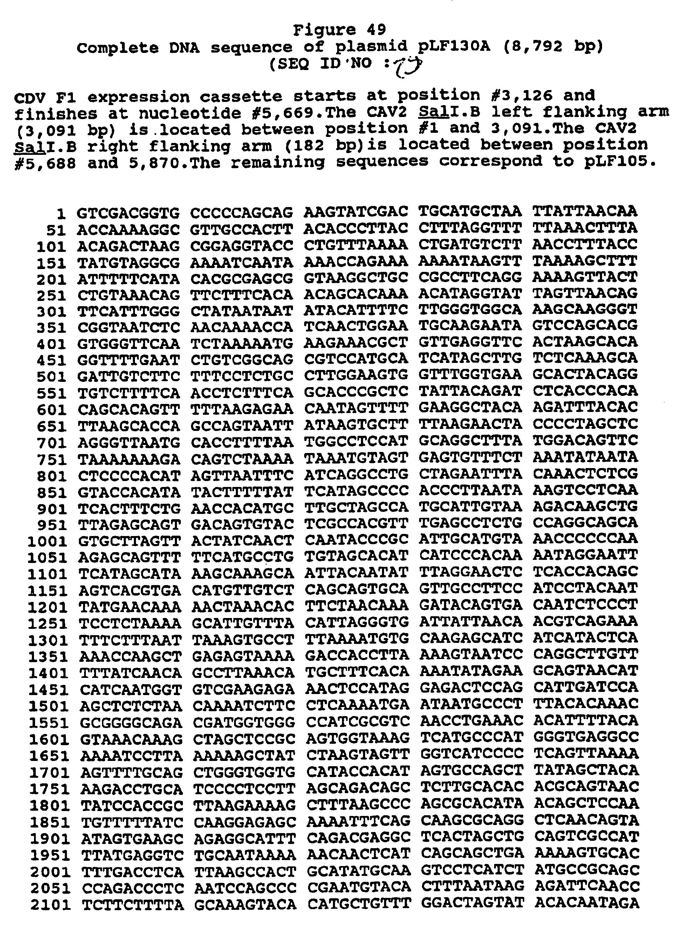

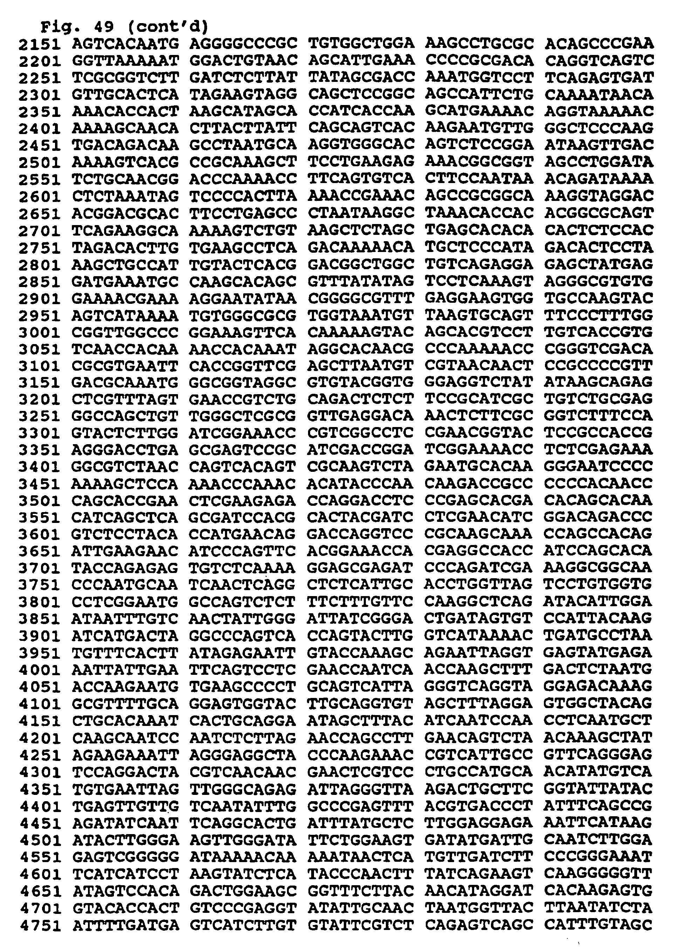

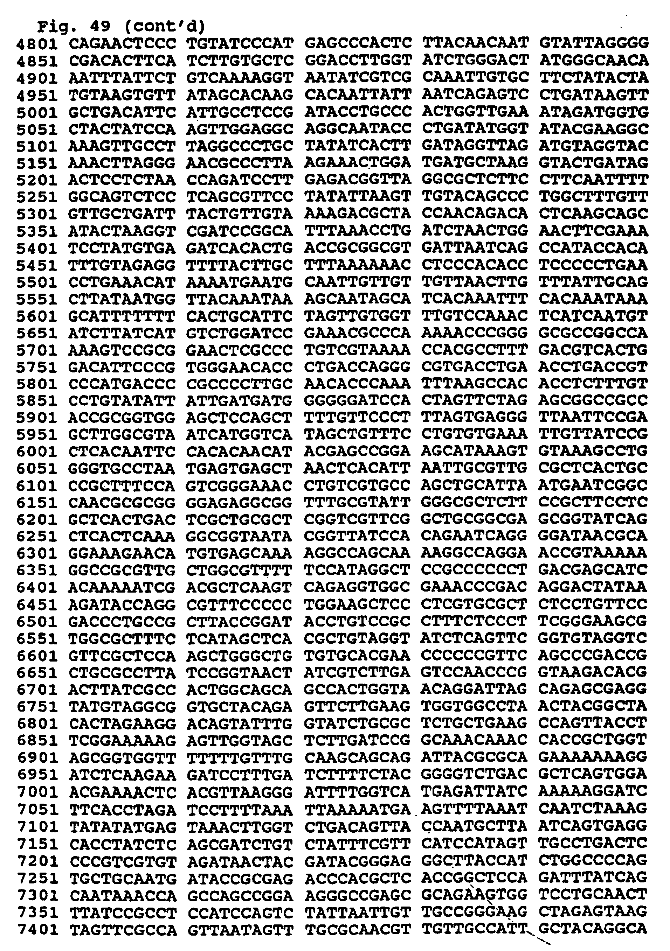

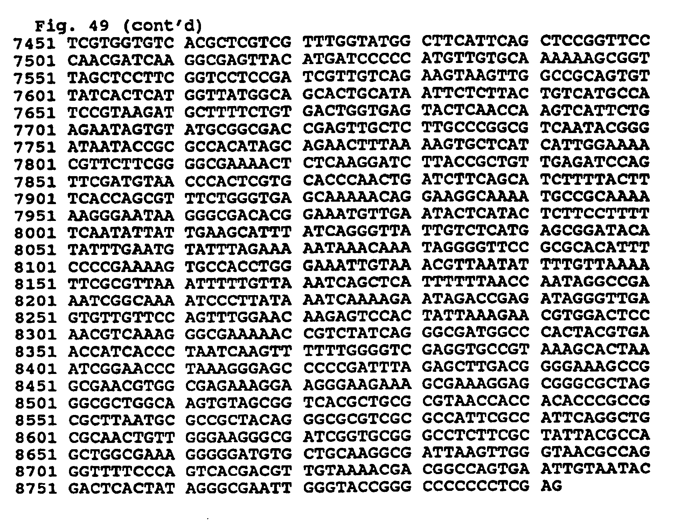

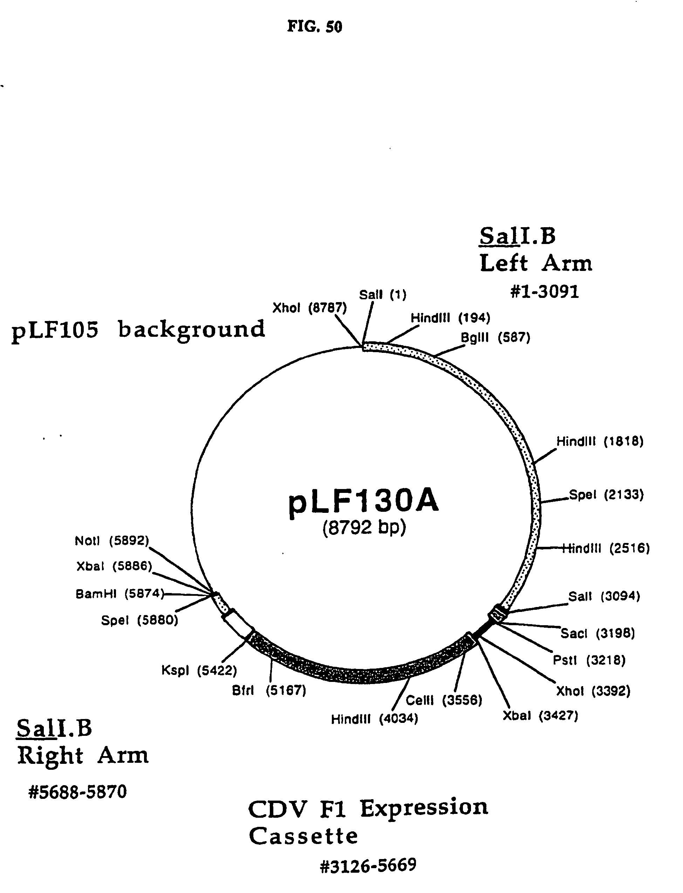

Plasmid pLF128 was digested by BamHI and subsequently partially digested by HindIII. The BamHI/HindIII 2,451 bp fragment contains the CDV F1 coding sequence coupled to the regulatory sequences in pLF077, and was treated with the Klenow fragment from the *E. coli* DNA polymerase before being ligated with the 6,243 bp NruI linearized pLF105, generating pLF130A and pLF130B. pLF130A corresponds to the rightward orientation of the expression cassette (Figures 49, 50, SEQ ID NO: 25).

### EXAMPLE 28 - Generation of vCA-CDVF1-@12bp-up-SmaI

Ten µg of pLF130A were digested with BglII and NotI and the resulting 5,305 bp DNA fragment was isolated using the Gene Clean procedure as previously described and resuspended in H₂O to a concentration of 100 ng/µl. MDCK cells were transfected using the Lipofectamine based procedure as previously described. Solution A was prepared by mixing 0.5 µg of 5.3 kbp BglII/NotI DNA fragment with 3 µg of purified vCA2 DNA. Solution A total volume was brought to 300µl with supplemented serum free MEM medium. Transfected cells were harvested after 8 days and plated out on 150 mm diameter tissue culture dishes as previously described. The 1.4 kbp EcoRI/BamHI DNA fragment of pATICDVF1 was labelled by random priming using the procedure previously described and subsequently hybridized with a nitrocellulose membrane to lift viral plaques, as previously described. Two viral plaques cross-reacting with the probe were picked and are subjected to a plaque purification process, as previously described to yield vCA-CDVF1-@12bp-up-SmaI. This virus is characterized by restriction digestion (DNA analysis) and Southern Blot radioimmunoprecipitation (expression analysis).

### EXAMPLE 29 - Additional Recombinants

Since the tag and other exogenous DNA had been incorporated into CAV2, other exogenous DNA can be incorporated into CAV2. Therefore, instead of the exogenous DNA used to generate vCA1, vCA2, vCA3, vCA4, vCA5, vCA6, vCA7, vCA8, and vCA-CDVF1-@12bp-up-SmaI, exogenous DNA as described in U.S. Patent Nos. 5,174,993 and 5,505,941 (e.g., recombinant avipox virus, vaccinia virus; rabies glycoprotein (G), gene, turkey influenza hemagglutinin gene, gp51,30 envelope gene of bovine leukemia virus, Newcastle Disease Virus (NDV) antigen, FelV envelope gene, RAV-1 env gene, NP (nudeoprotein gene of Chicken/Pennsylvania/1/83 influenza virus), matrix and preplomer gene of infectious bronchitis virus; HSV gD; entomopox promoter, *inter alia*), U.S. Patent No. 5,338,683, e.g., recombinant vaccinia virus, avipox virus; DNA encoding Herpesvirus glycoproteins, inter alia; U.S. Patent No. 5,494,807 (e.g., recombinant vaccinia, avipox; exogenous DNA encoding antigens from rabies, Hepatitis B, JEV, YF, Dengue, measles, pseudorabies, Epstein-Barr, HSV, HIV, SIV, EHV, BHV, HCMV, canine parvovirus, equine influenza, FeLV, FHV, Hantaan, C. tetani, avian influenza, mumps, NDV, inter alia); U.S. Patent No. 5,503,834 (e.g., recombinant vaccinia, avipox, Morbillivirus [e.g., measles F, hemagglutinin, inter alia]); U.S. Patent No. 4,722,848 (e.g., recombinant vaccinia virus; HSV tk, glycoproteins [e.g., gB, gD], influenza HA, Hepatitis B [e.g., HBsAg], inter alia); U.K. Patent GB 2 269 820 B and U.S. Patent No. 5,514,375 (recombinant poxvirus; flavivirus structural proteins); WO 92/22641 (e.g., recombinant poxvirus; immunodeficiency virus, *inter alia*); WO 93/03145 (e.g., recombinant poxvirus; IBDV, inter alia); WO 94/16716 and U.S. application Serial No. 08/184,009, filed January 19, 1994 (e.g., recombinant poxvirus; cytokine and/or tumor associated antigens, *inter alia*); PCT/US94/06652 (*Plasmodium* antigens such as from each stage of the *Plasmodium* life cycle); U.S. Patent No. 5,523,089, WO93/08306, PCT/US92/08697, Molecular Microbiology (1989), 3(4), 479-486, PCT publications WO 93/04175, and WO 96/06165 (*Borrelia* antigens and DNA therefor); and Briles et al. WO 92/14488 (pneumococcal DNA), are used to generate additional CAV2 recombinants with the exogenous DNA in regions as in vCA2 through vCA8 and vCA-CDVF1-@12bp-up-SmaI and deletions as in vCA2 through vCA8 and vCA-CDVF1-@12bp-up-SmaI (e.g., insertions in the E3 or at the region between the right ITR and the E4 transcription unit or at both sites and deletions in the E3 region) including recombinants containing coding for multiple antigens, as herein described (including with subfragment promoters, reduced or modified polyadenylation cassettes, and promoters with 5' UTR replaced). Analysis demonstrates expression. Compositions are prepared by admixture with a carrier or diluent for administration to a vertebrate (animal or human) hosts for generating responses, including antibody responses.

**Table 1. Sizes of CAV2 DNA restriction fragments.**

| | | | | | | | | | | | |
|---|---|---|---|---|---|---|---|---|---|---|---|
| CAV2 DNA restriction fragments sizes | | | | | | | | | | | |

| **Fragment #** | **A** | **B** | **C** | **D** | **E** | **F** | **G** | **H** | **I** | **J** | **K** |
|---|---|---|---|---|---|---|---|---|---|---|---|
| **BamHI** | | | | | | | | | | | |
| | 14 | 8.1 | 6.1 | 2.1 | 0.8 | 0.7 | | | | | |
| **EcoRI** | | | | | | | | | | | |
| | 20 | 8.2 | 3.8 | | | | | | | | |
| **Asp718** | | | | | | | | | | | |
| | 9.5 | 4.8 | 3.8 | 3.2 | 3.2 | 3 | 2.5 | 0.85 | 0.75 | | |
| **SalI** | | | | | | | | | | | |
| | 29 | 3.2 | | | | | | | | | |
| **BglII** | | | | | | | | | | | |
| | 29 | 2.8 | | | | | | | | | |
| **BglI** | | | | | | | | | | | |
| | 6.1 | 5 | 4.1 | 3.2 | 2 | 1.7 | 1.5 | 1.5 | 1 | 0.7 | ND |

**Table 2.**

| Characteristics of CAV2 E3 region ORFs | | | |
|---|---|---|---|
| | **ORF1** | **ORF2** | **ORF3** |
| **MW (KDa.)** | | | |
| | 12.6 | 40.7 | 18.6 |
| **pI** | | | |
| | 6.48 | 7.45 | 9.68 |
| **Limits in** **Fig 3** | | | |
| | 8-346 | 384-1478 | 1019-483 |

### REFERENCES

ABLETT, R.E. and L.A. BAKER. 1960. Veterinary Record, 72, 1202.
APPEL, M. , S.I. BISTNER, M. MENEGUS, D.A. ALBERT and L.E. CARMICHAEL. 1973. Pathogenicity of low-virulence strains of two canine adenovirus types. Am. J. Vet. Res., 34, 543-550.
APPEL, M.J.G. and D.H. PERCY. 1970. SV-5-like parainfluenza virus in dogs. J.A.V.M.A., 156, 1778-1781.
APPEL, M.J.G., PICKERILL, R.G., M. MENEGUS, D.H. PERCY, D.H. PARSONSON and B.E. SHEFFY. 1970. 20th Ganes Veterinary Symposium, Manhattan, USA, pp 15-23.
ASSAF, R., C. MONPETIT, G. MARSOLAIS, M. AMINZADEHM, L. LAMONTAGNE and P. MAROIS. 1978. MV Quebec, 8, 10-12.
BASS, E.P., M.A. GILL and W.H. BECKENHAUER. 1980. Evaluation of canine adenovirus type 2 as a replacement for infectious canine hepatatis vaccine. J. Am. Vet. Med. Assoc., 177, 234-242.
BETT, A.J., L. PREVEC and F.L. GRAHAM. 1993. Packaging capacity and stability of human adenovirus type 5 vectors. J. Virol., 67, 5911-5921.
BINN, L.N., EDDY, G.A., LAZAR, E.C., HELMS, J. and T. MURNANE. 1967. Viruses recovered from laboratory dogs with respiratory disease. Proceedings of the Society of Experimental Biology and Medicine, 126, 140-145.
BOSHART, M., F. WEBER, G. JAHN, K. DORSH-HÄSLER, B. FLECKENSTEIN and W. SCHAFFNER. 1985. A very strong enhancer is located upstream of an immediate early gene of human cytomegalovirus. Cell, 41, 521-530.
BOTH, G.W. , L.J. LOCKETT, V. JANARDHANA, S.J. EDWARDS, A.R. BELLAMY, F.L. GRAHAM, L. PREVEC and M.E. ANDREW. 1993. Protective immunuty to rotavirus-induced diarrhoea is passively transferred to newborn mice from naive dams vaccinated with single dose of a recombinant adenovirus expressing rotavirus VP7sc. Virology, 193, 940-950.
BREKER-KLASSEN, M., YOO, D., MITTAL, S.K., SORDEN, S.D., HAINES, D.M. and L.A. BABIUK. 1995. Recombinant type 5 adenovirus expressing bovine parainfluenza virus type 3 glycoproteins protect sigmodon hispidus cotton rat from bovine parainfluenza virus type 3 infection. J. Virol., 69, 4308-4315.
CABASSO, V.J., M.R. STEBBINS, T.W. NORTON and H.R. COX. 1954. Propagation of infectious canine hepatatis virus in tissue culture. Proceedings of the Society of Experimental Biology and Medicine, 85, 239-245.
CAVANAGH, H.M.A., C.F. GALLAGHER and N. SPIBEY. 1991. A mutant of canine adenovirus type 2 with a duplication of the E1a region exhibits altered expression of early region 4. J. Gen. Virol., 72, 2121-2127.
CHANDA, P.K., NATUK, R.J., B.B. MASON, B.M. BHAT, L. GREENBERG, S.K. DHEER, K.L. MOLNAR-KIMBER, S. MIZUTANI, M.D. LUBECK, A.R. DAVIS and P.P. HUNG. 1990. High level expression of the envelope glycoprotein of the human immunodeficiency virus type I in presence of rev gene using helper-independent adenovirus type 7 recombinants. Virology, 175, 535-547.
CHENGALVALA, M., M.D. LUBECK, A.R. DAVIS, S. MIZUTANI, K. MOLNAR-KIMBER, J. MORIN, and P.P. HUNG. 1991. Evaluation of adenovirus type 4 and type 7 recombinant hepatatis B vaccines in dogs. Vaccine, 9, 485-490.
CHENGALVALA, M.V. , B.M. BHAT, R. BHAT, M.D. LUBECK, S. MIZUTANI, A.R. DAVIS and P.P. HUNG. 1994. Immunogenicity of high expression adenovirus-hepatatis B virus recombinant vaccines in dogs. J. Gen. Virol., 75, 125-131.
DANSKIN, D. 1973. Isolation of canine adenovirus A26/61 (Toronto) using canine kidney (MDCK) cell line. The Veterinary Record, 126-127.
DARTEIL, R., BUBLOT, M., LAPLACE, E., J.-F. BOUQUET, J.-C. AUDONNET and M. RIVIÈRE. 1995. Herpesvirus of turkey recombinant viruses expressing infectious bursal disease virus (IBDV) VP2 immunogen induce protection against an IBDV virulent challenge in chickens. Virology 211, 481-490.
DEWAR, R.L. , V. NATARAJAN, M.B. VASUDEVACHARI and N.P. SALZMAN. 1989. Synthesis and processing of human immunodeficiency virus type 1 envelope proteins encoded by recombinant human adenovirus. J. Virol. , 63, 129-136.
DITCHFIELD, J., L.W. MACPERSON and A. ZBITNEW. 1962. Association of a canine adenovirus (Toronto A26/61) with an outbreak of laryngotracheitis ("kennel chough"). Can. Vet. Jour., 3, 238-247.
DORSCH-HÄSLER, K., G.M. KEIL, F. WEBER, M. JASIN, W. SCHAFFNER and U.H. KOSZINOWSKI. 1985. A long and complex enhancer activates transcription of the gene coding for the highly abundant early mRNA in murine cytomegalovirus. Proc. Natl. Acad. Sci., 82, 8325-8329.
EDBAUER, C., R. WEINBERG, J. TAYLOR, A. REY-SENELONGE, J-F. BOUQUET, P. DESMETTRE and E. PAOLETTI. 1990. Protection of chickens with a recombinant fowlpox virus expressing the newcastle disease virus hemagglutinin-neuraminidase gene. Virology, 179, 901-904.
ELOIT, M. , P. GILARDI-HEBENSTREIT, B. TOMA and M. PERRICAUDET. 1990. Construction of a defective adenovirus vector expressing the pseudorabies virus glycoprotein gp50 and its use as a live vaccine. J. Gen. Virol., 71, 2425-2431.
FAIRCHILD, G.A. and D. COHEN. 1969. Serological study of a canine adenovirus (Toronto A26/61) infection in dogs. Am. J. Vet. Res., 30, 923-928.
GALLICHAN, W.S., D.C. JOHNSON, F.L. GRAHAM and K.L. ROSENTHAL. 1993. Mucosal immunity and protection after intranasal immunization with recombinant adenovirus expressing herpes simplex virus glycoprotein B. J. of Infect. Dis. 168, 622-629.
GARCÍA-SASTRE, A. and P. PALESE. 1995. Influenza virus vectors. Biologicals, 23, 171-178.
GILLARD, S., SPEHNER, D., DRILLIEN, R., AND KIRN, A., Proc. Natl. Acad. Sci. USA 83, 5573-5577 (1986).
GOEBEL, S.J., G.P. JOHNSON, M.E. PERKUS, S.W. DAVIS, J.P. WINSLOW AND E. PAOLETTI, Virology 179, 517-563 (1990b).
GOEBEL, S.J., JOHNSON, G.P., PERKUS, M.E., DAVIS, S.W., WINSLOW, J.P., PAOLETTI, E., Virology 179, 247-266 (1990a).
GINSBERG, H.S. , U. LUNDHOLM-BEAUCHAMP, R.L. HORSWOOD, B. PERNIS, W.S.M. WOLD, R.M. CHANOCK and G.A. PRINCE. 1989. Role of early region 3 (E3) in pathogenesis of adenovirus disease. Proc. Natl. Acad. Sci. USA, 86, 3823-3827.
GIRARD, M., R. ALTMEYER, S. van der WERF, C. WYCHOWSKI and A. MARTIN. 1995. The use of picornaviruses as vectors for the engineering of live recombinant vaccines. Biologicals, 23, 165-169.
GORMAN, C.M., D. GIES, G. McCRAY and M. HUANG. 1989. The human cytomegalovirus major immediate early promoter can be trans-activated by adenovirus early proteins. Virology, 171, 377-385.
GRAHAM, F.L. , L.A. PREVEC, M. SCHEIDER, G. GHOSH-CHOUDHURY, M. McDERMOTT, and D.C. JOHNSON. 1988. Cloning and expression of glycoprotein genes in human adenovirus vectors. In: Technological Advances in Vaccine Development, 243-253.
GRAHAM, F. L., J. SMILEY, W. C. RUSSELL and R. NAIRN. 1977. Characteristics of a human cell line transformed by human adenovirus 5. J. Gen. Virol., 36, 59-72.
GRAND, R. J. A. 1987. The structure and function of the adenovirus early region 1 proteins. Biochem. J. , 241, 25-38.
HADDADA, H., B. KLONJKOWSKI and M. PERRICAUDET. 1994. Adenoviral vectors of animal origin and use in gene therapy. Patent # WO94/26914.
HAJ-AHMAD, Y. and F.L. GRAHAM. 1986. Development of a helper-independent human adenovirus vector and its use in the transfer of herpes simplex virus thymidine kinase gene. J. Virol., 57, 267-274.
HSU, K.-H.L., M.D. LUBECK, B.M. BHAT, R.A. BHAT, B. KOSTEK, B.H. SELLING, S. MIZUTANI, A.R. DAVIS and P.P. HUNG. 1994. Efficacy of adenovirus-vectored syncytial virus vaccines in a new ferret model. Vaccine, 12, 607-612.
IMLER, J-L. 1995. Adenovirus vectors as recombinant viral vaccines. Vaccine, 13, 1143-1151.
IMPERIALE, M., G. AKUSJARVI and K. LEPPARD. 1995. Post-transcriptional control of adenovirus gene expression. Curr. Top. Microbiol. Immunol., 199, 139-171.
JOHNSON, D.C., G. GHOSH-CHOUDHURY, J.R. SMILEY, L. FALLIS and F.L. GRAHAM. 1988. Abundant expression of herpes simplex virus glycoprotein gB using an adenovirus vector. Virology, 164, 1-14.
JOUVENNE, P., M. DION and C. HAMELIN. 1987. Cloning, physical mapping and cross-hybridization of the canine adenovirus types 1 and 2 genomes. Gene, 60, 21-28.
KELLY, T.J., JR. and A.M. LEWIS, JR. 1973. Use of nondefective adenovirus-simian virus 40 hybrids for mapping the simian virus 40 genome. J. Virol., 12, 643-652.
KIT, M., S. KIT, S.P. LITTLE, R.D. DI MARCHI, AND C. GALE. 1991. Bovine herpesvirus-1 (infectious bovine rhinotracheitis virus)-based viral vector which expresses foot-and-mouth disease epitopes. Vaccine, 9, 564-572.
KOPTOPOULOS, G. and H. J. C. CORNWELL. 1981. Veterinary bulletin, 51, 135-142.
LAFEMINA, R.L, M.C. PIZZORNO, J.D. MOSCA and G.S. HAYWARD. 1989. Expression of the acidic nuclear immediate early protein (IE1) of human cytomegalovirus in stable cell lines and its preferential association with metaphase chromosomes. Virology, 172, 584-600.
LINNÉ, T. 1992. Differences in E3 region of the canine adenovirus type 1 and type 2. Virus Research, 23, 119-133.
LUBECK, M.D. , A.R. DAVIS, M. CHENGALVALA, R.J. NATUK, J.E. MORIN, K. MOLNAR-KIMBER, B.B. MASON, B.M. BHAT, S. MIZUTANI, P.P. HUNG and R.H. PURCELL. 1989. Immunogenicity and efficacy testing in chimpanzees of an oral hepatatis B vaccine based on a live recombinant adenovirus. Proc. Natl. Acad. Sci. USA, 86, 6763-6767.
LUBECK, M.D. , R.J. NATUK, M. CHENGALVALA, P.K. CHANDA, K.K. MURTHY, S. MURTHY, S. MIZUTANI, S.-G. LEE, M.S. WADE, B.M. BHAT, R. BHAT, S.K. DHEER, J.W. EICHBERG, A.R. DAVIS and P.P. HUNG. 1994. Immunogenicity of recombinant adenovirus-human immunodeficiency virus vaccines in chimpanzees following intranasal administration. AIDS. Res. Hum. Retr., 10, 1443-1449.
MACARTNEY, L. , H.M.A. CAVANAGH and N. SPIBEY. 1988. Isolationof canine adenovirus-2 from faeces of dogs with enteric disease and its unambigous typing by restriction endonuclease mapping. Research in Veterinary Science, 44, 9-14.
McDERMOTT, M.R., F.L. GRAHAM, T. HANKE and D.C. JOHNSON. 1989. Protectiuon of mice against lethal challenge with herpes simplex by vaccination with an adenovirus vector expressing HSV glycoprotein B. Virology, 169, 244-247.
METTENLEITER, T.C., B.G. KLUPP, F. WEILAND and N. VISSER. 1994. Characterization of a quadruple glycoprotein-deleted pseudorabies virus mutant for use as a biologically safe live virus vaccine. 75, 1723-1733.
MITTAL, S.K., A.J. BETT, L. PREVEC and F.L. GRAHAM. 1995b. Foreign gene expression by human adenovirus type 5-based vectors studied using firefly luciferase and bacterial β-galactosidase genes as reporters. Virology, 210, 226-230.
MITTAL, S.K., L. PREVEC, F.L. GRAHAM and L.A. BABIUK. 1995a. Development of a bovine adenovirus type 3-based expression vector. J. Gen. Virol., 76, 93-102.
MORIN, J.E. , M.D. LUBECK, J.E. BARTON, A.J. CONLEY, A.R. DAVIS and P.P. HUNG. 1987. Recombinant adenovirus induces antibody response to hepatatis B virus surface antigen in hamsters. Proc. Natl. Acad. Sci. USA, 84, 4626-4630.
MUELLER, R.E., R.L. MULDOON and G.G. JACKSON. 1969. Communicability of enteric live adenovirus type 4 vaccine in families. J. Infect. Dis., 119, 60-66.
NATUK, R.J., M.D. LUBECK, P.K. CHANDA, M. CHENGALVALA, M. S. WADE, S.C.S. MURTHY, J. WILHELM, S.K. VERNON, S.K. DHEER, S. MIZUTANI, S.-G. LEE, K.K. MURTHY, J.W. EICHBERG, A.R. DAVIS and P.P. HUNG. 1993. Immunogenicity of recombinant human adenovirus-human immunodeficiency virus vaccines in chimpanzees. AIDS. Res. Hum. Retr., 9, 395-404.
NEVINS, J.R. 1993. Transcriptional activation by the adenovirus E1A prteins. Seminars in Virology, 4, 25-31.
OUALIKENE, W., P. GONIN and M. ELOIT. 1994. Short and long term dissemination of deletion mutants of adenovirus in permissive (cotton rat) and non-permissive (mouse) species. J. Gen. Virol., 75, 2765-2768.
PERKUS, M.E., LIMBACH, K., AND PAOLETTI, E., J. Virol. 63, 3829-3836 (1989).
PERKUS, M.E., J. TARTAGLIA and E. PAOLETTI. 1995. Poxvirus-based vaccine candidates for cancer, AIDS, and other infectious diseases. J. Leuk. Biol., 58, 1-13.
PERKUS, M.E., E.B. KAUFFMAN, J. TAYLOR, S. MERCER, D. SMITH, J. VANDERHOEVEN, and E. PAOLETTI. 1993. Methodology of using vaccinia virus to express foreign genes in tissue culture. J. Tiss. Cult. Meth. 15:72-81.
PERRICAUDET, M. and L.D. STRATFORD-PERRICAUDET. 1995. Adenovirus-mediated in vivo gene therapy. In: Viruses in human gene therapy. Carolina Academic Press, 1-32.
PREVEC, L., M. SCHNEIDER, K.L. ROSENTHAL, L.W. BELBECK, J.B. DERBYSHIRE and F.L. GRAHAM. 1989. Use of human adenovirus-based vectors for antigen expression in animals. J. Gen. Virol., 70, 429-434.
RAGOT, T. , S. FINERTY, P.E. WATKINS, M. PERRICAUDET and A.J. MORGAN. 1993. Replication-defective recombinant adenovirus expressing the Epstein-Barr virus (EBV) envelope glycoprotein gp340/220 induces protective immunity against EBV-induced lymphomas in cottontop tamarin. J. Gen. Virol., 74, 501-507.
RANDRIANARISON-JEWTOUKOFF, V. and M. PERRICAUDET. 1995. Recombinant adenovirus as vaccines. Biologicals, 23, 145-157.
ROBINSON, A.J., H.B. YOUNDHUSBAND and A.J.D. BELLETT. 1973. A circular DNA-protein complex from adenoviruses. Virology, 56, 54-69.
ROSS, L.J.N., M.M. BINNS, P. TYERS, J. PASTOREK, V. ZELNIK and S. SCOTT. 1993. Construction and properties of a turkey herpesvirus recombinant expressing the Marek's disease virus homolgue of glycoprotein B of herpes simplex virus. J. Gen. Virol. 74, 371-377.
SAITO, I. , Y. OYA, K. YAMAMOTO, T. YUASA and H. SHIMOJO. 1985. Construction of nondefective adenovirus type 5 bearing a 2.8 kilobase hepatatis B virus DNA near the right end of its genome. J. Virol. , 54, 711-719.
SCHWARTZ, A.R., Y. TOGO and R.B. HORNICK. 1974. Clinical evaluation of live types 1, 2 and 5 adenovirus vaccines. Am. Rev. Resp. Dis., 109, 233
SEDEGAH, M., C.H. CHIANG, W.R. WEISS, S. MELLOUK, M.D. COCHRAN, R.A. HOUGHTEN, the late R.L. BEUDOIN, D. SMITH, and S.L. HOFFMAN. 1992. recombinant pseudorabies virus carrying a plasmodium gene: herpesvirus as a new live viral vector for inducing T- and B-cell immunity. Vaccine, 10, 578-584
SHARP, P. 1984. Adenovirus transcription. In: The adenovirus, Ed. H. S. GINSBERG, Plenun Press, New-York and London. Pp. 173-204.
SPIBEY, N. and H.M.A. CAVANAGH. 1989. Molecular cloning and restriction endonuclease mapping of two strains of canine adenovirus type 2. J. Gen. Virol., 70, 165-172.
SUMMER, J.W. , J.H. SHADDOCK, G.-J. WU and G.M. BAER. 1988. Oral administration of an attenuated strain of canine adenovirus (type 2) to raccoons, foxes, shunk and mongoose. Am. J. Vet. Res., 49, 169-171.
SWANGO, L.J., W.L. WOODING and L.N. BINN. 1970. A comparison of the pathogenesis of infectious canine hepatatis virus and the A26/61 virus strain (Toronto). J.A.V.M.A., 156, 1687-1696.
TAYLOR, J., C. TRIMARCHI, R. WEINBERG, B. LANGUET, F. GUILLEMIN, P. DESMETTRE and E. PAOLETTI. 1991. Efficacy studies on a canarypox-rabies recombinant virus. Vaccine, 9, 190-193.
THUMMEL, C., R. TJIAN, S.-L. HU, and T. GRODZICKER. 1983. Translational control of SV40 T antigen expressed from the adenovirus late promoter. Cell, 33, 455-464.
TOP, JR, F.H., R.A. GROSSMAN, P.J. BARTELLONI, H.E. SEGAL, B.A. DUDDING, P.K. RUSSELL and E.L. BUESCHER. 1971b. Immunization with live types 7 and 4 vaccines. I. Safety, infectivity, antigenicity and potency of adenovirus type 7 vaccine in humans. J. Inf. Dis., 124, 148-154.
TOP, JR., F.H. , E.L. BUESCHER, W.H. BANCROFT and P K. RUSSELL. 1971a. Immunization with live types 7 and 4 vaccines. II. Antibody response and protective effect against accutate respiratory disease due to adenovirus type 7. J. Inf. Dis., 124, 155-160.
WESSELING, J.G. , G.-J. GODEKE, V.E.C.J. SCHIJNS, L. PREVEC, F.L. GRAHAM, M.C. HORZINEK and P.J.M. ROTTIER. 1993. Mouse hepatatis virus spike and nucleocapsid proteins expressed by adenovirus vectors protect mice against a lethal infection. J. Virol., 74, 2061-2069.
WOLD, W.S.M. and L.R. GOODING. 1991. Minireview: Region E3 of adenovirus: A cassette of genes involved in host immunosurveillance and virus-cell interactions. Virology, 184, 1-8.
XU, Z.Z., V. KROUGLIAK, L. PREVEC, F.L. GRAHAM and G.W. BOTH. 1995. Investigation of promoter function in human and animal cells infected with human recombinant adenoviruses expressing rotavirus antigen VP7sc. J. Gen. Virol., 76, 1971-1980. ZHANG, Y. and R.J. SCHNEIDER. 1993. Adenovirus inhibition of cellular protein synthesis and the specific translation of late viral inRNAs. Seminars in Virology, 4, 229-236.

## Claims

1. A canine adenovirus 2 (CAV2) synthetically modified to contain therein exogenous DNA, wherein the E3 region or a portion thereof has been deleted from the CAV2, and wherein exogenous DNA is present in a region located between the right ITR and the E4 region, or wherein the exogenous DNA is inserted into both the CAV2 E3 region and a region located between the right ITR and the E4 transcription unit and which is packaged as an infectious CAV2 with respect to cells in which CAV2 naturally replicates.

2. A vector for expression of heterologous DNA comprising the CAV2 of claim 1.

3. A vector for cloning heterologous DNA comprising the CAV2 of claim 1.

4. The vector of claim 2 wherein the heterologous DNA encodes an expression product comprising: an epitope of interest, a biological response modulator, a growth factor, a recognition sequence, a therapeutic gene, or a fusion protein.

5. The vector of claim 4 wherein the heterologous DNA encodes an epitope of interest.

6. The vector of claim 5 wherein the epitope of interest comprises an epitope of interest from an antigen of a veterinary pathogen or toxin.

7. The vector of claim 6 wherein the epitope of interest comprises: a Morbillivirus antigen; a rabies glycoprotein; an avian influenza antigen; a bovine leukaemia virus antigen, a Newcastle Disease Virus (NDV) antigen; FeLV envelope protein; RAV-1 env; matrix and/or preplomer of infectious bronchitis virus; a Herpesvirus glycoprotein; a flavivirus antigen, an immunodeficiency virus , antigen; a parvovirus antigen, an equine influenza antigen; a Marek's Disease virus antigen; a poxvirus antigen; or an infectious bursal disease virus antigen.

8. The vector of claim 7 wherein the Morbillivirus antigen comprises canine distemper virus HA or F.

9. The vector of claim 5 wherein the epitope of interest comprises an epitope of interest from an antigen of a human pathogen or toxin.

10. The vector of claim 9 wherein the epitope of interest comprises: a Morbillivirus antigen; a rabies glycoprotein; an influenza antigen; a Herpesvirus antigen; a flavivirus antigen; a Hepatitis virus antigen; an immunodeficiency virus antigen; a Hantaan virus antigen; a *C. tetani* antigen; a mumps antigen, a pneumococcal antigen; a *Borrelia* antigen; a *Plasmodium* antigen; or a chicken pox antigen.

11. The vector of claim 4 wherein the heterologous DNA encodes a growth factor or a therapeutic gene.

12. The vector of claim 11 wherein the growth factor or therapeutic gene encodes a disease-fighting protein, a molecule for treating cancer, a tumor suppressor, a cytokine, a tumor associated antigen, or interferon.

13. The vector of claim 11 wherein the growth factor or therapeutic gene is selected from the group consisting of a gene encoding alpha-globin, beta-globin, gamma-globin, granulocyte macrophage-colony stimulating factor, tumor necrosis factor, an interleukin, macrophage colony stimulating factor, granulocyte colony stimulating factor, erythropoietin, mast cell growth factor, tumor suppressor p53, retinoblastoma, interferon, melanoma associated antigen or B7.

14. An immunogenic, immunological or vaccine composition containing the virus of claim 1, and a pharmaceutically acceptable carrier or diluent.

15. An immunogenic, immunological or vaccine composition containing the vector of any one of claims 2 and 4 to 10 and a pharmaceutically acceptable carrier or diluent.

16. A therapeutic composition containing the vector of any one of claims 11, 12 and 13 and a pharmaceutically acceptable carrier or diluent.

17. A method of expressing a protein or gene product or an expression product which comprises infecting or transfecting a cell *in vitro* with a virus as claimed in claim 1.

18. A method of expressing a protein or gene product or an expression product which comprises infecting or transfecting a cell *in vitro* with a vector as claimed in any one of claims 2, 4, 5, 6 and 9.

19. A method according to claim 17 or 18 wherein said method further comprises extracting, purifying or isolating the protein, gene product or expression product from the cell.

20. A method of cloning a heterologous DNA sequence comprising infecting or transfecting a cell *in vitro* with a virus as claimed in claim 1.

21. A method for cloning a heterologous DNA sequence comprising infecting or transfecting a cell *in vitro* with a vector as claimed in claim 3.

22. A method according to claim 20 or 21 wherein said method further comprises extracting, purifying or isolating the DNA from the cell or progeny virus.

23. An *in vitro* method for preparing the virus of claim 1 comprising inserting the exogenous DNA into a non-essential region of the CAV2 genome.

24. The method of claim 23 comprising *in vivo* recombination which comprises transfecting a cell with CAV2 or CAV2 DNA in a cell compatible medium in the presence of donor DNA comprising the exogenous DNA flanked by DNA sequences homologous with portions of the CAV2 genome, whereby the exogenous DNA is introduced into the genome of the CAV2.

25. The method of claim 24 wherein the CAV2 modified by the *in vivo* recombination is recovered.

26. The method of claim 24 or 25 which comprises cleaving CAV2 DNA to obtain cleaved CAV2 DNA, ligating the exogenous DNA to the cleaved CAV2 DNA to obtain hybrid CAV2-exogenous DNA, transfecting a cell with the hybrid CAV2-exogenous DNA, and then recovering CAV2 modified by the presence of the exogenous DNA.

27. The virus of claim 1 wherein the exogenous DNA includes a promoter.

28. The virus of claim 27, wherein the promoter is from a herpesvirus.

29. The virus of claim 28 wherein the promoter is a CMV promoter.

30. The virus of claim 27 wherein the promoter is a truncated transcriptionally active promoter which comprises a region transactivated with a transactivating protein provided by the virus and the minimal promoter region of the promoter of a full-length promoter from which the truncated transcriptionally active promoter is derived.

31. The virus of claim 30 wherein the promoter is from a herpesvirus.

32. The virus of claim 31 wherein the promoter is from CMV.

33. The virus of claim 31 wherein the promoter is from the murine CMV-IE promoter.

34. The virus of claim 32 wherein the promoter is from HCMV-IE promoter.

35. The virus of claim 33 wherein the promoter is truncated so that there is up to a 40% reduction in size, from a full-length murine CMV-IE promoter, based upon base pairs.

36. The virus of claim 34 wherein the promoter is truncated so that there is up to a 90% reduction in size, from a full-length HCMV-IE promoter, based upon base pairs.

37. An immunogenic, immunological or vaccine composition according to claim 14 or 15 for use in vaccination.

## Patentansprüche

1. Canines Adenovirus 2 (CAV2), welches synthetisch so modifiziert ist, dass es exogene DNA enthält, wobei der E3-Bereich oder ein Teil davon aus CAV2 deletiert ist, und wobei die exogene DNA in einem Bereich zwischen dem rechten ITR und dem E4-Bereich lokalisiert ist, oder wobei die exogene DNA in den E3-Bereich von CAV2 und einen Bereich zwischen dem rechten ITR und der E4-Transkriptionseinheit eingefügt ist, und welches als CAV2 verpackt infektiös für Zellen ist, in welchen CAV2 natürlicherweise repliziert.

2. Vektor zur Expression heterologer DNA, umfassend CAV2 nach Anspruch 1.

3. Vektor zum Clonieren heterologer DNA, umfassend CAV2 nach Anspruch 1.

4. Vektor nach Anspruch 2, wobei die heterologe DNA ein Expressionsprodukt codiert, umfassend: ein Epitop von Interesse, einen biologischen Response-Modulator, einen Wachstumsfaktor, eine Erkennungssequenz, ein therapeutisches Gen oder ein Fusionsprotein.

5. Vektor nach Anspruch 4, wobei die heterologe DNA ein Epitop von Interesse codiert.

6. Vektor nach Anspruch 5, wobei das Epitop von Interesse ein Epitop von Interesse eines Antigens umfasst, das von einem tiermedizinisch relevanten Pathogen oder Toxin stammt.

7. Vektor nach Anspruch 6, wobei das Epitop von Interesse folgende Antigene umfasst: ein Morbillivirus-Antigen; ein Tollwut-Glykoprotein; ein Vogelgrippe-Antigen; ein Rinderleukämie-Virus-Antigen; ein Newcastle-Disease-Virus (NDV)-Antigen; ein FeLV-Hüllprotein; RAV-1 env; Matrix und/oder Preplomer des infektiöse Bronchitis-Virus; ein Herpesvirus-Glykoprotein; ein Flavivirus-Antigen; ein Immunschwächevirus-Antigen; ein Parvovirus-Antigen; ein equines Influenza-Antigen; ein Mareks-Disease-Virus-Antigen; ein Pockenvirus-Antigen; oder ein infektiöse Bursa1 Disease-Virus-Antigen.

8. Vektor nach Anspruch 7, wobei das Morbillivirus-Antigen das canine Staupe-Virus HA oder F umfasst.

9. Vektor nach Anspruch 5, wobei das Epitop von Interesse ein Epitop von Interesse eines Antigens eines humanen Pathogens oder Toxins umfasst.

10. Vektor nach Anspruch 9, wobei das Epitop von Interesse folgende Antigene umfasst: ein Morbillivirus-Antigen; ein Tollwut-Glykoprotein; ein Influenza-Antigen; ein Herpesvirus-Antigen; ein Flavivirus-Antigen; ein Hepatitisvirus-Antigen; ein Immunschwächevirus-Antigen; ein Hantaanvirus-Antigen; ein *C.tetani*-Antigen; ein Mumps-Antigen; ein Pneumokokken-Antigen; ein *Borrelia-*Antigen; ein *Plasmodium*-Antigen; oder ein Windpocken-Antigen.

11. Vektor nach Anspruch 4, wobei die heterologe DNA einen Wachstumsfaktor oder ein therapeutisches Gen codiert.

12. Vektor nach Anspruch 11, wobei der Wachstumsfaktor oder das therapeutische Gen ein krankheitsbekämpfendes Protein, ein Molekül zur Behandlung von Krebs, einen Tumorsuppressor, ein Cytokin, ein tumorassoziiertes Antigen oder ein Interferon codiert.

13. Vektor nach Anspruch 11, wobei der Wachstumsfaktor oder das therapeutische Gen ausgewählt ist aus der Gruppe, bestehend aus einem Gen, das Alpha-Globulin, Beta-Globulin, Gamma-Globulin, Granulocyten-Makrophagen-Kolonie-stimulierenden Faktor, Tumornekrosefaktor, ein Interleukin, Makrophagen-Kolonie-stimulierenden Faktor; Granulocyten-Kolonie-stimulierenden Faktor, Erythropoietin; Mastzellwachstumsfaktor, Tumorsuppressor p53, Retinoblastom, Interferon, Melanom-assoziiertes Antigen oder B7 codiert.

14. Immunogene, immunologische oder Impfstoff-Zusammensetzung enthaltend das Virus nach Anspruch 1 und einen pharmazeutisch verträglichen Träger oder Verdünnungsmittel.

15. Immunogene, immunologische oder Impfstoff-Zusammensetzung, enthaltend den Vektor nach einem der Ansprüche 2 und 4 bis 10 und einen pharmazeutisch verträglichen Träger oder Verdünnungsmittel.

16. Therapeutische Zusammensetzung enthaltend den Vektor nach einem der Ansprüche 11, 12 und 13 und einen pharmazeutisch verträglichen Träger oder Verdünnungsmittel.

17. Verfahren zur Expression eines Proteins oder Genprodukts oder eines Expressionsprodukts, umfassend die Infektion oder Transfektion einer Zelle *in vitro* mit einem Virus nach Anspruch 1.

18. Verfahren zur Expression eines Proteins oder Genprodukts oder eines Expressionsprodukts umfassend die Infektion oder Transfektion einer Zelle *in vitro* mit einem Vektor nach einem der Ansprüche 2, 4, 5, 6 und 9.

19. Verfahren nach Anspruch 17 oder 18, wobei das Verfahren ferner die Extraktion, Aufreinigung oder Isolierung des Proteins, Genprodukts oder Expressionsprodukts aus der Zelle umfasst.

20. Verfahren zur Clonierung einer heterologen DNA-Sequenz, umfassend die Infektion oder Transfektion einer Zelle *in vitro* mit einem Virus nach Anspruch 1.

21. Verfahren zur Clonierung einer heterologen DNA-Sequenz, umfassend die Infektion oder Transfektion einer Zelle *in vitro* mit einem Vektor nach Anspruch 3.

22. Verfahren nach Anspruch 20 oder 21, wobei das Verfahren ferner die Extraktion, Aufreinigung oder Isolierung der DNA aus der Zelle oder dem Nachkommenvirus umfasst.

23. *In vitro*-Verfahren zur Herstellung des Virus nach Anspruch 1, umfassend das Einfügen der exogenen DNA in einen nicht essentiellen Bereich des CAV2-Genoms.

24. Verfahren nach Anspruch 23, umfassend *in vivo*-Rekombination, umfassend die Transfektion einer Zelle mit CAV2 oder CAV2-DNA in einem zellkompatiblen Medium in Gegenwart von Donor-DNA, umfassend die exogene DNA, die von DNA-Sequenzen flankiert wird, die homolog zu Bereichen des CAV2-Genoms sind, wobei die exogene DNA in das Genom von CAV2 eingefügt ist.

25. Verfahren nach Anspruch 24, wobei durch *in vivo*-Rekombination modifiziertes CAV2 gewonnen wird.

26. Verfahren nach Anspruch 24 oder 25, umfassend die Spaltung der CAV2-DNA zur Gewinnung gespaltener CAV2-DNA, die Ligierung der exogenen DNA mit der gespaltenen CAV2-DNA, um ein Hybrid aus CAV2 und exogenener DNA zu erhalten, die Transfektion der Zelle mit dem Hybrid aus CAV2 und exogenener DNA und die anschließende Gewinnung von CAV2, welches durch die Anwesenheit von exogener DNA modifiziert worden ist.

27. Virus nach Anspruch 1, wobei die exogene DNA einen Promotor umfasst.

28. Virus nach Anspruch 27, wobei der Promotor von einem Herpesvirus stammt.

29. Virus nach Anspruch 28, wobei der Promotor ein CMV-Promotor ist.

30. Virus nach Anspruch 27, wobei der Promotor ein verkürzter transkriptionsaktiver Promotor ist, der einen durch ein transaktivierendes Protein transaktivierten Bereich umfasst, welcher durch das Virus bereitgestellt wird, und die minimale Promotorregion des Promotors eines Promotors voller Länge, von dem der verkürzte transkriptionsaktive Promotor stammt.

31. Virus nach Anspruch 30, wobei der Promotor von einem Herpesvirus stammt.

32. Virus nach Anspruch 31, wobei der Promotor von CMV stammt.

33. Virus nach Anspruch 31, wobei der Promotor vom murinen CMV-IE-Promotor stammt.

34. Virus nach Anspruch 32, wobei der Promotor vom HCMV-IE-Promotor stammt.

35. Virus nach Anspruch 33, wobei der Promotor so verkürzt ist, dass er bis auf 40% der Größe eines murinen CMV-IE-Promotors voller Länge, basierend auf Basenpaaren, reduziert ist.

36. Virus nach Anspruch 34, wobei der Promotor so verkürzt ist, dass er bis auf 90% der Größe eines murinen HCMV-IE Promotors voller Länge, basierend auf Basenpaaren, reduziert ist.

37. Immunogene, immunologische oder Impfstoff-Zusammensetzung, nach Anspruch 14 oder 15 zur Verwendung bei der Impfung.

## Revendications

1. Adénovirus canin 2 (CAV2) synthétiquement modifié de manière à contenir de l'ADN exogène, dans lequel la région E3 ou une portion de celle-ci a été supprimée du CAV2, et dans lequel l'ADN exogène est présent dans une région localisée entre les séquences répétées inversées terminales droites et la région E4, ou dans lequel l'ADN exogène est inséré à la fois dans la région E3 de CAV2 et dans une région localisée entre les séquences répétées inversées terminales droites et l'unité de transcription E4 et qui est encapsidé sous forme de CAV2 infectieux par rapport aux cellules dans lesquelles le CAV2 se réplique naturellement.

2. Vecteur pour l'expression d'ADN hétérologue comprenant le CAV2 selon la revendication 1.

3. Vecteur pour le clonage d'ADN hétérologue comprenant le CAV2 selon la revendication 1.

4. Vecteur selon la revendication 2, dans lequel l'ADN hétérologue code un produit d'expression comprenant : un épitope d'intérêt, un modulateur de réponse biologique, un facteur de croissance, une séquence de reconnaissance, un gène thérapeutique ou une protéine de fusion.

5. Vecteur selon la revendication 4, dans lequel l'ADN hétérologue code un épitope d'intérêt.

6. Vecteur selon la revendication 5, dans lequel l'épitope d'intérêt comprend un épitope d'intérêt provenant d'un antigène d'une toxine ou d'un pathogène vétérinaire.

7. Vecteur selon la revendication 6, dans lequel l'épitope d'intérêt comprend : un antigène du morbillivirus ; une glycoprotéine de la rage ; un antigène de la grippe aviaire ; un antigène du virus de la leucémie bovine, un antigène du virus de la maladie de Newcastle ; une protéine d'enveloppe FeLV ; RAV-1 env ; une matrice et/ou un prépolymère du virus de la bronchite infectieuse ; une glycoprotéine de l'herpèsvirus ; un antigène du flavivirus, un antigène du virus de l'immunodéficience ; un antigène du parvovirus, un antigène de la grippe équine ; un antigène du virus de la maladie de Marek ; un antigène du poxvirus ; ou un antigène du virus de la bursite infectieuse.

8. Vecteur selon la revendication 7, dans lequel l'antigène de morbillivirus comprend le virus HA ou F de la maladie de Carré canine.

9. Vecteur selon la revendication 5, dans lequel l'épitope d'intérêt comprend un épitope d'intérêt provenant d'un antigène d'un pathogène humain ou d'une toxine.

10. Vecteur selon la revendication 9, dans lequel l'épitope d'intérêt comprend : un antigène du morbillivirus ; une glycoprotéine de la rage ; un antigène de la grippe ; un antigène de l'herpèsvirus ; un antigène du flavivirus ; un antigène du virus de l'hépatite ; un antigène du virus de l'immunodéficience ; un antigène du virus Hantaan ; un antigène de *C. tetani ;* un antigène des oreillons, un antigène pneumococcique ; un antigène de *Borrelia* ; un antigène de *Plasmodium* ; ou un antigène de la varicelle.

11. Vecteur selon la revendication 4, dans lequel l'ADN hétérologue code pour un facteur de croissance ou un gène thérapeutique.

12. Vecteur selon la revendication 11, dans lequel le facteur de croissance ou le gène thérapeutique code pour une protéine de lutte contre une maladie, une molécule pour traiter le cancer, un suppresseur de tumeur, une cytokine, un antigène tumoral, ou un interféron.

13. Vecteur selon la revendication 11, dans lequel le facteur de croissance ou le gène thérapeutique est choisi dans le groupe constitué d'un gène codant pour une alpha-globine, une bêta-globine, une gamma-globine, le facteur de croissance hématopoïétique GM-CSF, le facteur de nécrose tumorale, une interleukine, le facteur de croissance hématopoïétique M-CSF, le facteur de croissance hématopoïétique G-CSF, l'érythropoïétine, le facteur de croissance des mastocytes, le suppresseur de tumeur p53, le rétinoblastome, l'interféron, l'antigène associé au mélanome ou B7.

14. Composition immunogène, immunologique ou vaccinale contenant le virus selon la revendication 1, et un support ou diluant pharmaceutiquement acceptable.

15. Composition immunogène, immunologique ou vaccinale contenant le vecteur selon l'une quelconque des revendications 2 et 4 à 10, et un support ou diluant pharmaceutiquement acceptable.

16. Composition thérapeutique contenant le vecteur selon l'une quelconque des revendications 11, 12 et 13, et un support ou diluant pharmaceutiquement acceptable.

17. Procédé d'expression d'une protéine ou d'un produit de gène ou d'un produit d'expression qui comprend l'infection ou la transfection d'une cellule *in vitro* avec un virus selon la revendication 1.

18. Procédé d'expression d'une protéine ou d'un produit de gène ou d'un produit d'expression qui comprend l'infection ou la transfection d'une cellule *in vitro* avec un vecteur selon l'une quelconque des revendications 2, 4, 5, 6 et 9.

19. Procédé selon la revendication 17 ou la revendication 18, dans lequel ledit procédé comprend en outre l'extraction, la purification ou l'isolement de la protéine, du produit de gène ou du produit d'expression à partir de la cellule.

20. Procédé de clonage d'une séquence d'ADN hétérologue comprenant l'infection ou la transfection d'une cellule *in vitro* avec un virus selon la revendication 1.

21. Procédé de clonage d'une séquence d'ADN hétérologue comprenant l'infection ou la transfection d'une cellule *in vitro* avec un vecteur selon la revendication 3.

22. Procédé selon la revendication 20 ou la revendication 21, dans lequel ledit procédé comprend en outre l'extraction, la purification ou l'isolement de l'ADN à partir de la cellule ou du virus de descendance.

23. Procédé *in vitro* de préparation du virus selon la revendication 1, comprenant l'insertion de l'ADN exogène dans une région non-essentielle du génome de CAV2.

24. Procédé selon la revendication 23, comprenant la recombinaison *in vivo* qui comprend la transfection d'une cellule avec le CAV2 ou l'ADN de CAV2 dans un milieu compatible avec la cellule en présence d'ADN de donneur comprenant l'ADN exogène flanqué par des séquences d'ADN homologues de portions du génome de CAV2, l'ADN exogène étant ainsi introduit dans le génome de CAV2.

25. Procédé selon la revendication 24, dans lequel le CAV2 modifié par la recombinaison *in vivo* est récupéré.

26. Procédé selon la revendication 24 ou la revendication 25, qui comprend le clivage de l'ADN de CAV2 pour obtenir l'ADN de CAV2 clivé, la ligature de l'ADN exogène à l'ADN de CAV2 clivé pour obtenir un hybride CAV2-ADN exogène, la transfection d'une cellule avec l'hybride CAV2-ADN exogène, et ensuite la récupération de CAV2 modifié en présence de l'ADN exogène.

27. Virus selon la revendication 1, dans lequel l'ADN exogène comprend un promoteur.

28. Virus selon la revendication 27, dans lequel le promoteur provient d'un herpèsvirus.

29. Virus selon la revendication 28, dans lequel le promoteur est un promoteur de CMV.

30. Virus selon la revendication 27, dans lequel le promoteur est un promoteur tronqué transcriptionnellement actif qui comprend une région transactivée avec une protéine de transactivation fournie par le virus et la région de promoteur minimale d'un promoteur de pleine longueur à partir duquel le promoteur tronqué transcriptionnellement actif est dérivé.

31. Virus selon la revendication 30, dans lequel le promoteur provient d'un herpèsvirus.

32. Virus selon la revendication 31, dans lequel le promoteur provient du CMV.

33. Virus selon la revendication 31, dans lequel le promoteur provient du promoteur CMV-IE murin.

34. Virus selon la revendication 32, dans lequel le promoteur provient du promoteur HCMV-IE.

35. Virus selon la revendication 33, dans lequel le promoteur est tronqué de sorte qu'il y a une réduction en taille allant jusqu'à 40 %, à partir d'un promoteur CMV-IE murin de pleine longueur, par rapport aux paires de bases.

36. Virus selon la revendication 34, dans lequel le promoteur est tronqué de sorte qu'il y a une réduction en taille allant jusqu'à 90 %, à partir d'un promoteur HCMV-IE de pleine longueur, par rapport aux paires de bases.

37. Composition immunogène, immunologique ou vaccinale selon la revendication 14 ou la revendication 15 pour une utilisation en vaccination.
